(19) Europäisches Patentamt
European Patent Office
Office européen des brevets

(11) EP 3 004 134 B1

(12) **EUROPEAN PATENT SPECIFICATION**

(45) Date of publication and mention of the grant of the patent:
**27.02.2019 Bulletin 2019/09**

(21) Application number: **14806955.2**

(22) Date of filing: **04.06.2014**

(51) Int Cl.:
***C07K 1/107*** *(2006.01)* ***C07C 319/02*** *(2006.01)*
***C07C 321/04*** *(2006.01)* ***C07K 7/06*** *(2006.01)*
***C07K 7/08*** *(2006.01)* ***C07C 319/14*** *(2006.01)*
***C07C 323/59*** *(2006.01)*

(86) International application number:
**PCT/AU2014/000583**

(87) International publication number:
**WO 2014/194361 (11.12.2014 Gazette 2014/50)**

(54) **PEPTIDE LIGATION**

PEPTIDLIGATION

LIGATURE PEPTIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2013 AU 2013902006**

(43) Date of publication of application:
**13.04.2016 Bulletin 2016/15**

(73) Proprietor: **The University of Sydney**
**New South Wales 2006 (AU)**

(72) Inventors:
- **PAYNE, Richard, J.**
  **Camperdown NSW 2050 (AU)**
- **THOMPSON, Robert, E.**
  **Surry Hills NSW 2026 (AU)**

(74) Representative: **V.O.**
**P.O. Box 87930**
**2508 DH Den Haag (NL)**

(56) References cited:


- **DATABASE REGISTRY [Online] 16 November 2004 'GLUTAMIC ACID, 4-MERCAPTO- (9CI) (CA INDEX NAME)', XP055297229 Retrieved from STN Database accession no. 781601-14-7**
- **DATABASE REGISTRY [Online] 05 March 2003 'L-ASPARTIC ACID, 3-MERCAPTO- (CA INDEX NAME)', XP055297234 Retrieved from STN Database accession no. 496919-12-1**
- **KRASNOV V. P. ET AL.: 'Synthesis of 4-mercaptoglytamic acid derivatives' RUSSIAN JOURNAL OF ORGANIC CHEMISTRY ''. vol. 35, no. 4, 1999, pages 572 - 577, XP008181363**
- **SHIBATA N. ET AL.: 'Electrophilic sulfenylation in a stereocontrolled synthesis of protected (2R,3R)-3-mercaptoaspartic acid from L-aspartic acid' TETRAHEDRON vol. 52, no. 39, 1996, pages 12839 - 12852, XP055297177**
- **SHIBATA N . ET AL.: 'A stereocontrolled synthesis of protected (2R,3R)-3-mercaptoaspartic acid via a beta-aspartyl enolate sulfenylation' SYNLETT. vol. 6, 1996, pages 519 - 520, XP001182595**
- **HASSE C. ET AL.: 'Native Chemical Ligation at Valine' ANGEW. CHEM. INT. ED. vol. 47, 2008, pages 6807 - 6810, XP008146650**
- **SIMAN P. ET AL.: 'Chemical Synthesis and Expression of the HIV-1 Rev Protein' CHEMBIOCHEM. vol. 12, 2011, pages 1097 - 1104, XP000051763**
- **CHEN J . ET AL.: 'A program tor ligation at threonine sites: application to the controlled total synthesis of glycopeptides' TETRAHEDRON . vol. 66, 2010, pages 2277 - 2283, XP026932573**
- **TAN Z. ET AL.: 'Insights into the Finer Issues of Native Chemical Ligation: An Approach to Cascade Ligations' ANGEW. CHEM. INT. ED. vol. 49, 2010, pages 9500 - 9503, XP055297182**
- **SIMAN P. ET AL.: 'Native chemical ligation at glutamine' ORGANIC LETTERS. vol. 14, no. 6, 2012, pages 1520 - 1523, XP055297183**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- THOMPSON, R. E. ET AL.: 'Chemoselective Peptide Ligation-Desulfurization at Aspaitate' ANGEW. CHEM. INT. ED. vol. 52, no. 37, 2013, pages 9723 - 9727, XP055297184
- THOMPSON, R. E. ET AL.: 'Trifluoroethanethiol: An Additive for Efficient One-Pot Peptide Ligation -Desulfurization Chemistry' J. AM. CHEM. SOC. vol. 136, 2014, pages 8161 - 8164, XP055297185

## Description

### Field

The invention relates to thiolation of amino acids that are used in peptide ligation.

### Background

Native chemical ligation represents an extremely powerful method for the convergent assembly of proteins from smaller peptide fragments. The methodology has been employed in the synthesis of numerous homogeneous proteins, including those possessing post-translational modifications, and has therefore contributed to our understanding of protein structure and function. The native chemical ligation reaction involves the reversible thioesterification reaction between a cysteine (Cys) residue, located at the N-terminus of a peptide fragment, with another peptide bearing a C-terminal thioester (Fig. 1). The resulting thioester intermediate subsequently rearranges through a rapid S→N acyl shift to provide the ligated peptide or protein product linked through a native amide bond. The reaction is high yielding, completely chemoselective in the presence of free side chains of all of the proteinogenic amino acids and proceeds in aqueous media at neutral pH.

The outcome of a Cys residue at the ligation site following the reaction has been circumvented through the use of hydrogenation or radical-based desulfurization chemistry, which can convert Cys residues to alanine (Ala). The radical-based desulfurization reaction, first introduced by Wan and Danishefsky (Q. Wan, S. J. Danishefsky, Angew. Chem. 2007, 119, 9408-9412) using the water-soluble radical initiator 2,2'-azobis(2-(2-imidazolin-2-yl)propane)dihydrochloride (VA-044), has been widely adopted and has featured in the total chemical synthesis of several complex proteins and glycoproteins. Further expansion of the native chemical ligation-desulfurization concept has been made possible through synthetic amino acids bearing side-chain thiol groups, which can facilitate ligation reactions in a similar manner to a Cys residue when incorporated at the N-terminus of peptide fragments. These amino acids can be efficiently desulfurized to afford the native residue following the ligation event (Fig. 1). Although these thiol-derived amino acids have greatly expanded the repertoire of peptide ligation chemistry, highlighted through their use in the assembly of large peptides and proteins, the vast majority of building blocks synthesized to date have not yet been widely adopted by the chemistry community owing, in major part, to the lengthy chemical syntheses required to access them.

N. Shibata et al., Synlett, 1996, Vol. 6, 519-520 describes the stereocontrolled synthesis of the unnatural α-amino acid, (2*R*,3*R*)-3-mercaptoaspartic acid, in a protected form via a β-aspartyl enolate sulfenylation for subsequent use in the biosynthesis of β-lactam antibiotics. The α-amine of the therein described unnatural α-amino acids is protected by allyloxycarbonyl, while the side chain β-carboxyl group is protected with a 4-methoxybenzyl ester. Peptide ligation is not described in the journal article. In the later published article, N. Shibata et al., Tetrahedron, 1996, Vol. 52, No. 39, 12839-12852, the authors reiterate, as well as describe the optimization of, the aforementioned stereocontrolled synthesis of the protected unnatural α-amino acid, and its subsequent use in the synthesis of tripeptides.

### Summary of Invention

The present invention provides a process for introducing a thiol group α to a carbonyl group in a side chain of a protected α-amino acid, said protected α-amino acid having protecting groups on both the α-amine group and the α-carboxyl group, said process comprising:

a) if the side chain contains a functional group comprising a heteroatom bearing a hydrogen atom, protecting said functional group, wherein if the α-amino acid is either aspartic acid or glutamic acid and the side chain carboxyl group is protected by an ester, the ester is t-butyl, methyl or allyl ester;

b) treating the protected amino acid with a base of sufficient strength to abstract a hydrogen atom α to said carbonyl group, so as to form an anion;

c) treating the anion with a reagent of structure Pr-S-L in which L is a leaving group and Pr is a thiol-protecting group, so as to introduce a Pr-S- group α to the carbonyl group; and

d) converting the Pr-S- group to an H-S- (thiol) group.

The following options may be used in conjunction with the foregoing process, either individually or in any suitable combination.

The carbonyl group in the side chain of the protected α-amino acid may be present in an aldehyde, ketone,

carboxylic acid, carboxylic ester or amide group. In particular, it may be present in a carboxylic acid group or a carboxylic ester group. In this case, the protected α-amino acid may be either aspartic acid or glutamic acid, each having both the α-amino group and the α-carboxyl group protected, and step a) may comprise forming an ester, i.e. a t-butyl, methyl or allyl ester, of the side chain carboxyl group. Thus, the carbonyl group on the side chain of the protected α-amino acid and the functional group comprising a heteroatom (if present) may be in the same functional group, e.g. a carboxylic acid, an amide etc. Alternatively, they may be separate (e.g. the side chain may comprise a ketone group and a separate hydroxyl group). It will therefore be understood that the α-amino acid may be a naturally occuring α-amino acid or a non-naturally occuring α-amino acid.

The α-amine group of the protected amino acid may be protected as a carbamate or an amide. A suitable protecting group is a Boc (t-butyloxycarbonyl) protecting group. The α-carboxyl group of the protected amino acid may be protected as an ester, e.g. as an allyl ester.

Pr may be an electron rich group and L may be an electron poor group. Pr may be, for example, a methoxy substituted benzyl group such as a dimethoxy or trimethoxy substituted benzyl group. L may be a sulfonyl group, for example an arylsulfonyl group.

The process may comprise step c') reacting a functional group in the side chain so as to produce a modified natural amino acid, or a protected form of a modified natural amino acid, the modification being a β- or γ-thiol group. Step c') may be conducted either after step c) and before step d) or after step d). Thus, if the protected amino acid used in the process of this aspect comprises a side chain having a functional group, step c') comprises reacting this functional group so as to convert it into a functional group of a naturally occurring amino acid.

The process may comprise step c") deprotecting the α-carboxyl group and coupling the α-carboxyl group of the product of step c) with a peptide so as to produce a peptide having an N-terminus protected amino acid residue having a Pr-S- group in the side chain. In this case, the process represents a process for producing a peptide. In the event that the further elaboration of step c') is conducted, step c") may comprise deprotecting the α-carboxyl group and coupling the α-carboxyl group of the product of step c') (or a protected form thereof) with a peptide. Step c") may be conducted on resin, i.e. it may comprise coupling the product of step c) with a resin-bound peptide. This step may therefore also comprise the step of removing the resulting peptide (having an N-terminus with a side chain protected thiol) from the resin. The typical acidic conditions for doing so may also deprotect the protected thiol so as to form a free thiol group (i.e. step d). The process may also include the step of synthesizing the peptide to which the α-carboxyl group is coupled. This may be by standard solid phase peptide synthesis methods.

The process may comprise additional step c"') coupling the amino acid having a Pr-S- group, or SH- group, in its side chain or peptide having an N-terminal amino acid residue having a Pr-S- group, or SH- group, in its side chain with a thioester of an amino acid or of a peptide so as to form a ligated peptide having a Pr-S- group, or more commonly an HS-group, in the side chain of the amino acid residue derived from the amino acid having the Pr-S- group in the side chain or peptide having an N-terminal amino acid residue having the Pr-S- group in the side chain. The thioester may be an alkoxycarbonylalkylthioester or some other alkyl or aryl thioester, e.g. MESNA thioester (mercaptoethylsulfonate sodium salt) or MPAL (mercaptopropionic acid-leucine) thioester or TFET (2,2,2-trifluoroethanethiol) or other suitable thioester. In this case, the process represents a process for producing a peptide, in particular a ligated peptide. This reaction may be facilitated by the presence of a thiol GrSH. This can form an equilibrium with the thioester so as to form a GrS- thioester. Whereas GrSH may be used in catalytic amounts, greater acceleration of the ligation reaction may be achieved by using larger amounts. Suitable compounds, such as TFET mentioned above, are often quite volatile and may therefore be readily removed from the reaction mixture and may be recycled. Step c"') should be performed after deprotection of the side chain thiol group (or should include this step), i.e. the coupling step should occur after the conversion of the Pr-S group to an HS- group. It may therefore be conducted after step d) (or may include step d). The deprotection and ligation may be conducted in a one pot reaction. As noted above, the deprotection can occur under the same reaction conditions as cleavage of the peptide from a resin to which it is bound.

Step d) may comprise reacting the Pr-S- group with a phosphine, and/or with an acid. In some embodiments, step d) comprises converting the Pr-S- group to a disulfide group RS-S-, or to some other protecting group. The purpose of this is to change the conditions required for deprotection of the protected thiol. This may be, for example, by reacting the Pr-S- group with a reagent $R^aR^bS^+$-SR. Subsequently, the disulfide group may be reduced to the desired thiol. This may, for example, involve mild reducing conditions, e.g. using a phosphine. The step of converting the Pr-S- group to a disulfide group RS-S- may be conducted prior to step c"), or prior to step c"'), so as to provide a more acid stable protecting group to the thiol group during subsequent manipulations such as acidic cleavage of the peptide from solid supported resin, acidic deprotection of side chain protecting groups and elaboration to longer peptides. It will be recognized that in this instance, reference above to Pr-S- (e.g. to "the amino acid having a Pr-S- group in its side chain or peptide having an N-terminal amino acid residue having a Pr-S- group in its side chain") may equally refer to R-S-S-, (e.g. to "the amino acid having an R-S-S- group in its side chain or peptide having an N-terminal amino acid residue having a R-S-S- group in its side chain") or to such compounds having different thiol protecting groups. The Pr-S group may be converted to an R-S-S- group prior to step c"). In this instance, the R-S-S- group may be converted to an HS-

group after step c"). In some embodiments of the invention, step d) is not conducted. This allows for subsequent manipulation of the peptide whilst maintaining the protected thiol in the side chain. In this instance, the PrS-group may be converted to an acid stable group such as an RSS- or other group. Thus in such embodiments the process may involve: a) protecting a functional group comprising a heteroatom bearing a hydrogen atom, said functional group being in the side chain of a protected amino acid; b) treating the protected amino acid with a base of sufficient strength to abstract a hydrogen atom α to said functional group, so as to form an anion; c) treating the anion with a reagent of structure Pr-S-L in which L is a leaving group and Pr is a thiol-protecting group, so as to introduce a Pr-S- group α to the carbonyl group; c') optionally reacting a functional group in the side chain so as to produce a modified natural amino acid, or a protected form of a modified natural amino acid, the modification being a β- or γ-thiol group, and converting the Pr-S- group to an R-S-S- group or other acid stable group; and c") deprotecting the α-carboxyl group and coupling the α-carboxyl group with a peptide so as to produce a peptide having an N-terminus protected amino acid residue having an R-S-S- group or other acid stable group in the side chain. It should be noted that the alphabetic order of the steps, and the number of primes in a step do not necessarily indicate the order in which the steps are conducted. Therefore, for example, in some instances step c''') is conducted after step d). Similarly, step c' may be conducted before or after step c". However, in some instances, the order of conducting the steps will be in alphabetical order and/or in the order of primes.

A suitable process according to an embodiment of the invention involves the steps of:

a) protecting a functional group comprising a heteroatom bearing a hydrogen atom, said functional group being in the side chain of a protected α-amino acid, which protected α-amino acid has protecting groups on both the α-amine group and the α-carbonyl group, wherein if the α-amino acid is either aspartic acid or glutamic acid and the side chain carboxyl group is protected by an ester, the ester is t-butyl, methyl or allyl ester;

b) treating the protected amino acid with a base of sufficient strength to abstract a hydrogen atom α to said functional group, so as to form an anion;

c) treating the anion with a reagent of structure Pr-S-L in which L is a leaving group and Pr is a thiol-protecting group, so as to introduce a Pr-S- group α to the carbonyl group;

c') optionally reacting a functional group in the side chain so as to produce a modified natural amino acid, or a protected form of a modified natural amino acid, the modification being a β- or γ-thiol group, and optionally converting the Pr-S-group to an R-S-S- group;

c") deprotecting the α-carboxyl group and coupling the α-carboxyl group with a peptide so as to produce a peptide having an N-terminus protected amino acid residue having a Pr-S- group or an R-S-S- group in the side chain

d) converting the Pr-S- or R-S-S- group to an H-S- (thiol) group;

c''') coupling the resulting peptide acid having an H-S- group in its side chain, optionally in the presence of a thiol having a pKa of about 5 to about 10, with a thioester of an amino acid or of a peptide, so as to form a ligated peptide having an H-S- group in the side chain of the amino acid residue derived from the amino acid having the H-S- group in the side chain or peptide having an N-terminal amino acid residue having the H-S- group in the side chain.

Steps d) and c''') may be conducted concurrently or sequentially. In particular, if the RSS- protecting group is present, the reducing conditions under which the coupling c''') is conducted may also reduce the RSS- group to an HS- group.

The process may additionally comprise step e) desulfurizing the ligated peptide. The ligated peptide may comprise a cysteine residue and step e) may comprise selectively desulfurizing the ligated peptide so as not to desulfurize the cysteine residue. Step e) may comprise reacting the ligated peptide with a mild reducing agent. The mild reducing agent may comprise a phosphine. The phosphine may be water soluble. It may be, for example, tris-(2-carboxyethyl)phosphine. The reducing agent may additionally comprise a thiol, e.g. dithiothreitol. In some instances, step e) is not chemoselective, i.e. it desulfurizes all thiol groups in the ligated peptide.

Step e) may be conducted at acidic pH, e.g. at about pH 3 or may be conducted at some other pH. This may improve the chemoselectivity of the desulfurization.

Steps c''') and e) may be conducted in a one-pot reaction. Steps c'''), d) and e) may be conducted in a one pot reaction. Steps d) and e) may be conducted in a one pot reaction. Other combinations of steps that may be conducted in one pot include b) and c), c''') and d), and c'''), d) and e). In this context, "one pot" signifies that no separation or purification of intermediate species is conducted. Commonly steps c") and c''') will not be conducted in one pot, since it is generally necessary to purify the product of step c") (optionally including step d), prior to the ligation step c'''). However

there are instances in which these steps may be conducted in one pot.

In an embodiment there is provided a process for introducing a thiol group α to a carbonyl group in a side chain of a protected α-amino acid, said protected α-amino acid being either a protected aspartic acid or a protected glutamic acid, and having protecting groups on both the α-amine group and the α-carboxyl group, said process comprising:

a) protecting the side chain carboxyl group as an ester, i.e. a t-butyl, methyl or allyl ester;

b) treating the protected amino acid with a base of sufficient strength to abstract a hydrogen atom α to said side chain carboxyl group, so as to form an anion;

c) treating the anion with a reagent of structure Pr-S-L in which L is an electron deficient leaving group and Pr is an electron rich thiol-protecting group, so as to introduce a Pr-S- group α to the carbonyl group; and

d) converting the Pr-S- group to an H-S- (thiol) group by reaction with a phosphine or other suitable cleavage reagent.

It should be noted in this context that Pr may be cleaved with acid, in the case that it is Tmob (trimethoxybenzyl) or Dmb (dimethoxybenzyl). A phosphine is not used to deprotect any of the side chain protecting groups. It is only used in the desulfurisation reaction. SFm, as Pr, may be removed with piperidine, and o-nitrobenzyl is UV labile and may therefore be removed by irradiation with a suitable wavelength of UV light. An exception to this is if Pr-S- is, or is initially converted to, a disulfide (RS-S-) prior to conversion to a thiol (whereby the RS- group may be regarded as protecting group Pr-). In this instance, the disulfide protecting group may be reduced to a thiol by means of a phosphine. This may be conducted under mild conditions, e.g. at room temperature and at approximately neutral pH. By contrast, the desulfurization step e) requires more vigorous conditions, commonly acidic pH and elevated temperatures (e.g. 50-60°C).

In another embodiment there is provided a process for introducing a thiol group α to a carbonyl group in a side chain of a protected α-amino acid, said protected α-amino acid being either a protected aspartic acid or a protected glutamic acid, and having protecting groups on both the α-amine group and the α-carboxyl group, said thiolated amino acid being at the N-terminus of a peptide, said process comprising:

a) protecting the side chain carboxyl group as an ester, i.e. a t-butyl, methyl or allyl ester;

b) treating the protected amino acid with a base of sufficient strength to abstract a hydrogen atom α to said side chain carboxyl group, so as to form an anion;

c) treating the anion with a reagent of structure Pr-S-L in which L is an electron deficient leaving group and Pr is an electron rich thiol-protecting group, so as to introduce a Pr-S- group α to the carbonyl group;

c") deprotecting the α-carboxyl group and coupling the α-carboxyl group of the product of step c) with a peptide so as to produce a peptide having an N-terminus protected amino acid residue having a Pr-S- group in the side chain;

c') optionally reacting the side chain carboxylic ester so as to produce a modified natural amino acid, or a protected form of a modified natural amino acid, the modification being a β- or γ-thiol group, said reacting being conducted either between steps c) and d) or after (or instead of) step d).

d) converting the Pr-S- group to an H-S- (thiol) group by reaction with a phosphine or other suitable cleavage reagent, e.g. acid.

In a further embodiment there is provided a process for introducing a thiol group α to a carbonyl group in a side chain of a protected α-amino acid, said protected α-amino acid being either a protected aspartic acid or a protected glutamic acid, and having protecting groups on both the α-amine group and the α-carboxyl group, said thiolated amino acid being within a ligated peptide, said process comprising:

a) protecting the side chain carboxyl group as an ester, i.e. a t-butyl, methyl or allyl ester;

b) treating the protected amino acid with a base of sufficient strength to abstract a hydrogen atom α to said side chain carboxyl group, so as to form an anion;

c) treating the anion with a reagent of structure Pr-S-L in which L is an electron deficient leaving group and Pr is an electron rich thiol-protecting group, so as to introduce a Pr-S- group α to the carbonyl group;

c") deprotecting the α-carboxyl group and coupling the α-carboxyl group of the product of step

c) with a peptide so as to produce a peptide having an N-terminus protected amino acid residue having a Pr-S-group in the side chain

c') optionally reacting the side chain carboxylic ester so as to produce a modified natural amino acid, or a protected form of a modified natural amino acid, the modification being a β- or γ-thiol group, said reacting being conducted either between steps c) and d) or after (or instead of) step d),

d) converting the Pr-S- group to an H-S- (thiol) group by reaction with a phosphine or other suitable cleavage reagent such as acid,

c'") coupling the amino acid having an H-S- group in its side chain or peptide having an N-terminal amino acid residue having an H-S- group in its side chain with a thioester of an amino acid or of a peptide so as to form a ligated peptide having an H-S- group in the side chain of the amino acid residue derived from the amino acid having an H-S- group in the side chain or peptide having an N-terminal amino acid residue having an H-S- group in the side chain, and

e) desulfurizing the ligated peptide, wherein if the ligated peptide comprises a cysteine residue, step e) comprises selectively desulfurizing the ligated peptide so as not to desulfurize the cysteine residue.

In another embodiment there is provided a process for introducing a thiol group α to a carbonyl group in a side chain of a protected α-amino acid, said protected α-amino acid having protecting groups on both the α-amine group and the α-carboxyl group, said process comprising:

a) treating the protected amino acid with a base of sufficient strength to abstract a hydrogen atom α to said side chain carboxyl group, so as to form an anion;

b) treating the anion with a reagent of structure Pr-S-L in which L is an electron deficient leaving group and Pr is an electron rich thiol-protecting group, so as to introduce a Pr-S-group α to the carbonyl group; and

c) converting the Pr-S- group to an H-S- (thiol) group by reaction with a phosphine or other suitable cleavage reagent.

wherein the carbonyl group is not contained in a primary or secondary amide, a carboxylic acid or a thiocarboxylic acid.

Herein is described a method for selectively desulfurizing an α-carbonyl functional thiol in the presence of a thiol having no α-carbonyl group, the method comprising exposing said α-carbonyl functional thiol to a mild reducing agent.

The following options may be used in conjunction with the above-mentioned selective desulfurization method, either individually or in any suitable combination.

The mild reducing agent may comprise a phosphine. The phosphine may be water soluble. It may be for example tris-(2-carboxyethyl)phosphine. The reducing agent may additionally comprises a thiol such as dithiothreitol.

The reaction may be conducted at acidic pH, e.g. about pH 3, or may be conducted at some other pH.

The α-carbonyl functional thiol and the thiol having no α-carbonyl group may be in the same molecule. They may be in different molecules.

The α-carbonyl functionality may be an ester, a carboxyl, an amide, an aldehyde, a ketone or some other carbonyl containing functional group.

In an exemplary method for selectively desulfurizing an α-carbonyl (e.g. carboxy) functional thiol in the presence of a thiol having no α-carbonyl group, the method comprises exposing said α-carbonyl functional thiol to a mild reducing agent comprising a phosphine and a thiol at about pH 3.

Further described herein is a modified amino acid which is a naturally occurring amino acid having a side chain in which a hydrogen atom α to a carbonyl group in said amino acid has been replaced by a thiol group.

The modified amino acid may not be γ-thiolated glutamine. It may be any one or more of β-thiolated aspartic acid, β-thiolated asparagine, γ-thiolated glutamic acid, γ-thiolated glutamine, β-thiolated methionine, β- or γ -thiolated arginine and γ-thiolated lysine. It may be made by the process of the present invention.

It will be understood that the invention also encompasses a product made by the process of the invention described above.

## Brief Description of Drawings

Figure 1 is a scheme showing native chemical ligation and ligation at thiolated amino acids followed by desulfurization.

Figure 2 is a scheme showing synthesis of β-thiolated Asp building block 1.

Figure 3 shows C-S bond dissociation energies of model peptides 14-16.

Figure 4 is a scheme showing synthesis of CXCR4(1-38) 37 via a one-pot Asp ligation-chemoselective desulfurization reaction.

Figure 5 is a graph showing reaction kinetics between model peptide 8 and thioesters 9-13 under ligation conditions.

Figure 6 is a retrosynthetic scheme for compound CXCR4(1-38) **(37)** providing target peptide **38** and peptide thioester **39.**

Figure 7 is a scheme illustrating Fmoc-SPPS of CXCR4(1-19) thioester **38.**

Figure 8 is a scheme illustrating Fmoc-SPPS of CXCR4(20-38) **(39).**

Figure 9 is a scheme illustrating one-pot ligation/selective desulfurization of compounds 39 and 38 to give compound 37.

## Description of Embodiments

The inventors have developed a novel route to synthetic thiolated amino acids which proceeds efficiently in few steps and good yield. In particular, the invention provides a process for synthesising amino acids containing thiol groups in a side chain of the amino acid. The route commences with a protected amino acid. Suitable protected amino acids include protected aspartic acid and protected glutamic acid. In the protected amino acid, the α-amino group and the α-carboxyl group are both protected. Thiol-functional amino acids may be coupled to peptides or amino acids to synthesise peptides, and therefore this additional step may be incorporated into the present process in order to provide a synthetic route to peptides. Finally, the resulting peptides, which still bear a thiol group, may be desulfurised. This may result in a synthetic or a natural peptide.

In the present specification, the term "amino acid" refers to an α- amino acid, "α-amino group" to the amino group attached directly to the carbon atom bearing both an amino and a carboxyl group and "a-carboxyl group" to the carboxyl group attached directly to the carbon atom bearing both an amino and a carboxyl group. In some instances, the term "amino acid" may refer to an amino acid residue within a peptide. This will be dictated by the context. The term "carboxyl" may refer to either a -COOH group or a $-COO^-$ group. Amino acids are of the general form $H_2N-CHR-COOH$, where R is a side chain or H. The side chain in general is an alkyl chain, which is optionally substituted, commonly but not necessarily at its distal end. The N terminus of the amino acid (or of a peptide) is that end at which the amine functionality (optionally ionised or substituted/protected) is located, and the C terminus is the end at which the carboxyl functionality (optionally ionised or substituted/protected) is located. Naturally occurring amino acids have L stereochemistry. The amino acids used in the present invention may be L or may be D or may be racemic. The presently described chemistry may preserve the stereochemistry of the amino acid.

The skilled person will readily appreciate suitable protecting groups which may be used for amino acids. Commonly the amine group will be protected as a carbamate derivative, e.g. t-butyloxycarbonyl (Boc), allyloxycarbonyl (Alloc), fluorenylmethyloxycarbonyl (Fmoc) or ortho-nitrobenzyloxy carbamates, however other types of protecting group, e.g. urea derivatives or amides may also be used in certain cases. In cases where the N-terminus is an amino acid residue containing a thiol group, the thiol and terminal amino group may be protected as a cyclic sulfur-nitrogen containing structure, commonly a cyclic structure containing $NH-CH_2-S$. If the thiol is a β-thiol, the cyclic structure may be a thiazolidine. If the thiol is a γ-thiol, the cyclic structure may be a thiazinane. Such cyclic sulfur-nitrogen protecting groups may be deprotected when required using an acidified amine - suitable conditions include for example methoxyamine ($H_2NOMe$) at about pH 4. The process of the invention may comprise the step of protecting the amino group of the amino acid so as to prepare the protected amino acid or a precursor thereto.

Similarly, the skilled person will appreciate suitable protecting groups for the carboxylic acid group(s). In the event that the amino acid has two carboxylic acid groups (i.e. the α carboxyl group and a side chain carboxyl group), it

may be convenient to have these protected with different protecting groups which have different deprotection conditions so as to enable selective deprotection of one or other of the carboxylic acid groups selectively if required. Carboxylic acids are commonly protected as their esters, however amides, hydrazides or other known protecting groups may also be used. Suitable esters include alkyl, aryl, allyl, benzyl, silyl or thiol esters. For example, as in examples provided in the present specification, an allyl ester may be used for one carboxylic acid and an alkyl ester for another. This enables selective removal of the allyl protecting group (e.g. by a palladium catalyst) without affecting the alkyl ester protecting group, which is of benefit. The process may comprise the step(s) of protecting the carboxyl group(s) of the amino acid so as to prepare the protected amino acid or a precursor thereto.

Thus, the protected amino acid used as a starting point for the process described herein may be purchased as such or may be prepared from the original amino acid (i.e. from an unprotected amino acid) or from a partially protected derivative thereof.

The protected amino acid is subjected to a strong base in order to deprotonate the β- or γ- carbon atom (i.e. that carbon α to a side chain carbonyl group) so as to produce an anion. The deprotonation is commonly facilitated by the presence of a functional group, e.g. an ester, attached to the β-carbon atom or γ-carbon atom. However it will be understood that common protecting groups for the amine group, e.g. Boc, leave a proton attached to the protected nitrogen group, which is also abstractable by strong base. Accordingly it is necessary to ensure firstly that the base is sufficiently strong that, if it first abstracts the hydrogen on the protected nitrogen atom, it is still capable of abstracting the β-hydrogen atom, and secondly that sufficient base is provided to abstract two hydrogen atoms (i.e. from the protected nitrogen and from the β- or γ- carbon atom). The base is preferably a non-nucleophilic base. A suitable base is LiHMDS (lithium bis(trimethylsilyl)amide), however other metal amide bases such as LDA (lithium diisopropylamide) may also be used. The base should be used in greater than molar equivalent to the protected amino acid, commonly at least about 2 molar equivalents. It may be used in about 1.5 to about 3 molar equivalents, or about 1.5 to 2, 2 to 3, 2 to 2.5, 1.8 to 2.2 or 2 to 2.2 molar equivalents, e.g. about 1.5, 1.6, 1.7, 1.8, 1.9, 2, 2.1, 2.2, 2.3, 2.4, 2.5 or 3 molar equivalents.

In general, the deprotonation reaction is conducted at low temperature to reduce or minimise side reactions. Suitable temperatures are below 0°C, or below -10, -20, -50 or - 70°C, or about -100 to about 0°C, or about -100 to -50, -100 to -70, -50 to 0, -20 to 0 or -80 to -60°C, e.g. about -100, -90, -80, -78, -70, -60, -50, -40, -30, -20, -10 or 0°C. The reaction may be conducted under an inert atmosphere, e.g. nitrogen, helium, argon, carbon dioxide etc.

The anion obtained from the above deprotonation is then treated with a thiolating reagent. The thiolating agent is of the general formula Pr-S-L, where Pr is a protecting group for a thiol and L is a leaving group. This reaction attaches the Pr-S- group to the β- or γ-carbon atom of the protected amino acid as a protected thiol group. The leaving group L is commonly an electron deficient group. It may be for example an arylsulfonyl group such as tosyl ($CH_3PhSO_2$), phenylsulfonyl ($PhSO_2$), an electron deficient thio group such as dinitrothiophenyl etc. or may be some other suitable leaving group, e.g. halide. The protecting group Pr may be any suitable thiol protecting group, commonly a thioether or thioester. Preferably Pr is an electron rich group such as a suitably substituted benzyl group. These include alkoxy substituted benzyl groups such as 2,4- or 3,5-dimethoxybenzyl or 2,3,4- or 2,4,6-trimethoxybenzyl groups or a trityl (triphenylmethyl) group, as well as S-Fm (S-fluorenylmethyl) group. The particular protecting group Pr may be designed so as to be cleavable under predetermined conditions as required. It may for example be cleavable under acid conditions (e.g. trimethoxybenzyl) or under photolytic conditions (e.g. o-nitrobenzyl) or under some other conditions, e.g. base (e.g. for piperidinyl or S-Fm protecting groups). As noted elsewhere herein, disulfides (RS-S-) may be suitable protecting groups and are conveniently cleaved to the unprotected thiol using mild reducing conditions e.g. phosphines. It will be understood that the addition of the thiolating reagent to the anion is conducted *in situ,* and is therefore under similar reaction conditions of solvent, temperature and atmosphere to those used in formation of the anion itself. The thiolating reagent may be used in small excess over the anion, e.g. about 10, 20, 30, 40, 50 or 60% mole excess. This reaction results in the production of a fully protected β- or γ- thiolated amino acid, i.e. having protecting groups on the α-amino group, the α-carboxyl group and, if present, the β- or γ- carboxyl group (or other functional group attached to the β- or γ- carbon atom).

An alternative route to the thiol is to convert the Pr-S- group initially to a disulfide group RS-S-. This has the advantage of providing a more acid stable group which can be of advantage in subsequent elaborations, e.g. in ligation reactions discussed elsewhere herein. Conversion to a disulfide may be effected for example by reacting the Pr-S- group with a reagent $R^aR^bS^+$-SR. Subsequently, the disulfide group may be reduced to the desired thiol. The nature of $R^a$, $R^b$ and R is not critical. They may each be, for example and alkyl group or an aryl group. Suitable groups include methyl, ethyl, propyl and phenyl. A suitable reagent therefore may be $Me_2S^+$-SMe. Counterions are also not critical, and may for example be $BF_4^-$, $Cl^-$, $Br^-$ or other commonly known and available anions. The reduction of the disulfide to a thiol is a reaction well known in the art. Suitable reducing conditions include zinc and acid, or phosphines such as tris(2-carboxyethyl)phosphine.

The fully protected β- or γ- thiolated amino acid may be at least partially deprotected. As noted above, suitable protecting groups may be selected so that selective deprotection of one or more protecting groups may be conducted without affecting others. Thus for example, an allyl ester protecting group for the α-carboxyl group may be removed without affecting a Boc protecting group on the α-amine group or a t-butyl ester protecting group on a β- or γ-carboxyl group.

The functional group of the side chain of the thiolated amino acid produced by the method described herein may be converted into a variety of other functional groups by known methods. This may be conducted either before or after deprotection of the newly introduced thiol group as appropriate. This may result in conversion to a thiolated form of a natural amino acid (optionally in protected form). For example, if the initial protected amino acid is a protected aspartic acid, the thiolated product would be β-thiolated aspartic acid (in protected form), which may be converted by standard chemical methods into the corresponding amide, i.e. β-thiolated asparagine.

The selectively deprotected α-carboxyl group may then be used for conjugation with the N-terminus of a peptide by conventional methods. These include SPPS (solid phase peptide synthesis), e.g. Fmoc or Boc type SPPS. It will be recognised that the selectively deprotected amino acid may equally be coupled to the amine function of a second amino acid (having an unprotected α-amino group) so as to form a dipeptide in which the N-terminal amino acid has a protected thiol group.

As used herein, the term "peptide" refers to a chain comprising (or consisting of) at least two amino acid residues joined by amide bond(s). They may be dipeptides, oligopeptides, polypeptides, proteins, glycopeptides, glycoproteins etc. and each amino acid residue may, independently, optionally be protected. It will therefore be understood that proteins, either natural or synthetic, come within the scope of the term "peptide". A peptide may have at least 2 amino acids, or at least 5 or at least 10 amino acids. It may have for example from about 2 to about 10,000 amino acids or from about 2 to about 1000 amino acids. It may refer to an oligopeptide (between 2 and about 20 amino acids), or a polypeptide, or a protein. In some definitions, proteins are considered to have greater than 70 amino acids.

The conjugation with a peptide or other amino acid proceeds smoothly regardless of the nature of the N-terminal amino acid residue of the peptide or of the other amino acid.

Deprotection of the resulting product (i.e. of the thiol thereof) provides a peptide (dipeptide or larger) having at its N-terminus an amino acid residue having an unprotected thiol, e.g. a β-thiol group. The nature of the deprotection reaction will depend on the nature of the protecting group. If the protecting group is photolabile, e.g. o-nitrobenzyl, the deprotection may comprise exposing the protected peptide to a suitable wavelength of light, e.g. UV light. Commonly, the thiol protecting group is acid sensitive (e.g. the benzyl group) however other groups will be sensitive to other conditions, e.g. allyl (sensitive to Pd(0)) or fluorenylmethyl (base sensitive). In this case (i.e. the case of an acid sensitive thiol protecting group), conveniently, the step of cleaving the peptide from a solid state support used in the SPPS may also result in deprotection of the thiol group in a single step.

As discussed earlier, peptides having N-terminal cysteine residues may be ligated to peptide thioesters and this reaction is useful in peptide synthesis. However this reaction has hitherto been limited by the requirement for an N-terminal cysteine residue. The inventors have found that this reaction may be extended to peptides in which the N-terminal residue is a non-natural amino acid residue derived from (commonly obtained from) a natural amino acid by β-thiolation or γ-thiolation, for example as described earlier herein. Reactions with the non-natural amino acid residue terminated peptides proceed in comparable yield and at comparable rate to those using cysteine residue terminated peptides. The reactions are commonly conducted in a denaturing buffer in the presence of an arylthiol or alkylthiol catalyst. A suitable arylthiol is thiophenol. A suitable alkylthiol is trifluoroethanethiol ($CF_3CH_2SH$). In general the thiol is of formula GrSH. Suitable Gr groups are such that the GrSH thiol is sufficiently nucleophilic to undergo transthioesterification with the thioester, and should be suitably labile to perform as a leaving group in the ligation reaction. Suitable Gr groups are such that the pKa of GrSH is between about 5 and about 10, or about 5 to 8, 6 to 8, 6 to 10 or 6 to 9, e.g. about 5, 5.5, 6, 6.5, 7, 7.5, 8, 8.5, 9, 9.5 or 10. Typical Gr groups include fluoroalkyl groups such as $CF_3CH_2$-, $C_2F_5$-, $C_2F_5CH_2$- and $C_3F_7$-. As noted above, the PrS- (or other) thiol protecting group should be removed before ligation to the N-terminal group of a peptide. This may occur *in situ* with removal of the peptide from a supporting resin for acid labile groups. For reductively labile groups, the reducing conditions commonly used in the ligation (i.e. with thiols and optionally phosphines) can reduce such groups in situ to the corresponding thiol. Thus, for example, if the PrS- group is converted to a disulfide protecting group, reaction as discussed above with a thioester, optionally in the presence of a thiol and/or phosphine, leads to initial deprotection of the thiol and subsequent in situ ligation with the thioester.

The sequence above therefore provides a convenient way for producing peptide and protein sequences. Initially, a protected thiol is introduced into the side chain of an amino acid. After suitable deprotection of the α-carboxyl group, this can be coupled to the N-terminus of a peptide so as to produce a peptide having an N-terminal amino acid with a protected thiol in its side chain. Following deprotection of the side chain thiol, and of the N-terminal amino group, the N-terminus can be coupled to a second peptide, this reaction proceeding by way of the C-terminus thioester of the second peptide. As discussed earlier, it is thought that this reaction occurs by initial formation of a thioester (-C(=O)S-), and subsequent rearrangement to an amide accompanied by regeneration of the free thiol group in the side chain. This effectively couples the two peptides through the original amino acid. The side chain thiol can then be desulfurized if required. The inventors have identified suitable selective desulfurization conditions which can be conducted in the presence of native cysteine moieties in the two ligated peptide moieties, as discussed below.

This ligation reaction, when applied to the non-natural side chain thiol functional amino acid residue terminated peptides, results in a peptide having an amino acid residue having a non-natural side chain thiol group. Commonly,

however, it is often desired to produce peptides consisting of only natural amino acid residues. It is in such cases desirable to desulfurize the thiol functional amino acid residue. However, since many desirable peptides contain cysteine residues which also contain thiols, reduction of the thiol group of the non-natural thiol functional amino acid residue would be expected to also reduce the thiol group of any cysteine residues present in the peptide.

The inventors have surprisingly discovered that it is possible to selectively reduce the thiol of the non-natural thiol functional amino acid residue, which is $\alpha$ to a carbonyl functionality, without reducing the thiol group of any cysteine residues present in the peptide. A particular example is when the thiol group has an $\alpha$-carboxyl group. In such cases, due to the differential susceptibilities of the different thiol groups, selective desulfurization is possible. Thus reaction of peptides containing $\alpha$-carboxythiol functionality in an amino acid residue side chain may be readily reduced to the corresponding desulfurized peptide in reasonable yield by exposure to a mild reducing agent. Suitable reducing agents include phosphines, optionally in combination with thiols. It is convenient for the phosphine, and if present the thiol, to be water soluble. Thus a suitable phosphine is tris-(2-carboxyethyl)phosphine. A suitable thiol is dithiothreitol. The reaction may be conducted at moderately elevated temperatures, or at room temperature or below. Suitable temperatures are, for example, about 10 to about 80°C, or about 20 to 80, 50 to 80, 70 to 80, 10 to 30, 10 to 50, 30 to 60, 30 to 40, 40 to 70 or 50 to 70°C, e.g. about 10, 15, 20, 25, 30, 35, 40, 45, 50, 55, 60, 65, 70, 75 or 80°C. The inventors have further found that this desulfurization reaction is facilitated or accelerated by the presence of a protonated carboxyl group attached to the carbon atom to which the thiol is bonded. Therefore, under acidic reaction conditions, selectivity of the thiol reduction is improved. The reaction may therefore be conducted at a pH at which the carboxyl group attached to the carbon atom to which the thiol is bonded is protonated. It may be conducted at a pH of less than about 4, or less than about 3.5, 3.4, 3.3, 3.2, 3.1 or 3. It may be conducted at a pH of about 1 to about 4, or about 2 to 4, 3 to 4, 1 to 3, 2 to 3, 2.5 to 3.5 or 2.5 to 3, e.g. at about pH 1, 1.5, 2, 2.5, 2.6, 2.7, 2.8, 2.9, 3, 3.1, 3.2, 3.3, 3.4, 3.5, or 4. In some cases the reaction may be conducted at other pH, e.g. at neutral or even basic pH. Such reaction conditions may however be less selective than those at acidic pH as described above. They may therefore result in concomitant reduction of other thiols in the molecule, e.g. in cysteine moieties. However, if no other thiols are present, i.e. no cysteine residues, or if it is desired to reduce other thiols that are present, there may be no requirement for selectivity and therefore pH control may be of lesser importance and therefore other pHs than those described above may be used.

It will be recognised that the reaction described above represents a general method for selectively desulfurizing $\alpha$-functional thiols in the presence of other thiols. The two thiol groups may be in the same molecule, or may be in different molecules in the same reaction mixture. They may each, independently, be in peptide molecules or may be in non-peptide molecules. The functionality $\alpha$ to the thiol may be a carbonyl group (e.g. aldehyde, ketone, carboxyl, carboxylate, carboxamide etc.), an ether, a thioether etc.

The inventors have found that the ligation and selective desulfurization steps described above may conveniently be conducted as a one pot reaction. They may be conducted without isolation or purification of intermediate species. Thus, following the ligation reaction, the crude reaction mixture may be subjected, without purification of intermediates (but optionally with at least partial removal of at least one reagent or catalyst used in the ligation reaction), to suitable desulfurization conditions and reagents. The resulting ligated and selectively desulfurized product peptide may be obtained from the resulting reaction mixture following a suitable time for reaction.

Particular examples of the general invention described above will now be outlined.

The inventors have developed a short and scalable route to a suitably protected $\beta$-thiolated aspartate (Asp) residue and its implementation in ligation-desulfurization chemistry. To this end, a three-step synthesis of protected $\beta$-thiolated Asp building block **1** from the affordable and commercially available amino acid Boc-Asp(O*t*Bu)-OH **2** has been used (see Fig. 2). The acid-labile 2,4,6-trimethoxybenzyl (Tmob) protected thiol moiety was installed at the $\beta$-position through the use of the novel sulfenylating reagent **3.** Other suitable protecting groups that may be used instead of Tmob include Dmb (2,4-dimethoxybenzyl) and S-Fm (S-fluorenylmethyl). Reagent **3** was prepared in high yield through the reaction of 2,4,6-trimethoxybenzyl alcohol **4** and potassium toluenethiosulfonate **5.** Allyl (All) ester protection of Boc-Asp(O*t*Bu)-OH **2** provided the fully protected Asp derivative **6.** Treatment of **6** with two equivalents of lithium hexamethylsilazide (LiHMDS) at low temperature generated the corresponding dianion, which was treated with sulfenylating agent **3** to afford the Tmob-protected $\beta$-thiolated amino acid **7,** produced as a 9:1 diastereomeric mixture in favor of the *syn*-(*erythro*) isomer. These two diastereoisomers could be separated by column chromatography to provide *erythro*-**7** in 56% yield, the stereochemistry of which was confirmed by NMR coupling constant analysis. Finally, palladium(0)-catalyzed All ester deprotection afforded the desired $\beta$-thiolated Asp building block **1** in 80% yield, which could be incorporated directly into solid-phase peptide synthesis (SPPS). Overall, **1** was prepared in three steps from commercially available **2** in 45% overall yield.

Having successfully prepared **1,** the building block was next incorporated into model peptide **8** using standard Fmoc-strategy SPPS. Coupling of **1** to a resin-bound peptide was achieved using standard amino acid coupling conditions (e.g. PyBOP) and the Tmob-protecting group on the $\beta$-thiol moiety was concomitantly removed under the standard acidolytic conditions used for cleavage of the peptide from the resin and removal of standard protecting groups. Ligation reactions between peptide **8** and a number of peptide thioesters **9-13** bearing a representative selection of C-terminal

residues (Gly, Ala, Met, Phe and Val) were next carried out to determine the scope of the reactions (Table 1). Ligations were conducted in a denaturing buffer comprising 6 M guanidine hydrochloride (Gn•HCl), 200 mM HEPES and 50 mM tris-(2-carboxyethyl)phosphine (TCEP) at 37 °C and pH 7.2-7.4. An excess of thiophenol (2vol.%) was used as the aryl thiol catalyst in each of the reactions. Surprisingly, each of these peptide ligations proceeded to completion with rates comparable to those reported for native chemical ligation of peptides bearing *N*-terminal cysteine residues (determined by LC-MS analysis). Specifically, reaction of **8** with Gly thioester **9** was complete in 20 minutes, reactions with Ala, Met and Phe thioesters **10-12** were complete within 90 minutes, while reactions with the more sterically demanding Val thioester **(13)** required 24 hours to reach completion. It is important to note that, although it has been shown that ligation rates at β-thiolated leucine are significantly faster when conducted on the *threo*-diasteroisomer compared with the *erythro*-counterpart, the inventors have determined that ligations at β-thiolated Asp are equally facile at both diastereoisomers. Following reverse-phase HPLC purification, the ligation products were isolated in excellent yields (71-82%, entries 1-5, Table 1). These rapid ligation rates and reaction yields (even at sterically hindered Val thioesters) would suggest that ligations at Asp may possess a similarly wide scope to native chemical ligation at Cys. Following isolation of the ligation products, these were subsequently subjected to a radical-based desulfurization reaction using VA-044 (2,2'-azobis[2-(2-imidazolin-2-yl)propane]dihydrochloride) in the presence of TCEP and reduced glutathione. All desulfurization reactions proceeded to completion within 16 h, and, following reverse-phase HPLC purification, the native peptide products were isolated in 63-76% yields (entries 1-5, Table 1).

***Table 1.*** Ligation-desulfurization reactions at Asp.

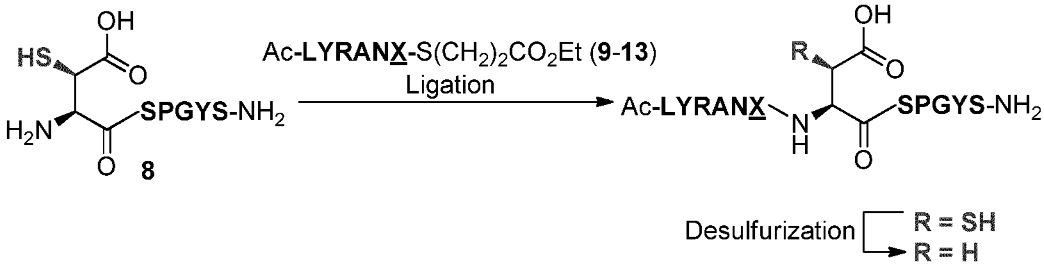

8

| Entry | Peptide Thioester (X =) | Ligation yield[a] [%] | Desulfurization yield[a] [%] |
|---|---|---|---|
| 1 | Gly (**9**) | 80 | 75 |
| 2 | Ala (**10**) | 82 | 71 |
| 3 | Met (**11**) | 71 | 63 |
| 4 | Phe (**12**) | 78 | 76 |
| 5 | Val (**13**) | 75 | 71 |

(a) Isolated yields after HPLC purification. **Ligation conditions:** 5 mM **8** in buffer (6 M Gn•HCl, 200 mM HEPES, 50 mM TCEP), PhSH (2 vol%), 37 °C, pH 7.2-7.4, 24 h. **Desulfurization conditions:** 5 mM in buffer (6 M Gn•HCl, 200 mM HEPES, 250 mM TCEP) reduced glutathione (40 mM), VA-044 (20 mM) pH 6.5-7.0), 37 °C, 16 h.

Although ligation-desulfurization reactions have greatly expanded the scope of ligation chemistry, a major limitation of this methodology is the inability to chemoselectively desulfurize the thiol moieties (used to facilitate the ligation reaction) in the presence of free sulfhydryl side chains of Cys residues, which are concomitantly converted to Ala under both reductive and radical conditions. This unwanted side reaction can be prevented by global protection of the Cys side chains in the sequence. However, this necessitates additional deprotection and purification steps in the synthesis and prevents the use of expressed protein ligation (EPL) methodologies with recombinantly expressed fragments.

Given these limitations, the inventors wished to develop a chemoselective desulfurization reaction at β-thiolated Asp. It is known that radical deselenization of selenocysteine, β-selenolphenylalanine and γ-selenolproline could be effected in the presence of unprotected cysteine residues in the absence of a radical initiator by treating ligation products with TCEP and dithiothreitol (DTT). The inventors hypothesised that this selectivity may arise from the significantly weaker carbon-selenium bond in the selenated amino acids compared with the carbon-sulfur bond of Cys. By analogy, it was therefore envisaged that the rate of radical desulfurization of thiolated amino acids would be correlated with C-S bond strengths i.e. the energy necessary to generate the β-carbon-centered radical, and that the propensity of radical formation would be governed by neighboring functional groups. For β-thiolated aspartate, it was thought that the electronic properties of the carboxylate/carboxylic acid functionality at the β-position may weaken the C-S bond, thus affecting the rate of desulfurization. Carbon-centered radicals with an adjacent carboxylic acid group have been found to be stabilized relative to the unsubstituted counterparts through *in silico* investigations (G. P. F. Wood, D. Moran, R. Jacob, L. Radom,

J. Phys. Chem. A 2005, 109, 6318-6325) and, as such, it was considered that selective desulfurization of β-thiolated Asp over Cys might be possible.

In order to probe this idea, the inventors used computational studies to predict the bond dissociation energies (BDEs) corresponding to the cleavage of the C-S and S-H bonds in cysteine, deprotonated β-thiolated aspartate and protonated β-thiolated aspartic acid. The BDEs of the S-H bonds of **14-16,** calculated with the high-level G3X(MP2)-RAD procedure (D. J. Henry, M. B. Sullivan, L. Radom, J. Chem. Phys. 2003, 118, 4849-4860), were found to be very similar (353.1-357.9 kJ $mol^{-1}$) and significantly larger than the energy required to break the C-S bonds in these molecules (see Figure 3). There was a negligible difference between the C-S BDEs of **14** and **15** (308.5 and 308.0 kJ $mol^{-1}$, respectively) despite the presence of a carboxylate side chain in **15.** However, upon protonation of the carboxylate (as in **16**), the C-S bond was predicted to be significantly weaker (BDE = 298.3 kJ $mol^{-1}$). Notably, the ~10 kJ $mol^{-1}$ lower BDE of **16** compared with **14** and **15** corresponds to a roughly two orders of magnitude increase in rate for the homolytic cleavage of the C-S bond at room temperature. The C-Se BDE in the selenium analogue of **14** is calculated to be 276.4 kJ $mol^{-1}$. These results suggest that selective desulfurization of β-thiolated Asp may be possible in the presence of Cys.

Based on computational results, the inventors investigated the development of a one-pot chemoselective ligation-desulfurization reaction at this residue. To this end, peptide **17** was synthesised, bearing both a β-thiolated aspartic acid residue on the N-terminus and a cysteine residue within the peptide sequence (Table 2). This peptide was reacted with peptide thioester **9** bearing a *C*-terminal Gly residue under identical conditions to those described previously (entry 1, Table 2). The reaction reached completion to provide the desired ligation product after 30 minutes, and after this time thiophenol was extracted from the ligation mixture using diethyl ether to prevent this from hindering the desulfurization reaction rate. Following removal of the aryl thiol, the mixture was immediately treated with 250 mM TCEP and 50 mM DTT and the reactions monitored at a variety of temperatures and pHs. The most rapid rate of desulfurization and complete selectivity was observed when the reaction was conducted at pH = 3 at 65 °C. Increasing the pH of the desulfurization reaction led to a distinct decrease in the desulfurization rate of the β-thiolated Asp residue, thus leading to a loss in desulfurization selectivity over the side chain of Cys. This result is consistent with the computational results where the C-S bond is predicted to be significantly weaker when the side chain is protonated (as in the carboxylic acid, pKa of β-$CO_2H$ of Asp = 3.86). After incubating for 20 h under the optimized conditions, HPLC-MS analysis revealed complete consumption of the ligation product and showed only the singly desulfurized **18** as the major product, together with some minor by-products. $^1$H NMR, analytical HPLC analysis and ms/ms sequencing of this product matched identically with synthetically prepared **18,** proving unequivocally that the cysteine residue had been retained in the product. Purification by reverse-phase HPLC provided **18** in 48% isolated yield over the two steps (entry 1, Table 2), comparable to Asp ligations carried out *via* separate ligation and desulfurization steps (45-60% over two steps, Table 1).

***Table 2.*** One-pot ligation-chemoselective desulfurization reactions.

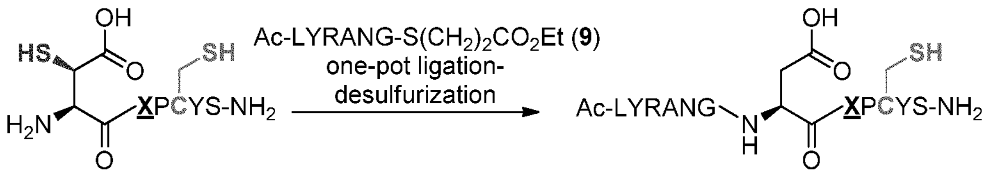


**17, 19-27** **18, 28-36**

| Entry | Peptide (X =) | Product (X=) | Yield[a] [%] |
|---|---|---|---|
| 1 | Ser (**17**) | Ser (**18**) | 48 |
| 2 | Gly (**19**) | Gly (**28**) | <5[b] |
| 3 | Pro (**20**) | Pro (**29**) | 0[b] |
| 4 | Ala (**21**) | Ala (**30**) | 45 |
| 5 | His (**22**) | His (**31**) | 59 |
| 6 | Lys (**23**) | Lys (**32**) | 47 |
| 7 | Glu (**24**) | Glu (**33**) | 57 |
| 8 | Asn (**25**) | Asn (**34**) | 50 |
| 9 | Phe (**26**) | Phe (**35**) | 63 |
| 10 | Ile (**27**) | Ile (**36**) | 58 |

[a] Isolated yields after HPLC purification over two steps. [b] Analytical yield from HPLC-MS analysis. **Conditions:** 5 mM **17, 19-27** in buffer (6M Gn•HCl, 200 mM HEPES, 50 mM TCEP), PhSH (2 vol.%), 37 °C, pH 7.2-7.4, 2 h; then $Et_2O$ washing and dilution to 2.5 mM in buffer (6M Gn•HCl, 200 mM HEPES, 250 mM TCEP), 50 mM DTT, 65 °C, pH 3.0, 20 h.

An investigation into the identity of the minor by-products showed that these arose from bond cleavage at the Asp-Ser junction to generate two peptides, Ac-LYRANGD-OH and H-SPCYS-OH. This reaction is a known degradation pathway of Asp-containing peptides and proteins at low pH, with the propensity of peptide bond cleavage dictated by the nature of the amino acid found on the *C*- terminal side of the Asp residue. It should be noted that peptides containing a Ser residue on the *C*-terminal side of Asp residues (such as in **18**) are known to be highly prone to amide bond scission, yet the one-pot ligation-desulfurization reaction still represents a synthetically useful transformation when this motif is present in the sequence. In order to further evaluate the utility and scope of the one-pot ligation-chemoselective desulfurization reaction, a range of peptides **19-27** were synthesised, bearing a representative range of amino acids on the *C*-terminal side of the β-thiolated Asp residue (Table 2). These peptides were ligated to peptide thioester **9** and after 2 h the reactions were treated with TCEP (250 mM) and DTT (50 mM) to effect the desulfurization. After incubation for 20 h, the reactions were assessed by HPLC-MS before purification by reverse-phase HPLC. The vast majority of the one-pot ligation-desulfurization reactions provided the native peptides as the major product without any detectable Cys desulfurization and only minimal peptide cleavage by-products. The exceptions were the reactions of peptides bearing glycine (**19,** entry 2, Table 2) and proline (**20,** entry 3, Table 2) on the *C*-terminal side of the Asp residue where the Asp-Gly and Asp-Pro bonds were almost quantitatively cleaved under the desulfurization conditions. This result was not unexpected and reflects the known lability of these bonds which, in the case of Pro, is successfully exploited in peptide and protein sequencing. Gratifyingly, the one-pot ligation-chemoselective desulfurization reactions of all the remaining peptides possessing Ala **(25),** His **(26),** Lys **(27),** Glu **(28),** Asn **(29),** Phe **(30)** and lie **(31)** residues on the *C*-terminal side of the β-thiolated Asp moiety provided excellent yields of the desired singly-desulfurized products **(28-36)** over the two steps following purification by reverse-phase HPLC (45-63% over two steps, entries 4-10, Table 2). Importantly, in all cases ligation-desulfurization of **19-27** provided synthetically useful yields of the target peptides **(28-36)** that were comparable or better than similar reactions conducted over two steps using a radical initiator (Table 1). This suggests that the one-pot ligation-chemoselective desulfurization reaction represents a general methodology that should have utility for a range of substrates.

Having investigated the scope of the one-pot Asp ligation-selective desulfurization methodology, the inventors used the methodology to assemble the extracellular *N*-terminal domain of the chemokine receptor CXCR4 bearing two homogeneous post-translational modifications (*N*-linked glycosylation and Tyr sulfation). The inventors were interested in the *N*-terminal domain of CXCR4 as a test of the synthetic utility of our methodology due to the presence of three Asp residues and one Cys residue within the 38 amino acid sequence. Doubly-modified CXCR4(1-38) **37** was assembled *via* ligation between glycopeptide **38** bearing a C-terminal Met thioester and neopentyl (nP) protected sulfopeptide **39** possessing an *N*-terminal β-thiolated Asp moiety. Ligation between **38** and **39** was carried out under the same conditions described for the model systems. After 24 h, LC-MS analysis indicated that the ligation reaction had proceeded to completion and proceeded with concomitant nP ester deprotection, which in control studies was shown to be due to nucleophilic deprotection by TCEP in the ligation buffer. At this stage, thiophenol was extracted from the reaction with diethylether before TCEP and DTT were added to the crude ligation reaction, and the reaction heated at 65 °C at pH 3.0 for 24 h to effect the chemoselective desulfurization reaction. After 24 h of incubation, HPLC-MS analysis indicated successful single desulfurization of the ligation product as well as a minor by-product corresponding to imide formation between the backbone amide and the side chain of Asp20. It was noted that the acidic desulfurization conditions did not lead to loss of the acid-labile sulfate ester moiety in **37.** Purification *via* reverse-phase HPLC then provided the full *N*-terminal domain of CXCR4(1-38) bearing an *N*-linked glycan and Tyr sulfation in 20% yield over the two steps. The regioselectivity of the desulfurization reaction was confirmed by ms/ms sequencing of the glycosulfopeptide product. Figure 4 illustrates the ligation and subsequent selective desulfurization reactions which were conducted as a one pot reaction.

In summary, the inventors have successfully developed an expedient and scalable route to a suitably protected β-thiolated aspartate building block that is capable of facilitating rapid ligation to peptide thioesters with rates similar to those observed for native chemical ligation at Cys. Computational studies were used to guide the development of an initiator free radical desulfurization reaction that can chemoselectively desulfurize the β-thiol of Asp in the presence of free sulfhydryl side chains of Cys residues. The development of this methodology has enabled ligation reactions to be carried out at β-thiolated Asp followed by chemoselective desulfurization in the same reaction vessel. Importantly, this represents the first chemoselective desulfurization reported for thiolated amino acids. The methodology reduces the number of intermediate HPLC purification steps and the need for side-chain protection of Cys residues, which are usually necessary for ligation-desulfurization chemistry. The one-pot ligation-chemoselective desulfurization methodology at β-thiolated Asp proved to be efficient for a number of examples, and was successfully employed in the synthesis of the *N*-terminal domain of CXCR4 bearing two post-translational modifications. Given the straightforward synthesis of the β-thiolated Asp building block **1** and the operationally simple nature of the one-pot ligation-desulfurization methodology described here, it is anticipated that this methodology will find widespread use in the chemical synthesis of peptides and proteins.

**Example 1**

**General Synthetic Experimental**

$^1H$ and $^{13}C$ NMR spectra were recorded at 300K using a Bruker Avance DPX 500 spectrometer. Chemical shifts are reported in parts per million (ppm) downfield from internal tetramethylsilane (TMS). $^1H$ NMR data is reported as chemical shift ($\delta_H$), multiplicity (s = singlet, d = doublet, t = triplet, q = quartet, dd = doublet of doublets, ddd = doublet of doublet of doublets) and coupling constant (*J* Hz) and relative integral. $^{13}C$ NMR data is reported as chemical shift ($\delta$).

Low-resolution mass spectra were recorded on a Shimadzu 2020 mass spectrometer (ESI) operating in positive mode unless indicated otherwise. High resolution ESI-TOF mass spectra were measured on a Bruker-Daltonics Apex Ultra 7.0T fourier transform mass spectrometer (FTICR). High resolution MALDI-FTICR mass spectra were measured on a Bruker-Daltonics Apex Ultra 7.0T Fourier transform mass spectrometer (FTICR) using a matrix of 10 mg/mL $\alpha$-cyano-4-hydroxycinnamic acid in water/acetonitrile (1:1 v/v) containing 0.1 vol.% TFA. Infrared (IR) absorption spectra were recorded on a Bruker ALPHA Spectrometer with Attenuated Total Reflection (ATR) capability, using OPUS 6.5 software. Optical rotations were recorded on a Perkin-Elmer 341 polarimeter at 589 nm (sodium D line) with a cell path length of 0.2 dm, and the concentrations are reported in g/100 mL.

Analytical reverse-phase HPLC was performed on a Waters System 2695 separations module with a 2996 photodiode array detector and an Alliance series column heater set at 30 °C. A Waters Sunfire 5 $\mu$m, 2.1 x 150 mm column (C-18) was used at a flow rate of 0.2 mL $min^{-1}$ using a mobile phase of 0.1% TFA in water (Solvent A) and 0.1% TFA in acetonitrile (Solvent B). Sulfated peptide **39** were eluted using a mobile phase of 0.1M $NH_4OAc$ (Solvent A) and acetonitrile (solvent B). Results were analyzed with Waters Empower software.

Preparative reverse-phase HPLC was performed using a Waters 600 Multisolvent Delivery System and Waters 500 pump with 2996 photodiode array detector or Waters 490E Programmable wavelength detector operating at 230 and 254 nm. $\beta$-Mercapto peptides were purified on a Waters Sunfire 5 $\mu$m (C-18) preparative column operating at a flow rate of 7 mL $min^{-1}$ using a mobile phase of 0.1% formic acid in water (Solvent A) and 0.1% formic acid in acetonitrile (Solvent B). Ligation and desulfurization products were purified on a Waters Sunfire 5 $\mu$m (C-18) 10 x 250 mm semi-preparative column operating a flow rate of 4 mL $min^{-1}$ using a mobile phase of 0.1% TFA in water (Solvent A) and 0.1% TFA in acetonitrile (Solvent B) and a linear gradient of 0-50% B over 40 min. CXCR4 peptide fragments **(37-39)** were purified on a Waters Sunfire 5 $\mu$m (C-18) 10 x 250 mm semi-preparative column operating a flow rate of 4 mL $min^{-1}$ using a mobile phase of 0.1% TFA in water (Solvent A) and 0.1% TFA in acetonitrile (Solvent B) and a linear gradient as noted.

LC-MS was performed on a Shimadzu LC-MS 2020 instrument consisting of a LC-M20A pump and a SPD-20A UV/Vis detector coupled to a Shimadzu 2020 mass spectrometer (ESI) operating in positive mode. Separations were performed on a Waters Sunfire 5 $\mu$m, 2.1 x 150 mm column (C18), Xbridge BEH300 5 $\mu$m, 2.1 x 150 mm column (C18) or a Waters Symmetry 300 5 $\mu$m, 2.1 x 150 mm (C4) column, operating at a flow rate of 0.2 mL $min^{-1}$. Separations were performed using a mobile phase of 0.1% formic acid in water (Solvent A) and 0.1% formic acid in acetonitrile (Solvent B) and a linear gradient of 0-50% B over 30 min or 0-30% B over 30 min.

**Materials**

Analytical thin layer chromatography (TLC) was performed on commercially prepared silica plates (Merck Kieselgel 60 0.25 mm F254). Flash column chromatography was performed using 230-400 mesh Kieselgel 60 silica eluting with analytical grade solvents as described. Ratios of solvents used for TLC and column chromatography are expressed in v/v as specified. Compounds were visualised by UV light at 254 nm or using vanillin or cerium molybdate stain.

Commercial materials were used as received unless otherwise noted. Reagents that were not commercially available were synthesized following literature procedures and referenced accordingly. Dichloromethane was distilled from calcium hydride, and THF was distilled from sodium/benzophenone. Anhydrous methanol, dimethylformamide and diethyl ether were purchased from Sigma Aldrich. Reactions were carried out under an atmosphere of nitrogen or argon unless otherwise stated.

**Synthetic Experimental Procedures**

**S-(2,4,6-trimethoxybenzyl)toluenethiosulfonate (3)**

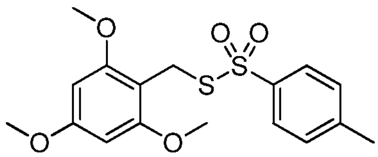

To a solution of 2,4,6-trimethoxybenzylalcohol (2.0 g, 10 mmol) and potassium *p*-toluenethiosulfonate (2.3 g, 10 mmol) in MeOH (50 mL) at 0 °C was added trifluoroacetic acid dropwise (0.84 mL, 11 mmol). The resulting mixture was stirred at 0 °C for 15 min and the colourless precipitate collected through filtration. The fine solid was then recrystallized from EtOAc, affording **3** as a colourless, crystalline solid (2.65 g, 72% yield); m.p 111-112 °C (EtOAc), **IR** $v_{max}$ 2977, 1601, 1415, 1205, 1148, 1140 $cm^{-1}$; **$^1$H NMR** (500 MHz, $CDCl_3$) δ 7.86 (2H, d, *J* = 8.1 Hz), 7.32 (2H, d, *J* = 8.1Hz), 6.02 (2H, s), 4.29 (2H, s), 3.77 (3H, s), 3.69 (6H, s), 2.45 (3H, s) ppm; **$^{13}$C NMR** (125.8 MHz, $CDCl_3$) δ 161.5, 159.1, 143.9, 142.5, 129.4, 127.2, 102.6, 90.4, 55.7, 55.3, 29.1, 21.6 ppm; **HRMS** (ESI) *m*/*z* calcd. for $C_{17}H_{20}O_5S_2Na+$ $(M+Na)^+$ 391.0644, found 391.0644.

**Boc-Asp(*t*Bu)-OAll (6)**

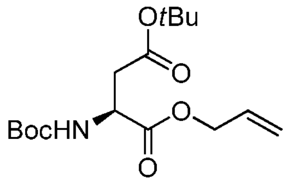

To a stirred solution of Boc-Asp(OtBu)-OH (5.0 g, 15 mmol), in DMF (50 mL) was added *i*$Pr_2$NEt (3.9 mL, 22.5 mmol) and allyl bromide (1.7 mL, 22.2 mmol). The resulting solution was stirred at room temperature for 16 h and then concentrated under reduced pressure. The crude residue was then filtered through a plug of silica eluting with hexane/ethyl acetate (4:1, v/v). Concentration of the filtrate afforded pure **6** as a colourless oil (4.6 g, 94%). $[\alpha]_D^{25}$ +25.0° (*c* 1.0, $CHCl_3$); **IR** $v_{max}$ 2979, 1718, 1499, 1367, 1156 $cm^{-1}$; **$^1$H NMR** (500 MHz, $CDCl_3$) δ 5.90 (dddd, *J* = 5.6, 5.7, 10.6, 17.2 Hz, 1H), 5.49 (br d, *J* = 8.7 Hz, 1H), 5.33 (dq, *J* = 17.2, 1.5 Hz, 1H), 5.24 (dq, *J* = 10.6, 1.1 Hz, 1H), 4.67 (ddt, *J* = 13.2, 5.6, 1.5 Hz, 1H), 4.62 (ddt, *J* = 13.2, 5.6, 1.5 Hz, 1H), 4.55 (dt, *J* = 4.4, 8.7 Hz, 1H), 2.90 (dd, *J* = 4.5, 16.8 Hz, 1H), 2.90 (dd, *J* = 4.7, 16.8 Hz, 1H), 1.45 (s, 9H), 1.44 (s, 9H) ppm; **$^{13}$C NMR** (125.8 MHz, $CDCl_3$) δ 171.1, 170.2, 155.6, 131.7, 118.6, 81.7, 80.1, 66.2, 50.3, 38.0, 28.4, 28.1 ppm; **HRMS** (ESI) *m*/*z* calcd. for $C_{16}H_{27}NO_6Na+$ $(M+Na)^+$ 352.1731, found 352.1730.

**(2R, 3R)-Boc-Asp(tBu, STmob)-OAllyl (7)**

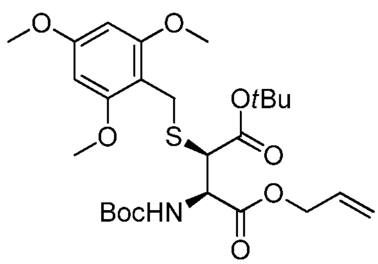

To a solution of **6** (1.0 g, 3.0 mmol) in THF (30 mL) at -78 °C was added LiHMDS (1M in THF, 6.6 mL, 6.6 mmol) and stirred for 2 h at -78 °C. A solution of **3** (1.5 g, 4.2 mmol) in THF (15 mL) was then added dropwise over 10 min. After a further 2 h at -78 °C the reaction was quenched with saturated aqueous $NH_4Cl$ and concentrated under reduced pressure. The residue was then partitioned between EtOAc (50 mL) and saturated aqueous $NH_4Cl$ (50 mL)

and the organic phase was washed with saturated aqueous $NH_4Cl$ (2 × 50 mL), brine (50 mL) and then dried over $MgSO_4$. The crude product (d.r 9:1) was then purified using flash column chromatography on silica gel, eluting with Hexane/EtOAc (6:1, v/v) affording pure diasteromer **7** as a colourless oil (0.91 g, 56%). $[\alpha]_D^{25}$ +25.6° (*c* 1.0, $CHCl_3$); **IR** $\nu_{max}$ 2975, 1716, 1595, 1496, 1368, 1149, 1110, 1058 $cm^{-1}$; **$^1$H NMR** (500 MHz, $CDCl_3$) δ 6.11 (s, 1H), 5.88 (dddd, *J* = 5.6, 5.7, 10.6, 17.2 Hz, 1H), 5.75 (br d, *J* = 10.1 Hz, 1H), 5.32 (dq, *J* = 17.2, 1.5 Hz, 1H), 5.23 (dq, *J* = 10.5, 1.3 Hz, 1H), 4.74 (dd, *J* = 10.1, 4.5 Hz, 1H), 4.65 (ddt, *J* = 13.3, 5.6, 1.5 Hz, 1H), 4.58 (ddt, *J* = 13.3, 5.6, 1.3 Hz, 1H), 4.00 (d, *J* = 12.3 Hz, 1H), 3.99 (d, *J* = 4.5 Hz, 1H), 3.91 (d, *J* = 12.3 Hz, 1H), 3.81 (s, 6H), 3.81 (s, 3H), 1.45 (s, 9H), 1.43 (s, 9H) ppm; **$^{13}$C NMR** (125.8 MHz, $CDCl_3$) δ 171.0, 170.2, 160.6, 159.0, 156.1, 131.6, 118.5, 107.0, 82.3, 79.9, 66.1, 55.3, 55.1, 49.0, 28.3, 28.2, 25.0 ppm; **HRMS** (ESI) *m/z* calcd. for $C_{26}H_{39}NO_9SNa+$ $(M+Na)^+$ 564.2238, found 564.2239.

**(2R,3R)-Boc-Asp(tBu, STmob)-OH (1)**

**(2R, 3S)-Boc-Asp(tBu, STmob)-OH (S1)**

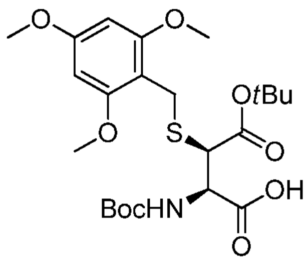

**1**

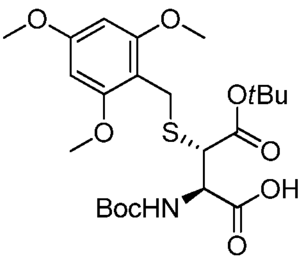

**S1**

**Method A:** To a solution of **7** (400 mg, 0.74 mmol) in THF (5 mL) was added *N*-methylaniline (160 μL, 1.5 mmol) and $Pd(PPh_3)_4$ (43 mg, 37 μmol). The solution was stirred at r.t for 30 min and then concentrated under reduced pressure. The crude residue was immediately purified through flash column chromatography on silica gel, eluting with a gradient of Hexane/EtOAc (3:1→7:3, v/v containing 1 vol.% AcOH) affording **1** as a colourless oil (297 mg, 80%), $[\alpha]_D^{25}$ +25.5 (*c* 1.0, $CHCl_3$); **IR** $\nu_{max}$ 2976, 1715, 1595, 1497, 1367, 1149, 1110 $cm^{-1}$; **$^1$H NMR** (500 MHz, $CDCl_3$) δ 8.34 (br s, 1H), 6.11 (s, 2H), 5.86 (br d, *J* = 8.6 Hz, 1H), 4.70 (dd, *J* = 8.5, 3.7 Hz, 1H) 4.00 (d, *J* = 12.6 Hz, 1H), 3.97 (d, *J* = 3.7 Hz, 1H), 3.91 (d, *J* = 3.9 Hz, 1H), 3.81 (s, 6H), 3.80 (s, 3H), 1.46, (s, 9H), 1.43 (s, 9H) ppm; **$^{13}$C NMR** (125.8 MHz, $CDCl_3$) δ 172.9, 172.5, 160.7, 159.0, 106.8, 90.9, 90.4, 83.2, 80.5, 55.3, 55.0, 48.4, 28.3, 28.0, 25.5 ppm; **HRMS** (ESI) *m/z* calcd. for $C_{23}H_{35}NO_9SNa$ $(M+Na)^+$ 524.1925, found 524.1925.

**Method B:** 1M aqueous NaOH (1 mL) was added to a solution of **7** (100 mg, 0.18 mmol) in MeOH (5 mL) and stirred at ambient temperature for 16 h. The solution was partially concentrated and carefully acidified to pH 3 with 1M HCl. The mixture was then extracted with $CH_2Cl_2$ (3 × 20 mL) and the organic phase was then dried with $MgSO_4$ and concentrated to afford a 1:1 diastereomeric mixture of **1** and **S1**. [The lability of the β-proton under basic conditions is in accordance with that observed by N. Shibata. 3. E. Baldwin. A. Jacobs. M. E. Wood. Tetrahedron 1996. 52. 12839-12852.] Separation of **1** and **S1** was achieved by reverse-phase HPLC (0→100% B over 40 min), affording pure **1** (29 mg, 32% yield, spectroscopic data identical to that above) and pure **SI,** (35 mg, 39% yield), $[\alpha]_D^{25}$ −15.4° (*c* 1.0, $CHCl_3$); **IR** $\nu_{max}$ 2975, 1715, 1596, 1596, 1456, 1368, 1149, 1110, 1057 $cm^{-1}$; **$^1$H NMR** (500 MHz, $CDCl_3$) δ 7.00-6.40 (br s, 1H), 6.11 (s, 2H), 5.43 (br d, *J* = 7.2 Hz, 1H), 4.67 (dd, *J* = 7.2, 6.4 Hz, 1H), 3.90 (s, 2H), 3.85 (d, *J* = 6.4 Hz, 1H), 3.82 (s, 6H), 3.80 (s, 3H), 1.47 (s, 9H), 1.43 (s, 9H) ppm; **$^{13}$C NMR** (125.8 MHz, $CD_3Cl_3$) δ 172.6, 169.1, 161.0, 159.1, 156.5, 106.7, 91.1, 82.3, 81.0, 55.9, 55.4, 54.7, 49.6, 28.3, 27.9, 24.8 ppm; **HRMS** (ESI) *m/z* calcd. for $C_{23}H_{35}NO_9SNa$ $(M+Na)^+$ 524.1925, found 524.1925.

Coupling constants between Hα and Hβ of **7** (4.5 Hz) strongly suggest that **1** is the *erythro* diastereomer which is in accordance with the high *erythro* selectivity observed by Shibata *et al.* in electrophilic sulfenylation of protected aspartate dianions with 2,4-dimethoxybenzylthio-tosylate (reported $J$ = 4.5 Hz). Large differences in coupling constants between Hα and Hβ of **1** ($J$ = 3.7 Hz) compared with **S1** ($J$ = 6.4 Hz) is consistent with these stereochemical assignments.

## Peptide Synthesis

Model peptide thioesters (Ac-LYRANX-$S(CH_2)_2CO_2Et$, X = G, A, M, F, V) **(9-13)** were prepared according to literature methods.[2]

## Solid-Phase Peptide Synthesis

*Loading Rink Amide resin:* Rink amide resin was initially washed with DCM (5 × 3 mL) and DMF (5 × 3 mL), followed by removal of the Fmoc group by treatment with 20% piperidine/DMF (2 × 5 min). The resin was washed with DMF (5 × 3 mL), DCM (5 × 3 mL) and DMF (5 × 3 mL). PyBOP (4 eq.) and NMM (8 eq.) were added to a solution of Fmoc-AA-OH (4 eq.) in DMF (final concentration 0.1 M). After 5 min of pre-activation, the mixture was added to the resin. After 2 h the resin was washed with DMF (5 × 3 mL), DCM (5 × 3 mL) and DMF (5 × 3 mL), capped with acetic anhydride/pyridine (1:9 v/v) (2 × 3 min) and washed with DMF (5 × 3 mL), DCM (5 × 3 mL) and DMF (5 × 3 mL).

*Loading 2-chloro-trityl chloride resin:* 2-Chloro-trityl chloride resin (1.22 mmol/g loading) was swollen in dry DCM for 30 min then washed with DCM (5 × 3 mL). A solution of Fmoc-AA-OH (0.5 equiv. relative to resin functionalization) and $i$$Pr_2NEt$ (2.0 eq. relative to resin functionalization) in DCM (final concentration 0.1 M of amino acid) was added and the resin shaken at rt for 16 h. The resin was washed with DMF (5 × 3 mL) and DCM (5 × 3 mL). The resin was treated with a solution of DCM/$CH_3OH$/i$Pr_2NEt$ (17:2:1 v/v/v, 3 × 3 mL × 5 min) for 1 h and washed with DMF (5 × 3 mL), DCM (5 × 3 mL), and DMF (5 × 3 mL). The resin was subsequently submitted to iterative peptide assembly (Fmoc-SPPS).

*Loading estimation of amino acid loading:* The resin was treated with 20% piperidine/DMF (3 mL, 3 × 3 min) and the combined deprotection solution made up to 10 mL with DMF. The solution was diluted 200-fold with DMF and the UV absorbance of the resulting piperidine-fulvene adduct measured ($\lambda$ = 301 nm, $\varepsilon$ = 7800 $M^{-1}$ $cm^{-1}$) to estimate the amount of amino acid loaded onto the resin.

## General iterative peptide assembly (Fmoc-SPPS):

*Deprotection:* The resin was treated with 20% piperidine/DMF (3 mL, 3 × 3 min) and washed with DMF (5 × 3 mL), DCM (5 × 3 mL) and DMF (5 × 3 mL).

*General amino acid coupling:* A solution of protected amino acid (4 eq.), PyBOP (4 eq.) and NMM (8 eq.) in DMF (final concentration 0.1 M) was added to the resin. After 1 h, the resin was washed with DMF (5 × 3 mL), DCM (5 × 3 mL) and DMF (5 × 3 mL).

*Capping:* Acetic anhydride/pyridine (1:9 v/v) was added to the resin (3 mL). After 3 min the resin was washed with DMF (5 × 3 mL), DCM (5 × 3 mL) and DMF (5 × 3 mL).

*Coupling conditions for* **1** *and* **S1**: A solution of compound **1/S1** (2.0 eq.), PyBOP (2.0 eq.), and NMM (4.0 eq.) in DMF (final concentration 0.1 M) was then added to the resin (1.0 eq.) and shaken at rt for 16 h. The resin was then washed with DMF (5 × 3 mL), DCM (5 × 3 mL), DMF (5 × 3 mL), and DCM (10 × 3 mL). When coupling was conducted using HATU, significant guanylation of the N-terminus was observed.

*Coupling conditions for Fmoc-Asn(GlcNAc)-OH (**S2**) and Frnoc-Tyr($SO_3$nP)-OH (***S3***):* A solution of amino acid (1.2 eq.), HATU (1.15 eq.) and NMM (2.4 eq.) in DMF (final concentration 0.1 M) was added to the resin (1.0 eq.) and shaken. After 18 h, the resin was washed with DMF (5 × 3 mL), DCM (5 × 3 mL), and DMF (5 × 3 mL). A capping step was performed as described above, and synthesis of the desired glyco/sulfopeptide was completed using iterative Fmoc-SPPS.

*On resin O-deacetylation:* The resin (25 μmol) was washed with DMF (5 × 3 mL), DCM (5 × 3 mL), and DMF (5 × 3 mL). A 5 vol.% solution of hydrazine hydrate in DMF was prepared and added to the resin (3 mL). The peptide was shaken at room temperature for 16 h and washed with DMF (10 × 3 mL), DCM (10 × 3 mL), and DMF (10 × 3 mL). A small portion of resin was cleaved using the acidic cleavage conditions and analyzed *via* LC-MS to ensure complete removal of the acetate groups. In the case that the reaction had not reached completion after this time, the deacetylation procedure was repeated once.

*Cleavage*: A mixture of TFA, thioanisole, triisopropylsilane (TIS) and water (90:5:2.5:2.5 v/v/v/v) was added to the resin. After 2 h, the resin was washed with TFA (3 × 2 mL).

*Work-up*: The combined solutions were concentrated under a stream of nitrogen. The residue was dissolved in water containing 0.1% TFA, filtered and purified by preparative HPLC and analyzed by LC-MS and ESI mass spectrometry.

**Model peptides containing β(SH)Asp**

**H-(β-SH)DSPGYS-$NH_2$ (8)**

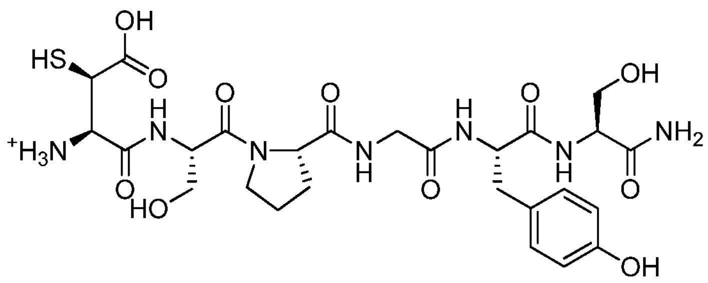

Peptide **8** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (14.8 mg, 84% yield based on the original 25 μmol resin loading).

Analytical HPLC: $R_t$ 18.6 min (0-30% B over 40 min, 0.1% TFA, λ = 230 nm); Calculated Mass $[M+H]^+$: 656.2; Mass Found ($ESI^+$); 656.6 $[M+H]^+$

**(epi)-H-(β-SH)DSPGYS-$NH_2$ (S5)**

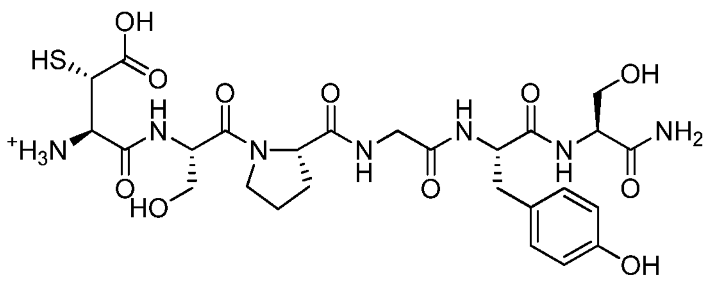

Peptide **S5** was prepared according to Fmoc-strategy SPPS outlined in the general procedures, incorporating protected amino acid **S1** and purified by semi-preparative reverse phase HPLC (0 to 30% B over 40 min, 0.1% formic acid) to afford the target compound as a colourless solid following lyophilization (5.3 mg, 76% yield based on the original 10 μmol resin loading). Analytical HPLC: $R_t$ 18.3 min (0-50% B over 40 min, 0.1% TFA, λ = 230 nm); Calculated Mass $[M+H]^+$: 656.2; Mass Found ($ESI^+$); 656.6 $[M+H]^+$.

**H-(β-SH)DSPCYS-$NH_2$ (17)**

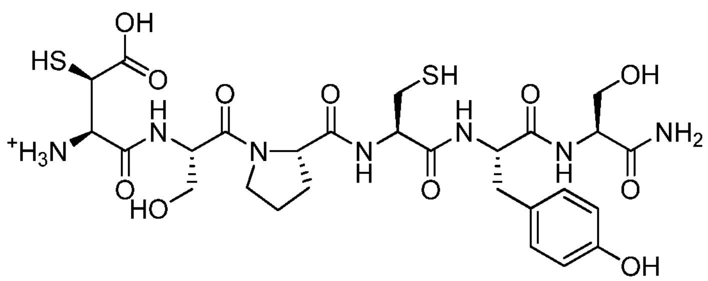

Peptide **17** was prepared according to Fmoc-strategy SPPS outlined in the general procedures and purified by preparative reverse phase HPLC (0 to 30% B over 40 min, 0.1% formic acid) to afford the target compound as a colourless

solid following lyophilization (4.6 mg, 62% yield based on the original 10 μmol resin loading).

Analytical HPLC: $R_t$ 21.5 min (0-30% B over 40 min, 0.1% TFA, λ = 230 nm); Calculated Mass $[M+H]^+$: 702.2; Mass Found ($ESI^+$); 702.3 $[M+H]^+$

**H-(β-SH)DGPCYS-$NH_2$ (19)**

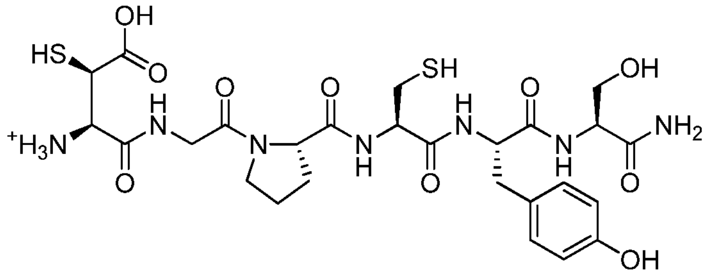

Peptide **19** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (4.2 mg, 58% yield based on the original 10 μmol resin loading).

Analytical HPLC: $R_t$ 22.6 min (0-50% B over 40 min, 0.1% TFA, λ = 230 nm); Calculated Mass $[M+H]^+$: 672.2; Mass Found ($ESI^+$); 672.3 $[M+H]^+$.

**H-(β-SH)DAPCYS-$NH_2$ (21)**

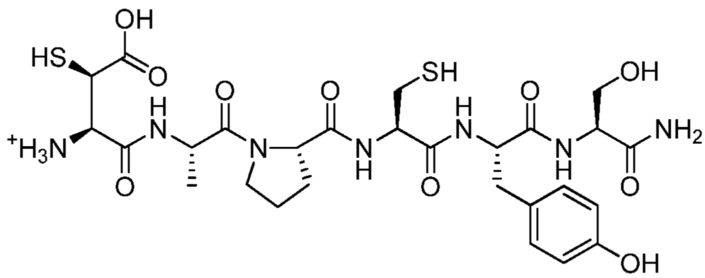

Peptide **21** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (4.3 mg, 59% yield based on the original 10 μmol resin loading).

Analytical HPLC: $R_t$ 22.8 min (0-30% B over 40 min, 0.1% TFA, λ = 230 nm); Calculated Mass $[M+H]^+$: 686.2; Mass Found ($ESI^+$); 686.3 $[M+H]^+$.

**H-(β-SH)DHPCYS-$NH_2$ (22)**

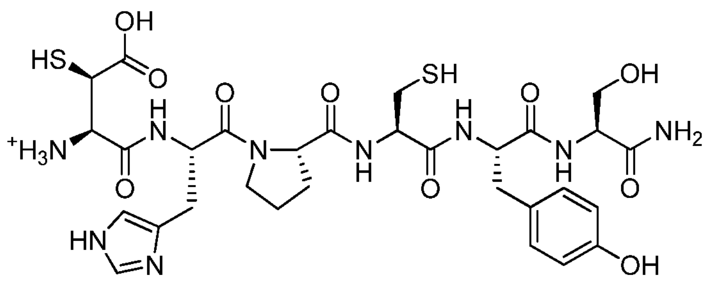

Peptide **22** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (3.6 mg, 43% yield based on the original 10 μmol resin loading).

Analytical HPLC: $R_t$ 21.3 min (0-50% B over 40 min, 0.1% TFA, λ = 230 nm); Calculated Mass $[M+H]^+$: 751.2; Mass Found ($ESI^+$); 752.3 $[M+H]^+$.

**H-(β-SH)DKPCYS-$NH_2$ (23)**

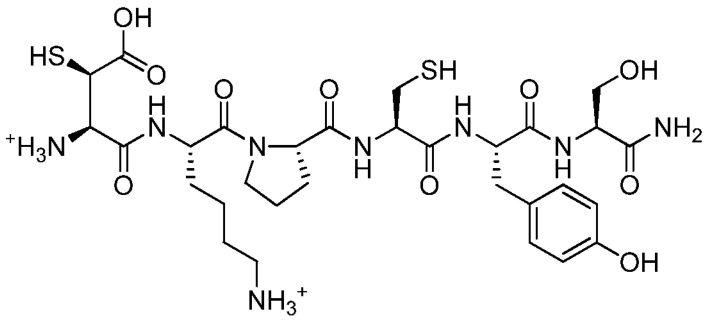

Peptide **23** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (5.0 mg, 60% yield based on the original 10 μmol resin loading).

Analytical HPLC: $R_t$ 20.7 min (0-50% B over 40 min, 0.1% TFA, λ = 220 nm); Calculated Mass $[M+H]^+$: 743.3; Mass Found ($ESI^+$); 743.3.8 $[M+H]^+$.

**H-(β-SH)DEPCYS-$NH_2$ (24)**

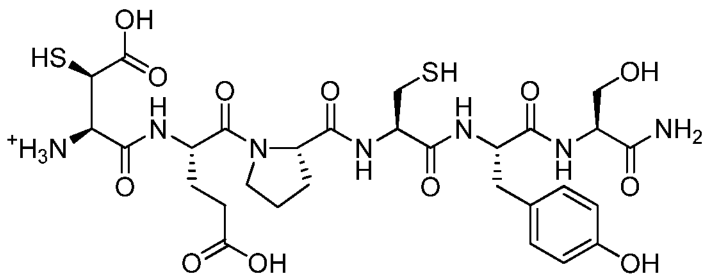

Peptide **24** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (4.0 mg, 51% yield based on the original 10 μmol resin loading).

Analytical HPLC: $R_t$ 33.5 min (0-50% B over 40 min, 0.1% TFA, λ = 230 nm); Calculated Mass $[M+H]^+$: 744.2; Mass Found ($ESI^+$); 744.3 $[M+H]^+$.

**H-(β-SH)DNPCYS-$NH_2$ (25)**

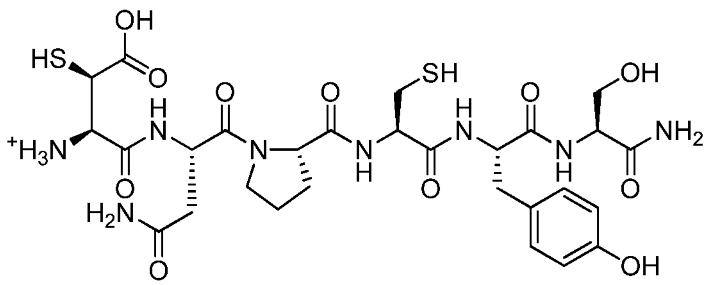

Peptide **25** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (5.2 mg, 67% yield based on the original 10 μmol resin loading).

Analytical HPLC: $R_t$ 22.4 min (0-30% B over 40 min, 0.1% TFA, λ = 230 nm); Calculated Mass $[M+H]^+$: 729.2; Mass Found ($ESI^+$); 729.2 $[M+H]^+$.

**H-(β-SH)DFPCYS-$NH_2$ (26)**

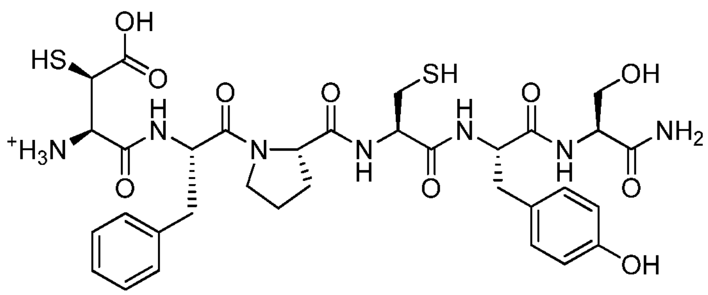

Peptide **26** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (5.6 mg, 69% yield based on the original 10 μmol resin loading).

Analytical HPLC: $R_t$ 22.5 min (0-50% B over 40 min, 0.1% TFA, λ = 220 nm); Calculated Mass $[M+H]^+$: 762.3; Mass Found ($ESI^+$); 762.3 $[M+H]^+$

**H-(β-SH)DIPCYS-$NH_2$ (27)**

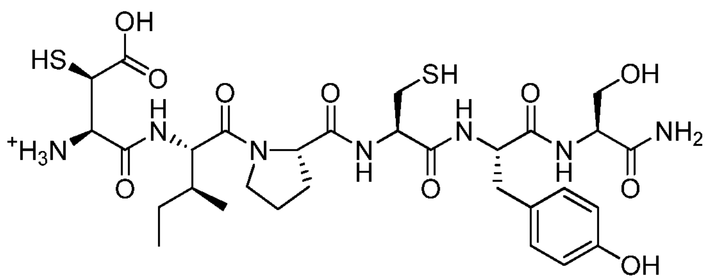

Peptide **27** was prepared according to Fmoc-strategy SPPS outlined in the general procedures and purified by preparative reverse phase HPLC (0 to 30% B over 40 min, 0.1% formic acid) to afford the target compound as a colourless solid following lyophilization (4.0 mg, 52% yield based on the original 10 μmol resin loading).

Analytical HPLC: $R_t$ 29.2 min (0-30% B over 40 min, 0.1% TFA, λ = 230 nm); Calculated Mass $[M+H]^+$: 728.3; Mass Found ($ESI^+$); 728.4 $[M+H]^+$.

**Ligation Reaction General Protocol**

Model peptide thioesters (Ac-LYRANX-$S(CH_2)_2CO_2Et$, X = G, A, M, F, V) **(9-13)** were prepared according to literature methods.[2]

Peptide thioesters (1.30-1.40 eq.) were dissolved in degassed buffer : 6 M guanidine hydrochloride, 200 mM 4-(2-hydroxyethyl)-1-piperazineethanesulfonate (HEPES) buffer, 50 mM tris-(2-carboxyethylphosphine (TCEP), adjusted to pH 7.4-7.5, 5 mM concentration based on the *N*-terminal β(SH)-peptide fragment. The solution was added to the peptide **8** (1.0 eq.) and thiophenol (2% v/v) was added to the solution and the reaction gently agitated. The final pH of the solution was measured and adjusted to 7.3-7.5, using 2 M NaOH or 1 M HCl solution, if necessary. The solution was flushed with argon and incubated at 37 °C. The progress of the reaction was monitored by LC-MS. Upon completion, the reaction was quenched by the addition of 1% TFA in water (0.5 mL) and immediately purified by reverse-phase HPLC employing a mobile phase of 0.1% TFA in water (Solvent A) and 0.1% TFA in acetonitrile (Solvent B) using a linear gradient of 0-50% B over 40 min. Ligation products were isolated as colourless solid TFA salts following lyophilization.

**Model Peptide Ligations**

**Ac-LYRANGD(β-SH)SPGYS-$NH_2$ (S6)**

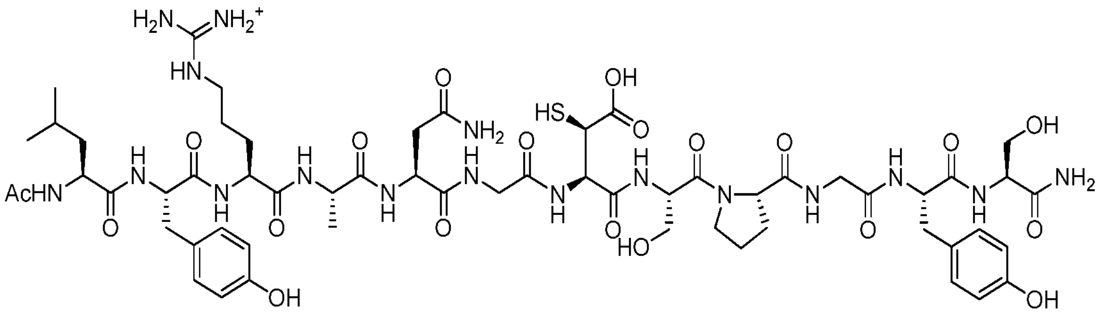

Native chemical ligation of H-(β-SH)DSPGYS-$NH_2$ **(8)** (3.0 mg, 3.9 μmol) and Ac-LYRANG-$S(CH_2)_2CO_2Et$ **(9)** (4.5 mg, 4.7 μmol) was performed as outlined in the general procedures. Purification *via* preparative reverse phase HPLC (0 to 50% B over 40 min, 0.1% TFA) followed by lyophilization afforded the title compound as a colourless solid (4.6 mg, 80% yield).

Analytical HPLC (purified ligation product): $R_t$ 27.6 min (0-50% B over 40 min, 0.1% TFA, λ = 220 nm); Calculated Mass $[M+H]^+$: 1372.6 (100%), 1373.6 (62.7%) $[M+2H]^{2+}$: 686.8 (100%), 687.3 (62.7%); Mass Found ($ESI^+$); 1372.7 $[M+H]^+$, 687.2 $[M+2H]^{2+}$.

**Ac-LYRANAD(β-SH)SPGYS-$NH_2$ (S7)**

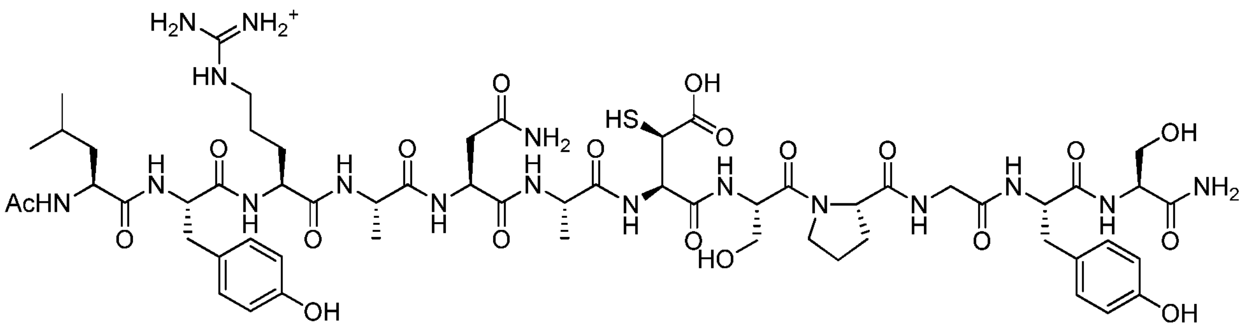

Native chemical ligation of H-(β-SH)DSPGYS-$NH_2$ **(8)** (3.0 mg, 3.9 μmol) and Ac-LYRANA-$S(CH_2)_2CO_2Et$ **(10)** (4.6 mg, 4.7 μmol) was performed as outlined in the general procedures. Purification *via* preparative reverse phase

HPLC (0 to 50% B over 40 min, 0.1% TFA) followed by lyophilization afforded the title compound as a colourless solid (4.8 mg, 82% yield).

Analytical HPLC (purified ligation product): $R_t$ 24.9 min (0-50% B over 40 min, 0.1% TFA, $\lambda$ = 220 nm); Calculated Mass $[M+H]^+$: 1386.6 (100%), 1387.6 (70.9%), $[M+2H]^{2+}$: 693.8 (100%), 694.3 (71.7%); Mass Found ($ESI^+$); 1386.7 $[M+H]^+$, 694.2 $[M+2H]^{2+}$.

**Ac-LYRANMD(β-SH)SPGYS-$NH_2$ (S8)**

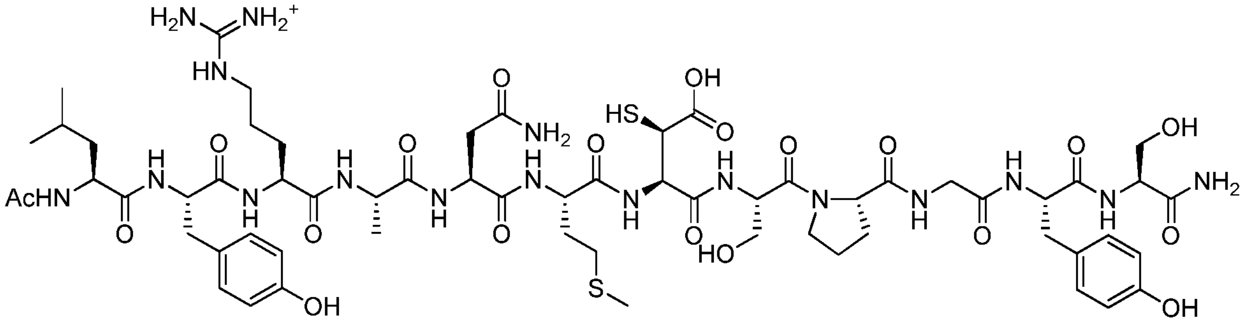

Native chemical ligation of H-(β-SH)DSPGYS-$NH_2$ **(8)** (3.0 mg, 3.9 μmol) and Ac-LYRANM-$S(CH_2)_2CO_2Et$ **(11)** (4.9 mg, 4.7 μmol) was performed as outlined in the general procedures. Purification *via* preparative reverse phase HPLC (0 to 50% B over 40 min, 0.1% TFA) followed by lyophilization afforded the title compound as a colourless solid (4.3 mg, 71% yield).

Analytical HPLC (purified ligation product): $R_t$ 26.5 min (0-50% B over 40 min, 0.1% TFA, $\lambda$ = 220 nm); Calculated Mass $[M+H]^+$: 1446.6 (100%), 1447.6 (72.5%), $[M+2H]^{2+}$: 723.8 (100%), 724.3 (67.8%); Mass Found ($ESI^+$); 1446.7 $[M+H]^+$, 724.2 $[M+2H]^{2+}$.

**Ac-LYRANFD(β-SH)SPGYS-$NH_2$ (S9)**

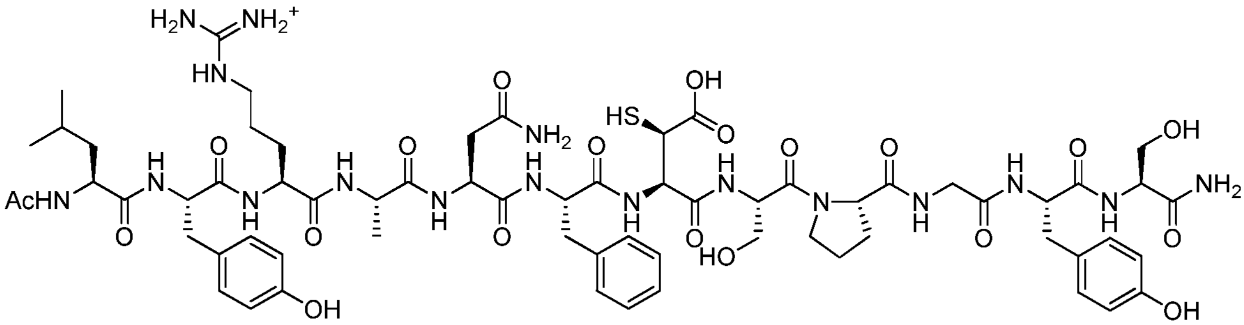

Native chemical ligation of H-(β-SH)DSPGYI-$NH_2$ **(8)** (3.0 mg, 3.9 μmol) and Ac-LYRANF-$S(CH_2)_2CO_2Et$ **(12)** (5.0 mg, 4.7 μmol) was performed as outlined in the general procedures. Purification *via* preparative reverse phase HPLC (0 to 50% B over 40 min, 0.1% TFA) followed by lyophilization afforded the title compound as a colourless solid (4.8 mg, 78% yield).

Analytical HPLC (purified ligation product): $R_t$ 30.2 min (0-50% B over 40 min, 0.1% TFA, $\lambda$ = 230 nm); Calculated Mass $[M+H]^+$: 1462.6 (100%), 1463.6 (70.3%), $[M+2H]^{2+}$: 731.8 (100%), 732.3 (70.3%); Mass Found ($ESI^+$); 732.1 $[M+2H]^{2+}$, 1463.8 $[M+H]^+$.

**Ac-LYRANVD(β-SH)SPGYS-$NH_2$ (S10)**

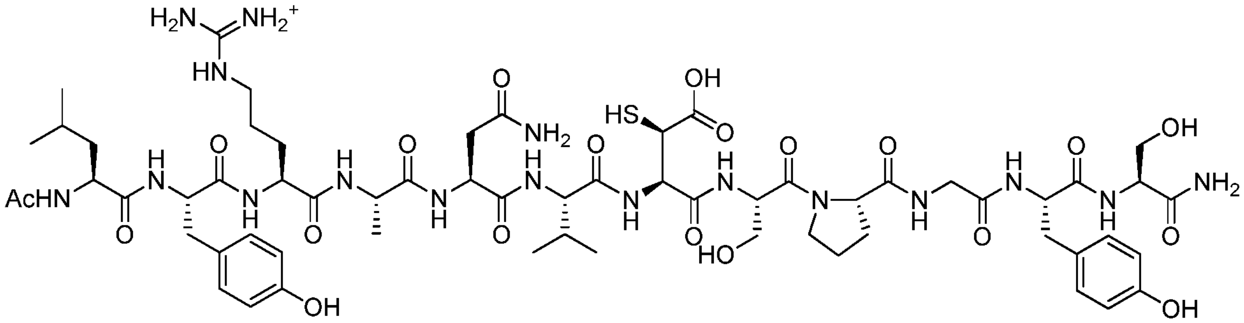

Native chemical ligation of H-(β-SH)DSPGYS-$NH_2$ **(8)** (3.0 mg, 3.9 μmol) and Ac-LYRANV-$S(CH_2)_2CO_2Et$ **(13)** (4.7 mg, 4.7 μmol) was performed as outlined in the general procedures. Purification *via* preparative reverse phase HPLC (0 to 50% B over 40 min, 0.1% TFA) followed by lyophilization afforded the title compound as a colourless solid (4.5 mg, 75% yield).

Analytical HPLC (purified ligation product): $R_t$ 25.6 min (0-50% B over 40 min, 0.1% TFA, λ = 230 nm); Calculated Mass $[M+H]^+$: 1414.6 (100%), 1415.6 (66%), $[M+2H]^{2+}$: 707.8 (100%), 708.3; Mass Found ($ESI^+$); 1414.7 $[M+H]^+$, 708.2 $[M+2H]^{2+}$.

## Kinetic Studies

Ligation time-courses were plotted for the reaction of compound **8** (H-(β-SH)DSPGYS-$NH_2$) with Ac-LYRANX-$S(CH_2)_2CO_2Et$ (X = G, A, F, S, V). Ligation experiments were carried out as outlined in the general methods at pH = 7.4. Aliquots of 5 μL were taken from the reaction mixture at various time intervals and quenched with 45 μL of 1% TFA in water and analyzed by means of analytical HPLC. Conversion estimations are based upon the relative peak areas of the thiol-containing starting material versus the desired ligation product at λ = 280 nm, taking into account the corresponding extinction coefficients based on the presence of tyrosine residues ($\varepsilon_{280}$(peptide thioester) = $\varepsilon_{280}$(thiol-containing peptide) = 1280 $M^{-1}cm^{-1}$; $\varepsilon_{280}$(ligation product) = 2560 $M^{-1}cm^{-1}$). Results are shown in Fig. 5.

## Radical Desulfurization Reaction General Protocol

*Desulfurization General Protocol:* A solution of peptide in buffer (6 M guanidine hydrochloride, 200 mM HEPES, 250 mM TCEP, adjusted to pH 6.5-7.0, 2.5 mM concentration of peptide) was degassed with argon gas for 10 min. To this was added sequentially glutathione (final conc. 40 mM) and 2,2'-azobis[2-(2-imidazolin-2-yl)propane]dihydrochloride (VA-044), final conc. 20 mM) in solid form. The solution was sparged with argon gas for a further 2 min, aiding dissolution of the reagents. The reaction vessel was then incubated at 37 °C for 16 h. The reaction was diluted with 0.1% TFA in water (1 mL) and immediately purified by reverse-phase HPLC employing a mobile phase of 0.1% TFA in water (Solvent A) and 0.1% TFA in acetonitrile (Solvent B) using a linear gradient of 0-50% B over 40 min. Desulfurization products were isolated as colourless solid TFA salts following lyophilization.

### Ac-LYRANGDSPGYS-$NH_2$ (S11)

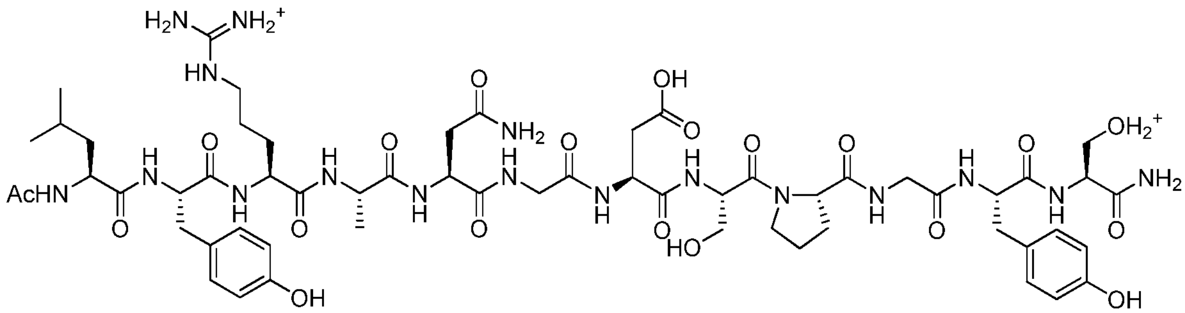

Desulfurization of Ac-LYRANGD(β-SH)SPGYS-$NH_2$ **(S6)** (2.5 mg, 1.7 μmol) was carried out according to the general procedure. Purification *via* preparative reverse phase HPLC (0 to 50% B over 40 min, 0.1% TFA) followed by

lyophilization afforded the title compound as a colourless solid (1.9 mg, 75% yield).

Analytical HPLC: $R_t$ 23.3 min (0-50% B over 40 min, 0.1% TFA, $\lambda$ = 230 nm); Calculated Mass $[M+H]^+$: 1340.6 (100%), 1341.6 (64.5%), $[M+2H]^{2+}$: 670.8 (100%), 671.3 (69.0%); Mass Found ($ESI^+$); 1340.7 $[M+H]^+$, 671.1 $[M+2H]^{2+}$.

**Ac-LYRANADSPGYS-$NH_2$ (S12)**

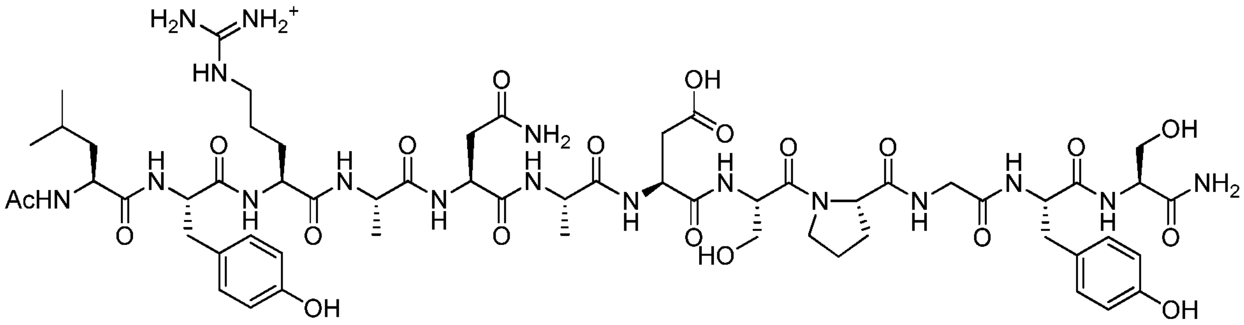

Desulfurization of Ac-LYRANAD($\beta$-SH)SPGYS-$NH_2$ **(S7)** (2.5 mg, 1.7 $\mu$mol) was carried out according to the general procedures. Purification *via* preparative reverse phase HPLC (0 to 50% B over 40 min, 0.1% TFA) followed by lyophilization afforded the title compound as a colourless solid (1.8 mg, 71% yield).

Analytical HPLC: $R_t$ 24.1 min (0-50% B over 40 min, 0.1% TFA, $\lambda$ = 230 nm); Calculated Mass $[M+H]^+$: 1354.6 (100%), 1355.6 (70.9%), $[M+2H]^{2+}$: 677.8 (100%), 678.3 (65.6%); Mass Found ($ESI^+$); 1354.7 $[M+H]^+$, 678.1 $[M+2H]^{2+}$.

**Ac-LYRANMDSPGYS-$NH_2$ (S13)**

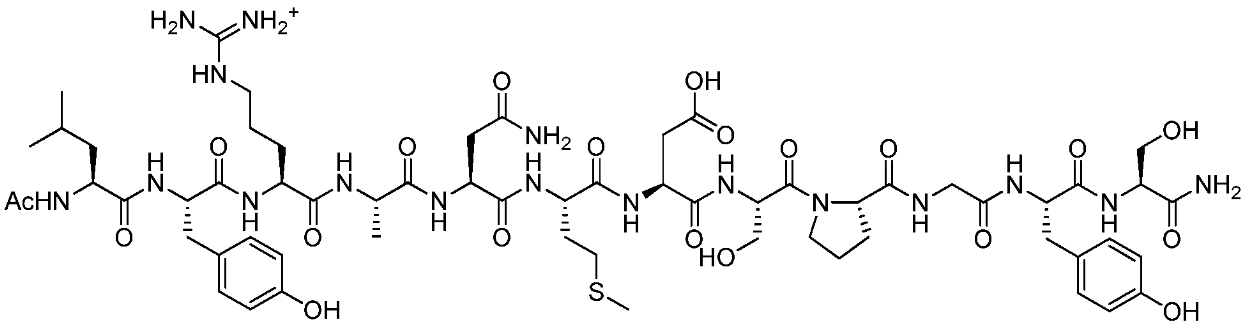

Desulfurization of Ac-LYRANMD($\beta$-SH)SPGYS-$NH_2$ **(S8)** (2.5 mg, 1.6 $\mu$mol) was carried out according to the general procedures. Purification *via* preparative reverse phase HPLC (0 to 50% B over 40 min, 0.1% TFA) followed by lyophilization afforded the title compound as a colourless solid (1.6 mg, 63% yield).

Analytical HPLC: $R_t$ 25.2 min (0-50% B over 40 min, 0.1% TFA, $\lambda$ = 230 nm); Calculated Mass $[M+H]^+$: 1414.6 (100%), 1415.6 (66.0%), $[M+2H]^{2+}$: 707.8 (100%), 708.3 (66.0%); Mass Found ($ESI^+$); 1414.7 $[M+H]^+$, 708.2 $[M+2H]^{2+}$.

**Ac-LYRANFDSPGYS-$NH_2$ (S14)**

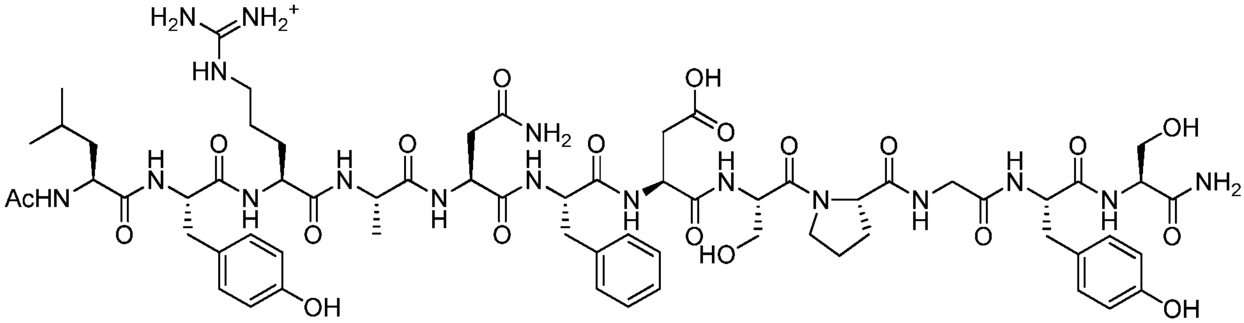

Desulfurization of Ac-LYRANFD(β-SH)SPGYS-$NH_2$ **(S9)** (2.5 mg, 1.6 μmol) was carried out according to the general procedures. Purification *via* preparative reverse phase HPLC (0 to 50% B over 40 min, 0.1% TFA) followed by lyophilization afforded the title compound as a colourless solid (1.9 mg, 76% yield).

Analytical HPLC: $R_t$ 27.3 min (0-50% B over 40 min, 0.1% TFA, λ = 230 nm); Calculated Mass $[M+H]^+$: 1430.7(100%), 1431.7 (70.3%), $[M+2H]^{2+}$: 715.8 (100%), 716.3 (78.4%); Mass Found ($ESI^+$); 1430.8 $[M+H]^+$, 716.2 $[M+2H]^{2+}$.

**Ac-LYRANVDSPGYS-$NH_2$ (S15)**

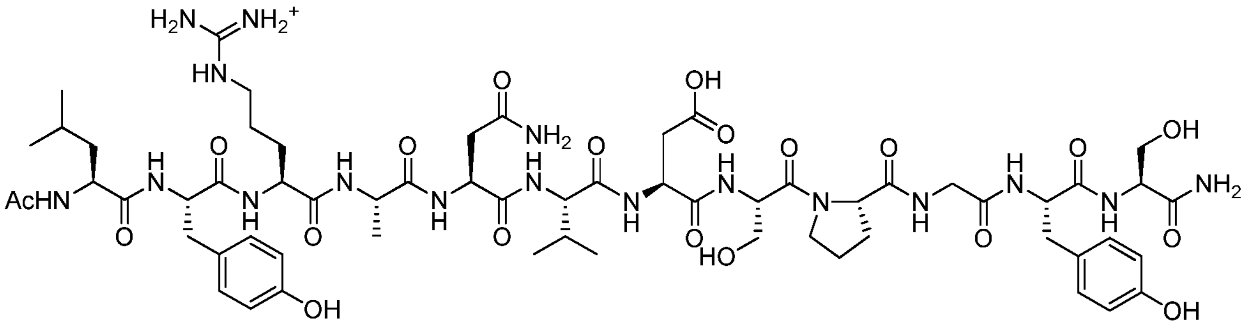

Desulfurization of Ac-LYRANVD(β-SH)SPGYS-$NH_2$ (**S10**) (2.5 mg, 1.6 μmol) was carried out according to the general procedures. Purification *via* preparative reverse phase HPLC (0 to 50% B over 40 min, 0.1% TFA) followed by lyophilization afforded the title compound as a colourless solid (1.7 mg, 71% yield).

Analytical HPLC: $R_t$ 24.7 min (0-50% B over 40 min, 0.1% TFA, λ = 230 nm). Calculated Mass $[M+H]^+$: 1382.7 (100%), 1383.7 (66.0%), $[M+2H]^{2+}$: 691.8 (100%), 692.3 (66.0%); Mass Found ($ESI^+$); 1382.7 $[M+H]^+$, 692.2 $[M+2H]^{2+}$.

**One-pot Ligation selective desulfurization reactions**

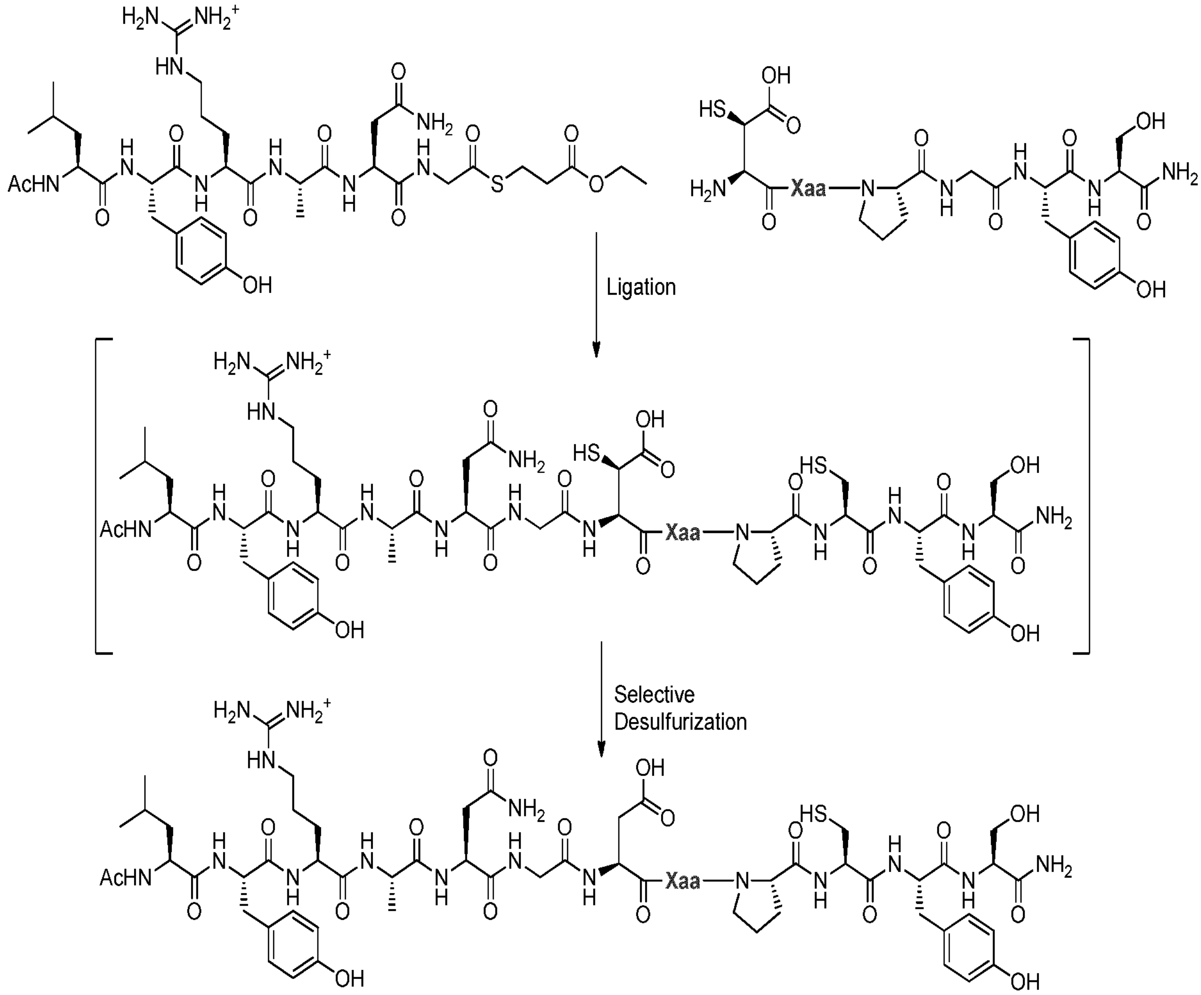


*General Protocol*: Ac-LYRANG-S$(CH_2)_2CO_2Et$ **(9)** (1.20-1.30 eq.) was dissolved in degassed buffer (6 M guanidine hydrochloride, 200 mM HEPES, 50 mM TCEP, adjusted to pH 7.4-7.5, 5 mM concentration based on the *N*-terminal mercaptoaspartyl-peptide fragment). The solution was added to the thiol-containing peptide (**17, 19-27**) (~2 mg, 1.0 eq.) in an Eppendorf tube. Thiophenol (2% v/v) was added to the solution and the reaction gently agitated. The final pH of the solution was measured and adjusted to 7.3-7.5, using 2 M NaOH or 1 M HCl solution, if necessary. The solution was flushed with argon and incubated at 37 °C. After 2 h, thiophenol was extracted into $Et_2O$ (0.5 mL, free of peroxides) which was carefully separated from the ligation buffer. After 4 further extractions the aqueous buffer was degassed with argon for 10 min. The solution was then diluted with a solution of TCEP.HCl (0.45 M) and dithiothreitol (0.1 M) in degassed buffer (6 M guanidine hydrochloride, 200 mM HEPES, final pH 2.8-3.0) to give final concentrations of peptide (2.5 mM), TCEP (250 mM) and dithiothreitol (0.5 M) and a pH of 3.0. The solutions were incubated at 65 °C for 20 h after which time the ligated peptide had been consumed. A solution of 0.1% TFA in water (0.5 mL) was added and the crude mixtures were purified by reverse-phase HPLC employing a mobile phase of 0.1% TFA in water (Solvent A) and 0.1% TFA in acetonitrile (Solvent B) using a linear gradient of 0-50% B over 100 min.

**Ac-LYRANGDAPCYS-$NH_2$ (18)**

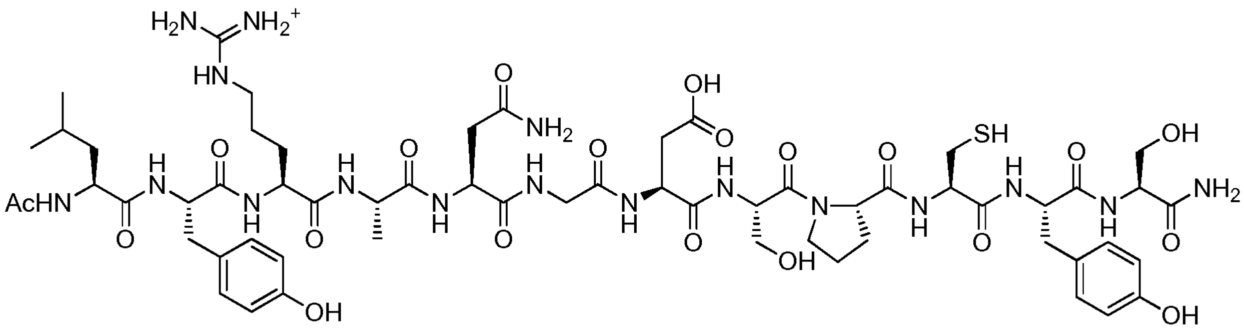

Peptide **17** (1.4 mg, 1.7 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 40 min, 0.1% TFA) followed by lyophilization afforded the title compound **(18)** as a colourless solid (1.2 mg, 48% yield).

Analytical HPLC: $R_t$ 24.5 min (0-50% B over 40 min, 0.1% TFA, λ = 230 nm). Calculated Mass $[M+H]^+$: 1386.6 (100%), 1387.6 (67%), $[M+2H]^{2+}$: 693.8 (100%), 694.3 (63.8%); Mass Found ($ESI^+$); 693.5 $[M+2H]^{2+}$.

**Ac-LYRANGDGPCYS-$NH_2$ (28)**

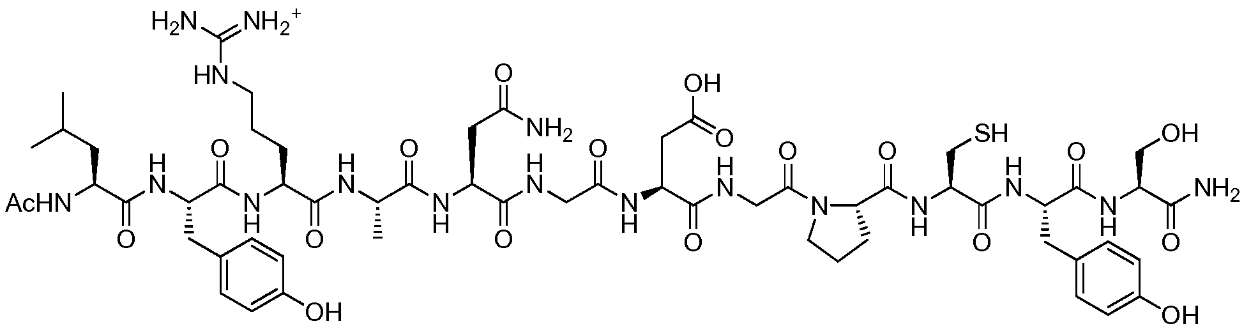

Peptide **19** (2.3 mg, 2.9 μmol) was ligated to Ac-LYRANG-S($CH_2$)$CO_2$Et **(9)** and desulfurized in one-pot according to the general procedure. HPLC-MS analysis indicated near quantitative decomposition of product peptide **28.**

**Ac-LYRANGDPPCYS-$NH_2$ (29)**

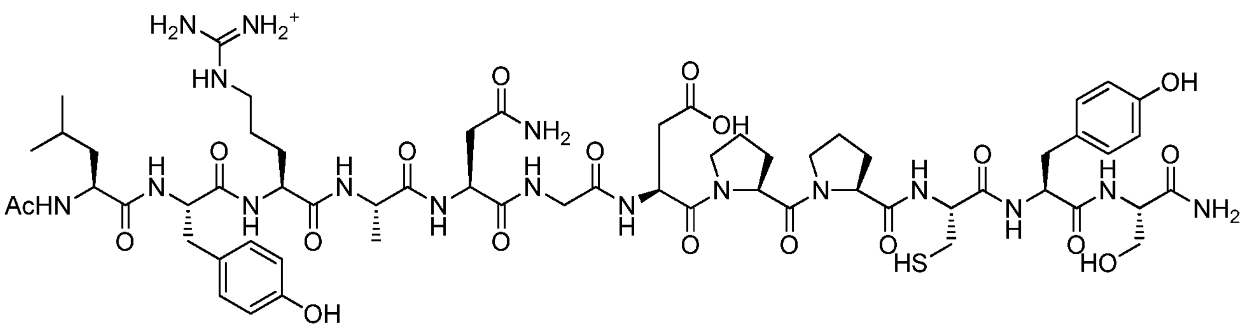

Peptide **20** (1.8 mg, 2.1 μmol) was ligated to Ac-LYRANG-S$(CH_2)_2CO_2$Et **(9)** and desulfurized in one-pot according to the general procedure. HPLC-MS analysis indicated quantitative decomposition of product peptide **29.**

**Ac-LYRANGDAPCYS-$NH_2$ (30)**

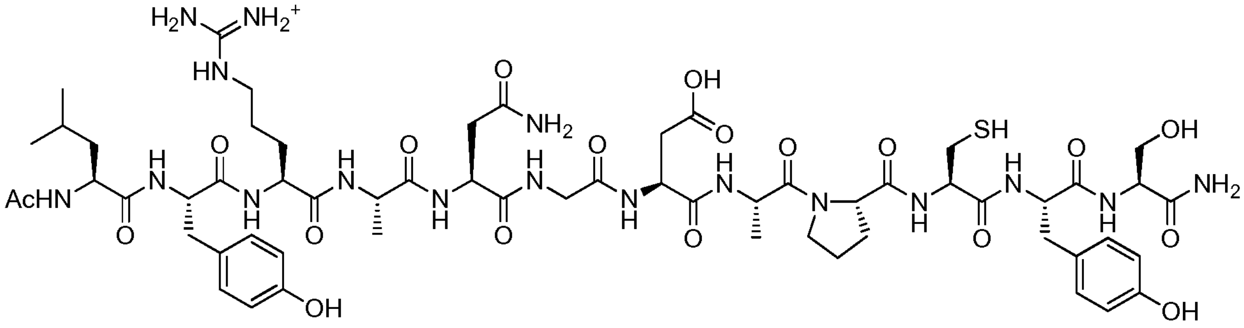

Peptide **21** (2.2 mg, 2.8 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 100 min, 0.1% TFA) followed by lyophilization afforded the title compound **(30)** as a colourless solid (1.9 mg, 45% yield).

Analytical HPLC: $R_t$ 24.9 min (0-50% B over 40 min, 0.1% TFA, λ = 230 nm). Calculated Mass $[M+H]^+$: 1370.6 (100%), 1371.6 (63.8%), $[M+2H]^{2+}$: 685.8 (100%), 686.3 (63.8%); Mass Found ($ESI^+$); 1370.6 $[M+H]^+$, 686.2 $[M+2H]^{2+}$.

**Ac-LYRANGDHPCYS-$NH_2$ (31)**

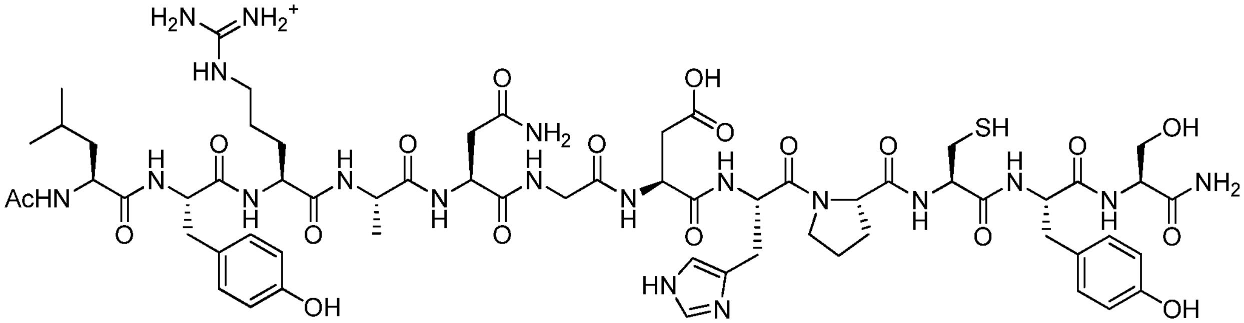

Peptide **22** (3.2 mg, 3.3 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 40 min, 0.1% TFA) followed by lyophilization afforded the title compound **(31)** as a colourless solid (3.2 mg, 59% yield).

Analytical HPLC: $R_t$ 24.0 min (0-50% B over 40 min, 0.1% TFA, λ = 230 nm). Calculated Mass $[M+H]^+$: 1436.6 (100%), 1437.6 (67.1%), $[M+2H]^{2+}$: 718.8 (100%), 719.3 (67.1%); Mass Found ($ESI^+$); 718.4 $[M+2H]^{2+}$.

**Ac-LYRANGDKPCYS-$NH_2$ (32)**

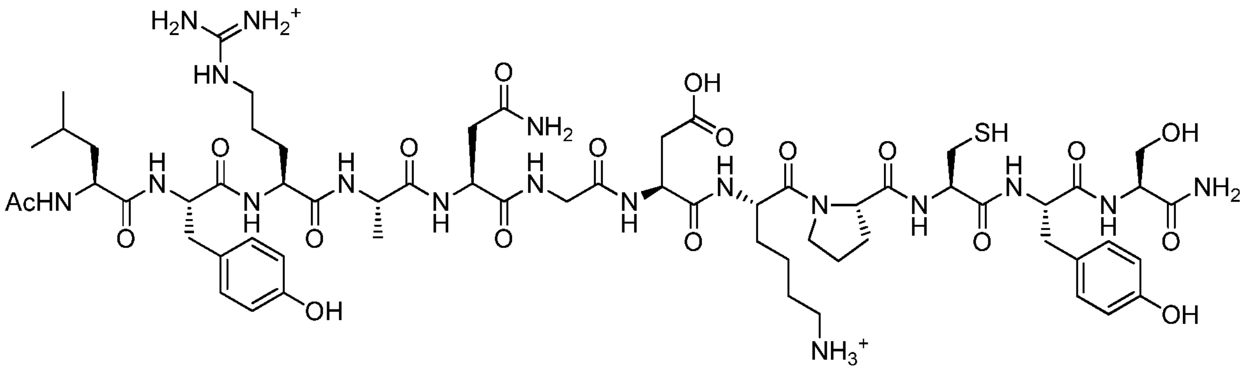

Peptide **23** (3.0 mg, 3.1 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 40 min, 0.1% TFA) followed by lyophilization afforded the title compound **(32)** as a colourless solid (2.4 mg, 47% yield).

Analytical HPLC: $R_t$ 23.8 min (0-50% B over 40 min, 0.1% TFA, $\lambda$ = 230 nm). Calculated Mass $[M+2H]^{2+}$: 714.3 (100%), 714.8 (67.1%); Mass Found ($ESI^+$); 714.8 $[M+2H]^{2+}$.

**Ac-LYRANGDEPCYS-$NH_2$ (33)**

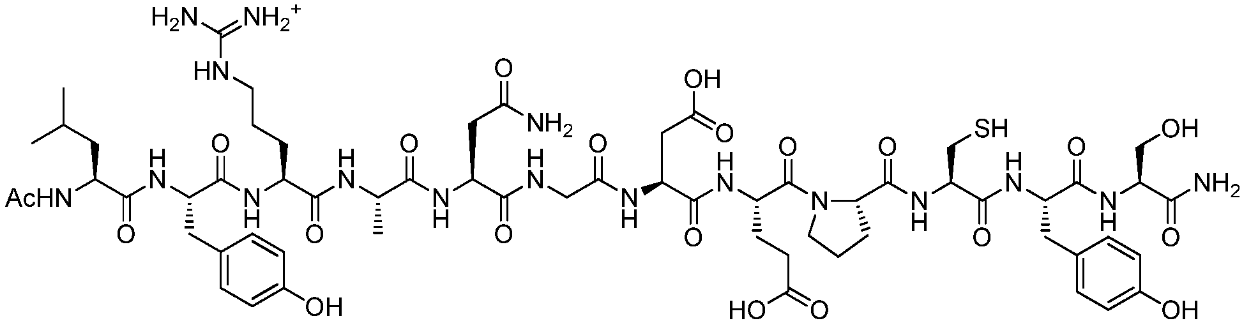

Peptide **24** (3.0 mg, 3.5 μmol, TFA salt) was ligated to Ac-LYRANG-$S(CH_2)CO_2Et$ **(9)** and desulfurized in one-pot according to the general procedure. Purification *via* preparative reverse phase HPLC (0 to 50% B over 40 min, 0.1% TFA) followed by lyophilization afforded the title compound **(33)** as a colourless solid (3.1 mg, 57% yield).

Analytical HPLC: $R_t$ 25.1 min (0-50% B over 40 min, 0.1% TFA, $\lambda$ = 230 nm). Calculated Mass $[M+H]^+$: 1428.6 (100%), 1429.6 (66.0%), $[M+2H]^{2+}$: 714.8 (100%), 715.3 (66.0%); Mass Found ($ESI^+$); 1428.5 $[M+H]^+$, 715.2 $[M+2H]^{2+}$, 489.9 $[M+2H+K]^{3+}$.

**Ac-LYRANGDNPCYS-$NH_2$ (34)**

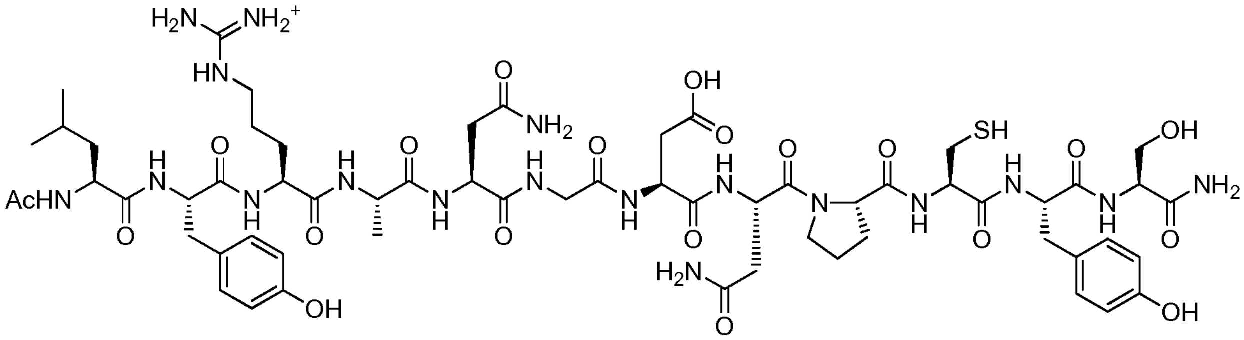

Peptide **25** (3.1 mg, 3.7 μmol, TFA salt) was ligated to Ac-LYRANG-$S(CH_2)CO_2Et$ **(9)** and desulfurized in one-pot according to the general procedure. Purification *via* preparative reverse phase HPLC (0 to 50% B over 40 min, 0.1% TFA) followed by lyophilization afforded the title compound **(34)** as a colourless solid (2.8 mg, 50% yield).

Analytical HPLC: $R_t$ 25.0 min (0-50% B over 40 min, 0.1% TFA, $\lambda$ = 230 nm). Calculated Mass $[M+H]^+$: 1413.6 (100%), 1414.6 (64.9%), $[M+2H]^{2+}$: 707.3 (100%), 707.8 (64.9%); Mass Found ($ESI^+$); 1413.5 $[M+H]^+$, 707.8 $[M+2H]^{2+}$, 484.9 $[M+2H+K]^{3+}$.

**Ac-LYRANGDFPCYS-$NH_2$ (35)**

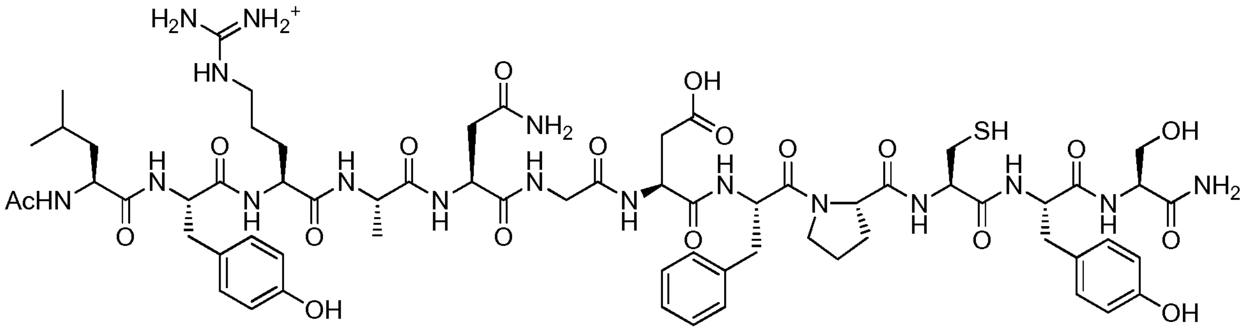

Peptide **26** (2.0 mg, 2.3 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 40 min, 0.1% TFA) followed by lyophilization afforded the title compound **(35)** as a colourless solid (2.3 mg, 63% yield).

Analytical HPLC: $R_t$ 28.9 min (0-50% B over 40 min, 0.1% TFA, λ = 230 nm). Calculated Mass $[M+H]^+$: 1446.6 (100%), 1447.7 ($[M+2H]^{2+}$: 723.8 (100%), 714.8 (67.1%); Mass Found ($ESI^+$); 1446.3 $[M+H]^+$, 723.8 $[M+2H]^{2+}$.

**Ac-LYRANGDIPCYS-$NH_2$ (36)**

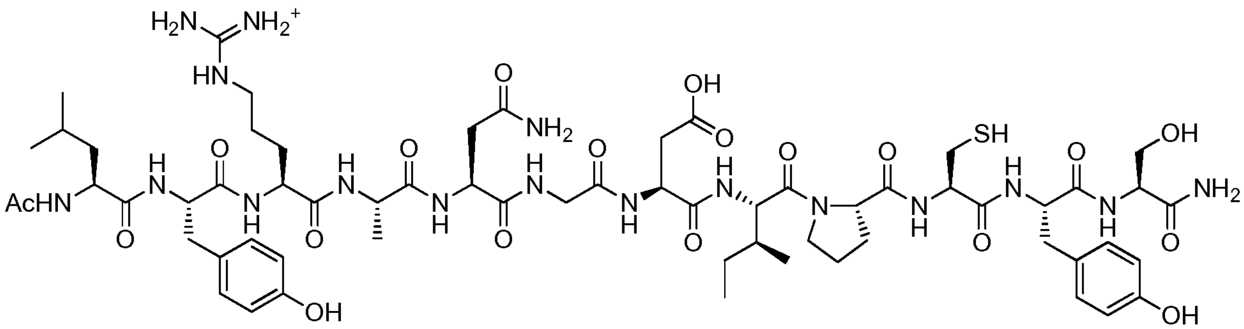

Peptide **27** (5.2 mg, 6.2 μmol, TFA salt) was ligated to Ac-LYRANG-S($CH_2$)$CO_2$Et **(9)** and desulfurized in one-pot according to the general procedure. Purification *via* preparative reverse phase HPLC (0 to 50% B over 40 min, 0.1% TFA) followed by lyophilization afforded the title compound **(36)** as a colourless solid (5.5 mg, 58% yield).

Analytical HPLC: $R_t$ 27.6 min (0-50% B over 40 min, 0.1% TFA, λ = 230 nm). Calculated Mass $[M+H]^+$: 1412.6 (100%), 1413.6 (67.1%), $[M+2H]^{2+}$: 706.8 (100%), 707.3 (67.1%); Mass Found ($ESI^+$); 1412.6 $[M+H]^+$, 706.3 $[M+2H]^{2+}$, 484.6 $[M+2H+K]^{3+}$.

**pH Dependence of Selective Desulfurization**

The intermediate ligation product between peptide H-D(SH)SPCYS-NH2 **(17)** and Ac-LYRANG-S($CH_2$)$CO_2$Et **(9)** was subjected to the selective desulfurization conditions outlined above at varied pH. After 20 h, the products were analysed by HPLC-MS.

**Synthesis of CXCR4(1-38) (37)**

Figure 6 shows retrosynthesis of CXCR4(1-38) **(37)** providing target peptide **38** and peptide thioester **39.**

**CXCR4(1-19) (38)**

Fig. 7 shows a scheme of the synthesis of thioester 38. 2-Chloro-trityl chloride resin was loaded with Fmoc-Gly-OH to give resin bound **S16** (12.5 μmol, 0.5 mmol/g). The peptide was elongated using standard Fmoc-SPPS procedures. and previously outlined coupling conditions for glyosylamino acid **S2** which was prepared as previously reported. The fully assembled resin-bound glycopeptide **S17** was *O*-deacetylated on-resin to afford **S18,** and then cleaved from the resin using HFIP/$CH_2Cl_2$ (4:1 v/v, 4×4mL×20 min) to give crude protected glycopeptide peptide **S19.** The

crude residue was placed under an atmosphere of argon, dissolved in dry DMF (3 mL) at room temperature. The mixture was treated with ethyl-3-mercaptopropionate (47 μL, 375 μmol, 30 equiv.) and *i*$Pr_2NEt$ (11 μL, 62.5 μmol, 5 equiv.), followed by PyBOP (32.5 mg, 62.5 μmol, 5 equiv.) and let stir for 2.5 h. The solvent was then removed under a stream of nitrogen and the residue dried thoroughly under vacuum. After removal of all traces of DMF, the crude mixture was cooled to 0 °C and treated with a solution of TFA/tri*iso*propylsilane/$H_2O$/thioanisole (90:5:2.5:2.5 v/v/v/v). The reaction was stirred at rt for 2 h and concentrated *in vacuo.* Crude peptide **38** was precipitated in cold diethyl ether, and purified by reverse-phase semi-preparative HPLC (0-40% B over 40 min, A 0.1% formic acid) to yield the pure glycopeptide thioester **38** as a colourless solid following lyophilization (3.1 mg, 10% yield based on original resin loading).

Analytical HPLC: $R_t$ 26.3 min (0-50% B over 40 min, A = 0.1 M $NH_4OAc$, B = MeCN, λ = 230 nm). HRMS $[M+Na]^+$: 2425.9409; Mass Found ($ESI^+$); 2425.9411 $[M+Na]^+$.

**CXCR4(20-38)**

Figure 8 shows synthesis of compound (39). The peptide was elongated using standard Fmoc-SPPS procedures on Rink amide resin (12.5 μmol) incorporating Fmoc-Tyr($SO_3$nP)-OH **(S4)** which was prepared as previously reported. After coupling of **1** to the N-terminus, the fully protected resin-bound peptide **S21** peptide cleaved and deprotected using a solution of TFA/tri*iso*propylsilane/$H_2O$/thioanisole (90:5:2.5:2.5 v/v/v/v). The reaction was agitated at rt for 2 h and concentrated *in vacuo.* Crude peptide **37** was precipitated from cold diethyl ether, and the crude product purified by reverse-phase preparative HPLC (0-40% B over 40 min, 0.1% TFA) to yield peptide **37** as a colourless solid following lyophilization (12.0 mg, 32% yield based on original resin loading).

Analytical HPLC: $R_t$ 31.4 min (0-50% B over 40 min, 0.1% TFA, λ = 230 nm). HRMS (ESI) (internal disulfide): Calculated $[M+3H]^{3+}$: 839.6721; Mass Found ($ESI^+$); 839.6725 $[M+3H]^{3+}$.

**CXCR4(1-38)**

Figure 9 shows a sckene for coupling of compounds 39 and 38 to produce compound 37. Glycopeptide thioester **39** (3.0 mg, 1.2 μmol) was dissolved in 6M Gn.HCl/200 mM HEPES buffer containing 50 mM TCEP (pH 7.4-7.5, 200 μL, 6 mM concentration of thioester) and added to sulfopeptide **37** (3.0 mg, 1.0 μmol, loss of the neopentyl group occurred immediately). Thiophenol (4 μL, 2 vol.%) was added and the mixture was incubated at 37 °C for 16 h after which time LC-MS analysis indicated consumption of starting materials. Thiophenol was then removed through extraction of the reaction mixture with diethyl ether (5 × 0.5 mL). The aqueous solution containing the ligation product was then diluted with 200 μL of 6M Gn.HCl/200 mM HEPES containing TCEP.HCl (450 mM) and DTT (100 mM), final pH 3.0. The solution was then sparged with argon gas for 5 min and then incubated at 65 °C for 24 h. The reaction mixture was immediately purified by reverse-phase HPLC (0 to 30% B over 60 min, A = 0.1 M $NH_4OAc_{(aq)}$ B = MeCN) to afford **37** as a colourless solid after lyophilization (1.0 mg, 20% yield).

Analytical HPLC: $R_t$ 23.7 min (0-50% B over 40 min, A = 0.1 M $NH_4OAc$, B = MeCN, λ = 230 nm). HRMS (MALDI): Calculated Mass $[M+H]^+$ 4685.87740; Mass Found ($ESI^+$); 765.88194 $[M+H]^+$

**Example 2**

This example describes an efficient methodology for ligation at glutamate (Glu). A γ-thiol-Glu building block was accessed in only three steps from protected glutamic acid and could be incorporated at the N-terminus of peptides. The application of these peptides in one-pot ligation-desulfurization chemistry is demonstrated with a range of peptide thioesters. The synthetic route is illustrated below.

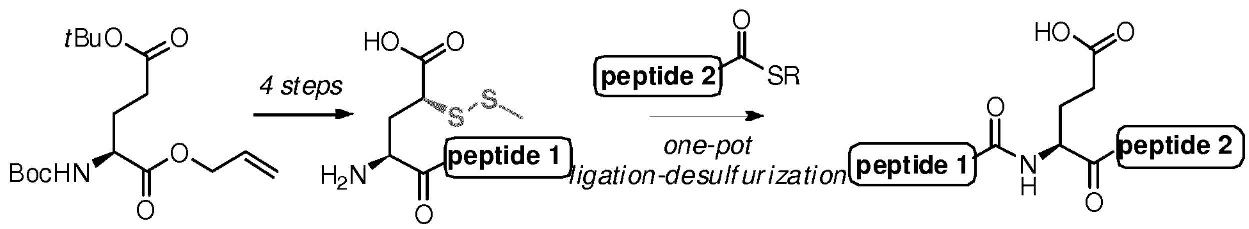


This synthesis proceeds through a short and scalable route, and is useful in the peptide ligation-desulfurization chemistry described elsewhere herein. Although this reaction would proceed through a six-membered ring during the S to N acyl shift, owing to the γ-position of the thiol, the inventors envisaged that the ligation reaction would still be facile based on prior ligation studies at homocysteine and other γ-thiol amino acids. Furthermore, conditions for a one-pot

ligation-desulfurization at Glu are described whereby desulfurization can be carried out on the crude ligation reaction, without the need for intermediate purification.

It should be noted that numbering of structures within this example are specific to this example and do not relate to numbering of structures elsewhere in this specification. Synthesis of the initially proposed γ-thiol-Glu building block **1** proceeded from Boc-Glu(O*t*Bu)-OAll **(2)** and began with installation of a 2,4-dimethoxybenzyl (DMB) protected thiol at the γ-position (Scheme 3). This was facilitated by sulfenylating reagent **3** following double deprotonation of **2.** Gratifyingly, the resulting DMB-protected γ-thiol-Glu **4** was isolated in good yield (83%, Scheme 3), and as a single diastereoisomer (>99% *dr*). Finally, **4** was subjected to Pd-catalyzed allyl ester deprotection conditions to afford the desired γ-thiol-Glu building block **1** in excellent yield. As it is known that both diastereomers of β-thiol-Asp facilitate ligation at comparable rates, the inventors focused only on the isolated diastereomer for ligation studies.

**Scheme 3.** Synthesis of γ-thiol-Glu building block **1** and **10**

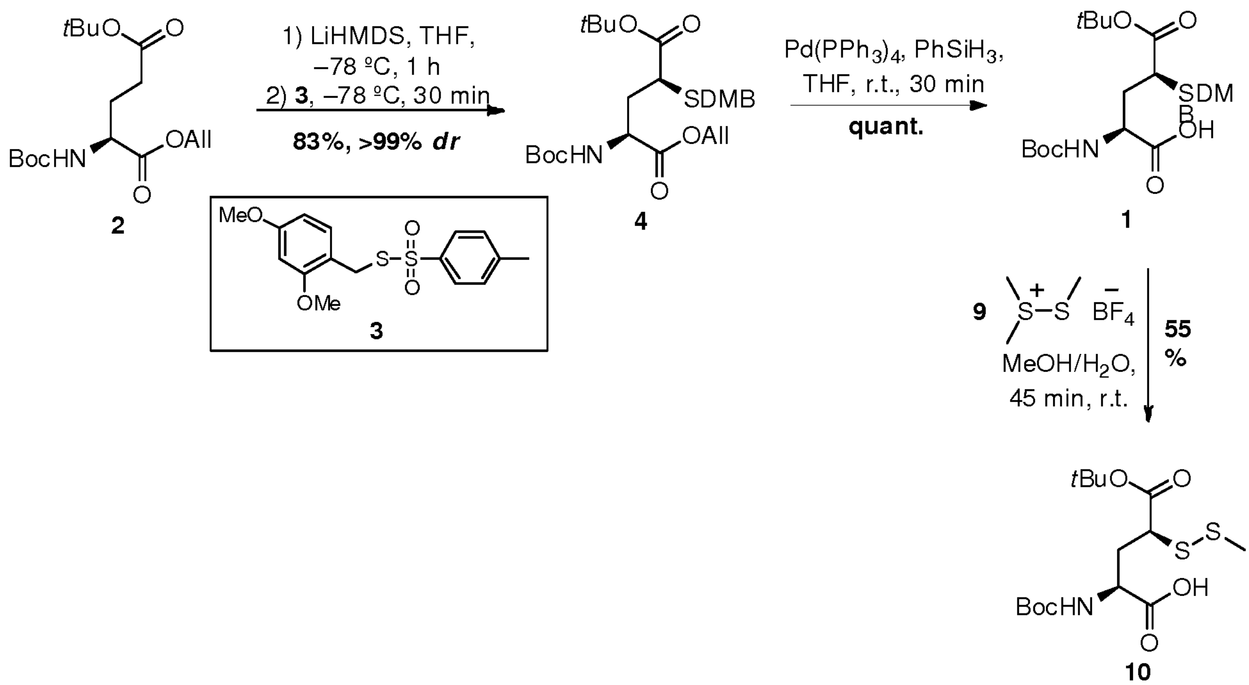


**2** **4** **1** **3** **9** **10**

In order to avoid acid catalyzed peptide bond cleavage, the acid labile DMB-thiol protecting group was exchanged for an acid stable but reductively labile methyl disulfide protecting group (Scheme 3). This transformation was achieved by subjecting DMB-protected γ-thiol-Glu **1** to the reagent dimethyl(methylthio)sulfonium tetrafluoroborate **(9)** which facilitated this protecting group exchange in moderate yield (55%, Scheme 3). Gratifyingly, incorporation of this modified γ-thiol-Glu building block **10** into resin-bound peptide **11** employing (benzotriazol-1-yloxy)tripyrrolidinophosphoniumhexafluorophosphate (PyBOP) and *N*-methylmorpholine (NMM) afforded the desired model peptide **12** in good yield after acidic deprotection and cleavage of the peptide from the resin and HPLC purification (68%, Scheme 4), without any trace of unwanted peptide splicing products.

**Scheme 4.** Synthesis of model peptide **12**

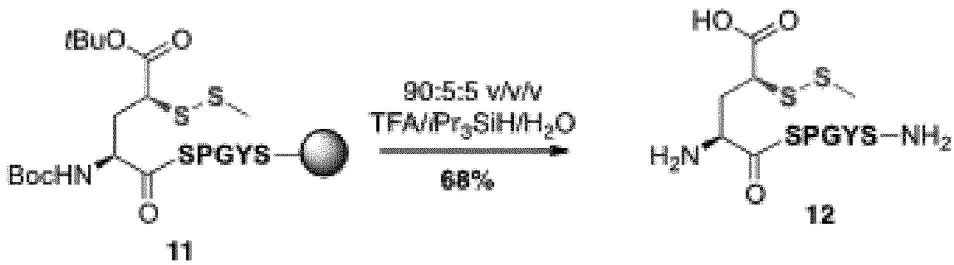


With the desired model peptide **12** in hand, the utility of the N-terminal γ-thiol-Glu moiety in peptide **12** in ligation-desulfurization chemistry was investigated using a variety of *C*-terminal model peptide thioesters to probe the scope of these reactions (entries 1-5, Table 1). Ligation reactions were carried out in ligation buffer (6 M Gn.HCl, 100 mM $Na_2HPO_4$, 50 mM TCEP, 5 mM with respect to **12**) at 37 °C and pH 7.2-7.4 with the addition of thiophenol as an aryl thiol catalyst. Pleasingly, each of the ligation reactions proceeded to completion within 16 hours and in excellent yields after reverse-phase HPLC purification (68%-83%, Table 1). It should be noted that HPLC fractions containing ligation product were immediately lyophilized to avoid acid-mediated peptide splicing caused by the acidic HPLC eluent.

Next the purified ligation products were subjected to radical-based desulfurization using VA-044 (2,2'-Azobis[2-(2-imidazolin-2-yl)propane]dihydrochloride) as the radical initiator, in the presence of TCEP and reduced glutathione. In all cases, desulfurization reactions proceeded to completion within 16 hours at 65 °C, affording the native peptide products in excellent yields after reverse-phase HPLC purification (Table 1, 84%-98% yield).

**Table 1.** Scope of γ-thiol-Glu ligation-desulfurization chemistry

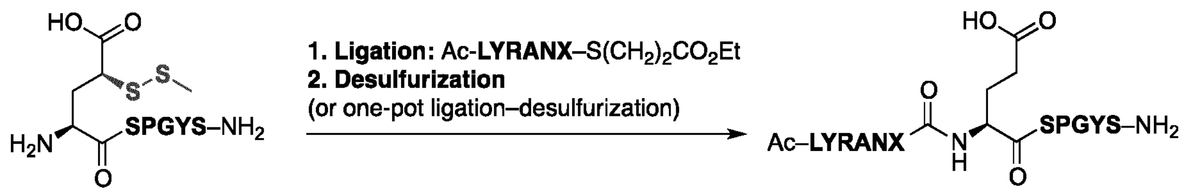


**12**

| entry | thioester (**X**=) | ligation yield[a] (%) | desulfurization yield[a] (%) | one-pot yield[c] |
|---|---|---|---|---|
| 1 | Gly | 72% | 89% (64%)[b] | 73% |
| 2 | Ala | 77% | 91% (70%)[b] | 67% |
| 3 | Met | 83% | 98% (81%)[b] | 72% |
| 4 | Phe | 80% | 84% (67%)[b] | 74% |
| 5 | Val | 68% | 98% (67%)[b] | 56% |

[a] Isolated yields after HPLC purification. Ligation: 5 mM **12** in buffer (6 M Gn.HCl, 100 mM $Na_2HPO_4$, 50 mM TCEP), PhSH (2 vol %), 37 °C, pH 7.2-7.4, 16 h. Desulfurization: 500 mM TCEP in buffer (6 M Gn.HCl, 100 mM $Na_2HPO_4$), reduced glutathione (40 mM), VA-044 (200 mM), pH 6.5-6.8, 65 °C, 16 h.

[b] Yield over 2 steps.

[c] Desulfurization reactions were carried out at 37 °C in the one-pot protocol.

The inventors next investigated developing this concept to effect the one-pot transformation at γ-thiol-Glu containing model peptides (Table 1, entries 1-5). They envisaged that this would not only streamline the methodology by preventing additional purification steps but would also avoid peptide splicing facilitated by the γ-thiol during purification. Specifically, each ligation reaction was first carried out with careful monitoring and shown to proceed to completion within 4 hours, as determined by LC-MS analysis, with the exception of the ligation reaction with model thioester Ac-LYRANV-$S(CH_2)_2CO_2Et$ (entry 5) which required a reaction time of 16 hours to reach completion. After this time, thiophenol was extracted from the reaction mixture by washing with diethyl ether in order to prevent poisoning of the desulfurization reaction by thiophenol. The ligation reaction mixture was immediately treated with TCEP (500 mM), reduced glutathione (40 mM) and VA-044 (200 mM), affording the desired peptide products in excellent yields over the two steps (Table 1, 56%-74% yield).

For one-pot ligation-desulfurization reactions with peptide thioesters bearing C-terminal Ala, Met, Phe and Val residues, peptide by-product **14** was also observed, which may result from reaction of the resulting VA-044 radical with the peptide radical formed following H-abstraction from the γ-thiol (Figure 1). As this by-product was not observed in the two-step ligation-desulfurization procedures, it is likely that this pathway results from trace amounts of aryl thiol remaining despite the extraction protocol. Nonetheless, in all cases this side reaction did not significantly impact the isolated yield or the overall efficiency of the reaction methodology.

In conclusion, the inventors have developed a concise and scalable synthesis of a novel γ-thiol-Glu building block **10** which can be readily incorporated into a variety of peptides to faciliate ligation chemistry. The resulting γ-thiol-Glu peptides undergo facile ligation reactions with a range of thioesters, and can be desulfurized to the native peptide products using radical-based conditions. Furthermore, the inventors have extended this methodology to include a one-pot ligation-desulfurization cascade which proved to be efficient and high yielding, whilst reducing the need for intermediate purification of the ligation products.

**Example 3**

It should be noted that numbering of structures within this example are specific to this example and do not relate to numbering of structures elsewhere in this specification.

As TFET (2,2,2-trifluoroethanethiol) has been shown to be an additive for efficient and operationally simple one-pot ligation-desulfurization reactions, the inventors were interested using this reagent in extending the scope of the methodology described herein to the practical synthesis of some small protein targets. The first target protein was the 70 amino acid thrombin inhibitory protein chimadanin **(12)** produced by the hard tick *Haemaphysalis longicornis* to facilitate the hematophagous activity of the organism. The synthesis of the protein was performed *via* the assembly of three fragments in the C- to N-direction. Specifically, the inventors proposed using a γ-thiol Glu ligation followed by a native chemical ligation-desulfurization at Cys that would proceed with concomitant desulfurization of the γ-thiol auxiliary on the Glu residue to generate the native protein. Importantly, this proposed one-pot strategy would abolish intermediary purification steps thus limiting the exposure of the sensitive γ-thiol moiety to acidic HPLC buffers that leads to thiolactamization and peptide cleavage.

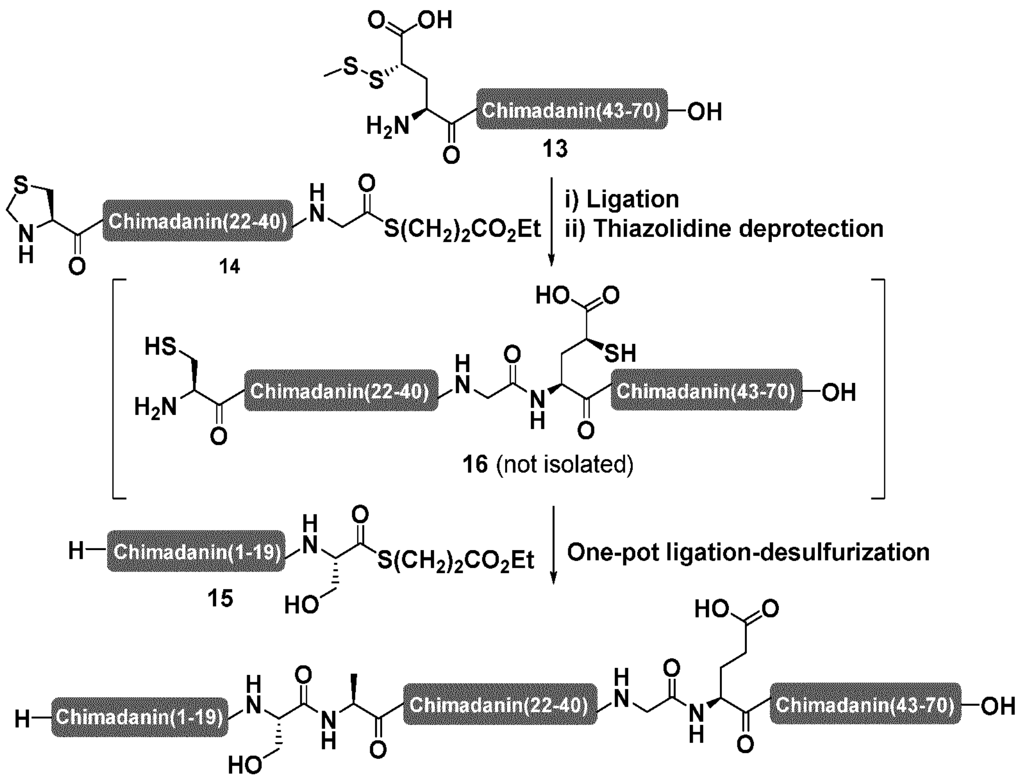


**Chimadanin (12)**
**35% yield over 4 steps after HPLC purification**

The above scheme illustrates the one-pot synthesis of Chimadanin (12) using TFET. Conditions were as follows: *i) Ligation:* **14** (1.0 equiv.) and **13** (1.2 equiv.) in buffer (6 M Gn·HCl, 100 mM $Na_2HPO_4$, 25 mM TCEP), pH 6.8, conc. 2.5 mM with respect to **14,** 2vol.% TFET, 30 °C, 2 h; ii) *Thiazolidine deprotection:* 120 μL of 0.2 M methoxyamine, final conc. 1.5 mM, 30 °C, 3 h; *One-pot ligation-desulfurization: Ligation:* pH readjusted to 7.0, addition of **15** (1.3 equiv. 3.0 mM) in buffer (6 M Gn·HCl, 100 mM $Na_2HPO_4$, 25 mM TCEP), pH 6.8, TFET (2 vol.%), conc. 1.0 mM with respect to **16,** 30 °C, 18 h. *Desulfurization:* readjust to 500 mM TCEP and 40 mM reduced glutathione in ligation buffer (500 μL), argon sparge, pH adjustment to 6.2, solid VA-044 (20 mM final conc.), 37 °C, 5 h.

The synthesis began with the preparation of the requisite fragments *via* Fmoc-strategy SPPS, including chimadanin 43-70 **(13)** possessing an N-terminal γ-thiol Glu residue, chimadanin 22-40 **(14)** bearing an N-terminal thiazolidine and a C-terminal thioester functionality, and chimadanin 1-19 thioester **15.** Peptide **13** (1.2 equiv.) bearing an N-terminal γ-thiol Glu residue was first ligated with peptide thioester **14** (1.0 equiv.) in the presence of TFET. Following completion of the ligation reaction (as judged by HPLC-MS analysis) the reaction mixture was subsequently treated with methoxyamine at a pH of 4.2 to unmask an N-terminal Cys residue and afford intermediate **16.** Rather than purifying the intermediate, the pH of the reaction mixture was readjusted to 6.8 before the addition of the N-terminal chimadanin fragment, peptide thioester **15** and TFET. The ligation of **15** and **16** was again monitored by HPLC-MS and, upon completion, the reaction was degassed before treating with additional TCEP, reduced glutathione and VA-044 to effect global desulfurization affording the native protein. Gratifyingly, chimadanin was isolated in 35% yield over the one-pot four step sequence following a single HPLC purification step (> 77% average yield per step).

To further probe the limits of the one-pot ligation-desulfurization reactions employing the TFET additive, the inventors next investigated the potential of combining kinetically-controlled ligation chemistry with the one-pot methodology to assemble the 60-amino acid protein madanin-1 **(17),** a Cys-free competitive thrombin inhibitor also produced by the hard tick *H. longicornis* that is proteolytically processed by thrombin. The use of a kinetically-controlled ligation sequence would enable the rapid assembly of multiple madanin-1 peptide segments in the N- to C-direction without intermediate purification steps through appropriate reactivity tuning of the requisite peptide thioesters. With a view to future analogue generation, the protein was assembled *via* three short segments, namely madanin-1 (1-27) **18** as a preformed TFET-thioester, madanin-1 (30-46) **19** bearing an N-terminal β-thiol Asp residue and an unreactive C-terminal alkyl thioester and madanin-1 (49-60) **20** possessing an N-terminal Cys residue.

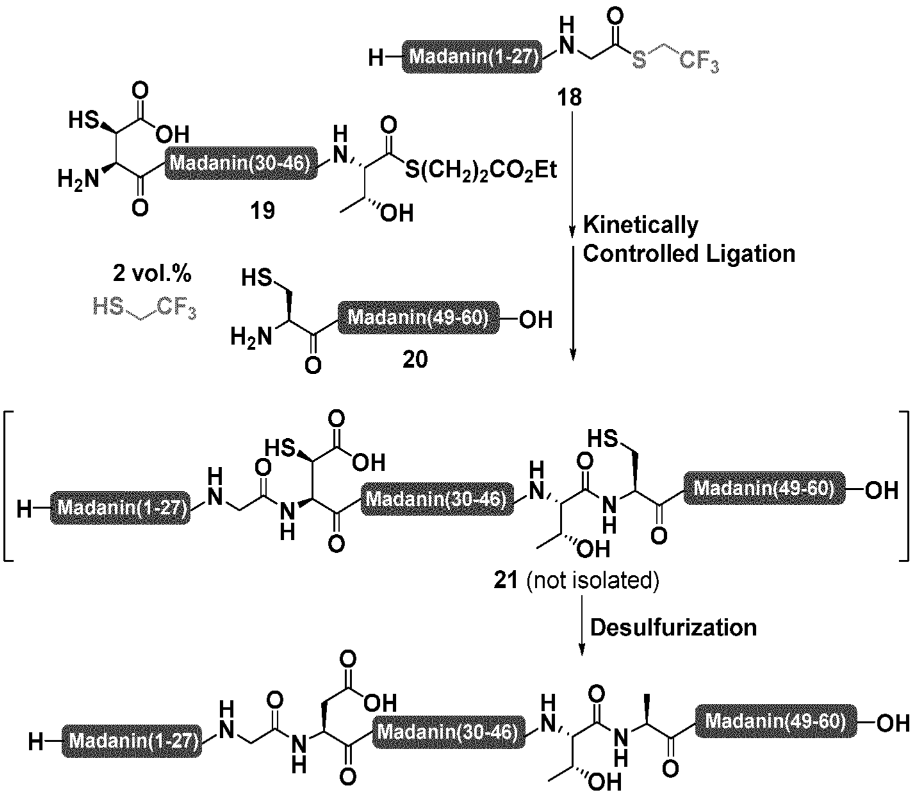


**Madanin-1 (17)**
**42% isolated yield over 3 steps after HPLC purification**

The above scheme illustrates synthesis of Madanin-1 (17) via a one-pot kinetically-controlled ligation-desulfurization with TFET. Conditions used in the synthesis are as follows: *Kinetically-controlled ligation:* **18** (1.2 equiv.), **19** (1.0 equiv., 5 mM) in buffer (6 M Gn·HCl, 100 mM $Na_2HPO_4$, 50 mM TCEP), pH 7.4-7.5, 37 °C, 1 h, then addition of **20** (1.8 equiv.), TFET (2 vol.%), 37 °C, 12 h; *Desulfurization:* Argon sparge, adjust to TCEP (200 mM), reduced glutathione (40 mM), VA-044 (20 mM) in buffer (6 M Gn·HCl, 100 mM $Na_2HPO_4$), 2.5 mM final conc. with respect to **21,** pH 6.5, 37 °C, 16 h.

Peptide thioester **18** activated as the preformed TFET-thioester was first ligated with peptide alkyl thioester **19** bearing an N-terminal β-SH Asp moiety and a C-terminal Thr residue. Following completion of the ligation after 1 h (as judged by HPLC-MS analysis) peptide **20** was added in combination with 2 vol.% TFET to activate the alkyl thioester and facilitate a second ligation reaction. Following completion of the second ligation (12 h) the product **21** was not isolated but rather subjected to *in situ* desulfurization of both the Cys and β-thiol Asp residues to afford the native protein madanin-1 **(17)** in an excellent 42% yield over the 3 steps. This represents the first report of a one-pot kinetically controlled ligation-desulfurization reaction and clearly highlights the utility of TFET in the context of chemical protein synthesis. Importantly, the *in vitro* inhibitory activity of chimadanin (**12,** $IC_{50}$ = 788 nM) and madanin-1 (**17,** $IC_{50}$ = 1590 nM) against the amidolytic activity of thrombin were shown to be similar to that known for recombinant madanin-1, thus confirming that the synthetic proteins possessed the expected thrombin-inhibiting activity.

In summary, the inventors have demonstrated that the alkyl thiol TFET can be successfully employed as an additive in native chemical ligation to facilitate ligations with rates comparable to the gold standard additive MPAA. More importantly, TFET can be used in ligation-desulfurization chemistry without the need for intermediate purification or removal/capture from the reaction mixture. The utility of TFET is highlighted as an additive for one-pot ligation-desulfurization reactions both on model peptide systems and in the assembly of multiple peptide fragments to access proteins. Specifically, the additive has been used for the efficient assembly of the tick-derived thrombin inhibitory proteins chimadanin and madanin-1 through C- to N-assembly and kinetically controlled approaches, respectively. Given the efficiency and simplicity of ligations employing TFET (a commercially available and affordable reagent) it is anticipated that it will find widespread use in the chemical synthesis of proteins and post-translationally modified proteins, greatly improving the efficiency of the processes and reducing handling and purification of intermediates.

## Claims

1. A process for introducing a thiol group α to a carbonyl group in a side chain of a protected α-amino acid, said protected α-amino acid having protecting groups on both the α-amine group and the α-carboxyl group, said process comprising:

   a) if the side chain contains a functional group comprising a heteroatom bearing a hydrogen atom, protecting said functional group, wherein if the α-amino acid is either aspartic acid or glutamic acid and the side chain carboxyl group is protected by an ester, the ester is t-butyl, methyl or allyl ester;
   b) treating the protected amino acid with a base of sufficient strength to abstract a hydrogen atom α to said carbonyl group, so as to form an anion;
   c) treating the anion with a reagent of structure Pr-S-L in which L is a leaving group and Pr is a thiol-protecting group, so as to introduce a Pr-S-group α to the carbonyl group; and
   d) converting the Pr-S- group to an H-S- (thiol) group.

2. The process of claim 1 wherein the carbonyl group in the side chain of the protected α-amino acid is present in an aldehyde, ketone, carboxylic acid, carboxylic ester or amide group.

3. The process of claim 2 wherein the carbonyl group is present in a carboxylic acid group or a carboxylic ester group.

4. The process of claim 3 wherein the protected α-amino acid is either aspartic acid or glutamic acid, each having both the α amino group and the α carboxyl group protected, and wherein step a) comprises forming an ester of the side chain carboxyl group.

5. The process of any one of claims 1 to 4 wherein the α-amine group of the protected amino acid is protected as a Boc (t-butyloxycarbonyl) protecting group.

6. The process of any one of claims 1 to 5 wherein the α-carboxyl group of the protected amino acid is protected as an allyl ester.

7. The process of any one of claims 1 to 6 wherein Pr is an electron rich group and L is an electron poor group.

8. The process of any one of claims 1 to 7 comprising step c') reacting a functional group in the side chain so as to produce a modified natural amino acid, or a protected form of a modified natural amino acid, the modification being a β- or γ-thiol group, step c') being conducted after step c) and before step d).

9. The process of any one of claims 1 to 8 comprising step c'') deprotecting the α-carboxyl group and coupling the α-carboxyl group of the product of step c) with a peptide so as to produce a peptide having an N-terminus protected amino acid residue having a Pr-S- group in the side chain.

10. The process of any one of claims 1 to 9 comprising additional step c''') coupling the amino acid having a Pr-S- group in its side chain or peptide having an N-terminal amino acid residue having a Pr-S- group in its side chain with a thioester of an amino acid or of a peptide so as to form a ligated peptide having an H-S- group in the side chain of the amino acid residue derived from the amino acid having the Pr-S-group in the side chain or peptide having an N-terminal amino acid residue having the Pr-S-group in the side chain.

11. The process of claim 10 additionally comprising step e) desulfurizing the ligated peptide.

12. The process of claim 11 wherein said ligated peptide comprises a cysteine residue and step e) comprises selectively desulfurizing the ligated peptide with a mild reducing agent so as not to desulfurize the cysteine residue.

13. The process of claim 12 wherein the mild reducing agent comprises a phosphine.

14. The process of claim 12 or claim 13 wherein the reducing agent additionally comprises a thiol.

15. The process of any one of claims 12 to 14 wherein step e) is conducted at acidic pH.

**Patentansprüche**

1. Verfahren zur Einführung einer Thiolgruppe α in eine Carbonylgruppe in einer Seitenkette einer geschützten α-Aminosäure, wobei die geschützte α-Aminosäure Schutzgruppen sowohl an der α-Amingruppe als auch an der α-Carboxylgruppe aufweist, das Verfahren umfassen:

   a) wenn die Seitenkette eine funktionelle Gruppe enthält, umfassend ein Heteroatom, das ein Wasserstoffatom trägt, das die funktionelle Gruppe schützt, wobei, wenn die α-Aminosäure entweder Asparaginsäure oder Glutaminsäure ist und die Seitenkettencarboxylgruppe durch einen Ester geschützt ist, der Ester t-Butyl-, Methyl- oder Allylester ist;
   b) Behandeln der geschützten Aminosäure mit einer Base von ausreichender Stärke, um ein Wasserstoffatom α von der Carbonylgruppe abzuziehen, um ein Anion zu bilden;
   c) Behandeln des Anions mit einem Reagens der Struktur Pr-S-L, worin L eine Abgangsgruppe ist und Pr eine Thiol-Schutzgruppe ist, um eine Pr-S-Gruppe α in die Carbonylgruppe einzuführen; und
   d) Umwandeln der Pr-S-Gruppe in eine HS-(Thiol)-Gruppe.

2. Verfahren nach Anspruch 1, wobei die Carbonylgruppe in der Seitenkette der geschützten α-Aminosäure in einer Aldehyd-, Keton-, Carbonsäure-, Carbonsäureester oder Amidgruppe vorhanden ist.

3. Verfahren nach Anspruch 2, wobei die Carbonylgruppe in einer Carbonsäuregruppe oder einer Carbonsäureestergruppe vorhanden ist.

4. Verfahren nach Anspruch 3, wobei die geschützte α-Aminosäure entweder Asparaginsäure oder Glutaminsäure ist, jede von denen sowohl die α-Amingruppe als auch die α-Carboxylgruppe geschützt haben, und wobei Schritt a) das Bilden eines Esters der Seitenkettencarboxylgruppe umfasst.

5. Verfahren nach einem der Ansprüche 1 bis 4, wobei die α-Amingruppe der geschützten Aminosäure als eine Boc (t-Butyloxycarbonyl) - Schutzgruppe geschützt ist.

6. Verfahren nach einem der Ansprüche 1 bis 5, wobei die α-Carboxylgruppe der geschützten Aminosäure als ein Allylester geschützt ist.

7. Verfahren nach einem der Ansprüche 1 bis 6, wobei Pr eine elektronenreiche Gruppe ist und L eine elektronenarme Gruppe ist.

8. Verfahren nach einem der Ansprüche 1 bis 7, umfassend Schritt c') Reagieren einer funktionellen Gruppe in der Seitenkette, um eine modifizierte natürliche Aminosäure oder eine geschützte Form einer modifizierten natürlichen Aminosäure herzustellen, wobei die Modifikation eine β- oder γ-Thiolgruppe ist, wobei Schritt c') nach Schritt c) und vor Schritt d) durchgeführt wird.

9. Verfahren nach einem der Ansprüche 1 bis 8, umfassend Schritt c"), Entschützen der α-Carboxylgruppe und Koppeln der α-Carboxylgruppe des Produkts von Schritt c) mit einem Peptid, um ein Peptid mit einem N-Terminus-geschützten Aminosäurerest mit einer Pr-S-Gruppe in der Seitenkette herzustellen.

10. Verfahren nach einem der Ansprüche 1 bis 9, umfassend den zusätzlichen Schritt c"') Koppeln der Aminosäure mit einer Pr-S-Gruppe in ihrer Seitenkette, oder Peptid, mit einem N-terminalen Aminosäurerest mit einer Pr-S-Gruppe in seiner Seitenkette mit einem Thioester einer Aminosäure oder eines Peptids, um ein ligiertes Peptid mit einer H-S-Gruppe in der Seitenkette des Aminosäurerests zu bilden, der von der Aminosäure mit der Pr-S-Gruppe in der Seitenkette stammt, oder Peptid mit einem N-terminalen Aminosäurerest mit der Pr-S-Gruppe in der Seitenkette.

11. Verfahren nach Anspruch 10, zusätzlich umfassend Schritt e) Entschwefeln des ligierten Peptids.

12. Verfahren nach Anspruch 11, wobei das ligierte Peptid einen Cysteinrest umfasst und Schritt e) selektives Entschwefeln des ligierten Peptids mit einem milden Reduktionsmittel umfasst, um den Cysteinrest nicht zu entschwefeln.

13. Verfahren nach Anspruch 12, wobei das milde Reduktionsmittel ein Phosphin umfasst.

**14.** Verfahren nach Anspruch 12 oder Anspruch 13, bei dem das Reduktionsmittel zusätzlich ein Thiol umfasst.

**15.** Verfahren nach einem der Ansprüche 12 bis 14, wobei Schritt e) bei saurem pH durchgeführt wird.

**Revendications**

**1.** Procédé pour introduire un groupe thiol en α dans un groupe carbonyle dans une chaîne latérale d'un acide α-aminé protégé, ledit acide α-aminé protégé ayant des groupes protecteurs tant sur le groupe α-amine que sur le groupe α -carboxyle, ledit procédé comprenant :

a) si la chaîne latérale contient un groupe fonctionnel comprenant un hétéroatome portant un atome d'hydrogène, la protection dudit groupe fonctionnel, et si l'acide α-aminé est soit l'acide aspartique soit l'acide glutamique et que le groupe carboxyle de chaîne latérale est protégé par un ester, alors l'ester est un ester de t-butyle, de méthyle ou d'allyle ;
b) le traitement de l'acide aminé protégé avec une base de force suffisante pour retirer un atome d'hydrogène en α audit groupe carbonyle, de façon à former un anion ;
c) le traitement de l'anion avec un réactif de structure Pr-S-L dans laquelle L est un groupe partant et Pr est un groupe protecteur de thiol, de façon à introduire un groupe Pr-S- en α dans le groupe carbonyle ; et
d) la conversion du groupe Pr-S- en un groupe H-S- (thiol).

**2.** Procédé selon la revendication 1, dans lequel le groupe carbonyle dans la chaîne latérale de l'acide α-aminé protégé est présent dans un groupe aldéhyde, cétone, acide carboxylique, ester carboxylate ou amide.

**3.** Procédé selon la revendication 2, dans lequel le groupe carbonyle est présent dans un groupe acide carboxylique ou un groupe ester carboxylate.

**4.** Procédé selon la revendication 3, dans lequel l'acide α-aminé protégé est soit l'acide aspartique soit l'acide glutamique, chacun ayant tant le groupe α-amino que le groupe α-carboxyle qui sont protégés, et dans lequel l'étape a) comprend la formation d'un ester du groupe carboxyle de chaîne latérale.

**5.** Procédé selon l'une quelconque des revendications 1 à 4, dans lequel le groupe α-amine de l'acide aminé protégé est protégé sous la forme d'un groupe protecteur Boc (t-butyloxycarbonyle).

**6.** Procédé selon l'une quelconque des revendications 1 à 5, dans lequel le groupe α-carboxyle de l'acide aminé protégé est protégé sous la forme d'un ester d'allyle.

**7.** Procédé selon l'une quelconque des revendications 1 à 6, dans lequel Pr est un groupe riche en électrons et L est un groupe pauvre en électrons.

**8.** Procédé selon l'une quelconque des revendications 1 à 7, comprenant l'étape c') consistant à faire réagir un groupe fonctionnel dans la chaîne latérale de façon à produire un acide aminé naturel modifié, ou une forme protégée d'un acide aminé naturel modifié, la modification étant un groupe β- ou γ-thiol, l'étape c') étant mise en oeuvre après l'étape c) et avant l'étape d).

**9.** Procédé selon l'une quelconque des revendications 1 à 8, comprenant l'étape c") consistant à déprotéger le groupe α-carboxyle et à coupler le groupe α-carboxyle du produit de l'étape c) avec un peptide de façon à produire un peptide ayant un résidu d'acide aminé protégé N-terminal ayant un groupe Pr-S- dans la chaîne latérale.

**10.** Procédé selon l'une quelconque des revendications 1 à 9, comprenant l'étape additionnelle c''') consistant à coupler l'acide aminé ayant un groupe Pr-S- dans sa chaîne latérale ou le peptide ayant un résidu d'acide aminé N-terminal ayant un groupe Pr-S- dans sa chaîne latérale avec un thioester d'un acide aminé ou d'un peptide de façon à former un peptide ligaturé ayant un groupe H-S- dans la chaîne latérale du résidu d'acide aminé dérivé de l'acide aminé ayant le groupe Pr-S- dans la chaîne latérale ou du peptide ayant un résidu d'acide aminé N-terminal ayant le groupe Pr-S-dans la chaîne latérale.

**11.** Procédé selon la revendication 10, comprenant de plus l'étape e) consistant à désulfurer le peptide ligaturé.

12. Procédé selon la revendication 11, dans lequel ledit peptide ligaturé comprend un résidu de cystéine et l'étape e) comprend la désulfuration sélective du peptide ligaturé avec un agent réducteur doux de façon qu'il ne désulfurise pas le résidu de cystéine.

13. Procédé selon la revendication 12, dans lequel l'agent réducteur doux comprend une phosphine.

14. Procédé selon la revendication 12 ou la revendication 13, dans lequel l'agent réducteur comprend de plus un thiol.

15. Procédé selon l'une quelconque des revendications 12 à 14, dans lequel l'étape e) est mise en oeuvre à un pH acide.

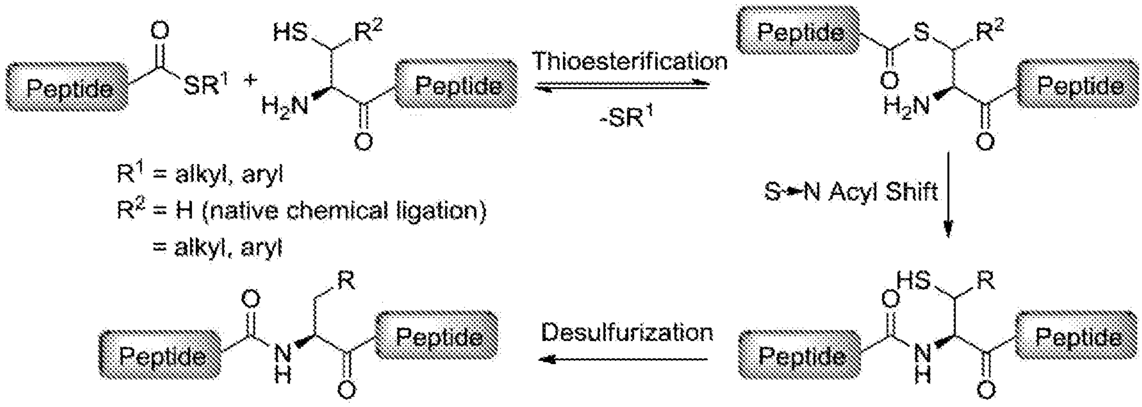


Fig. 1

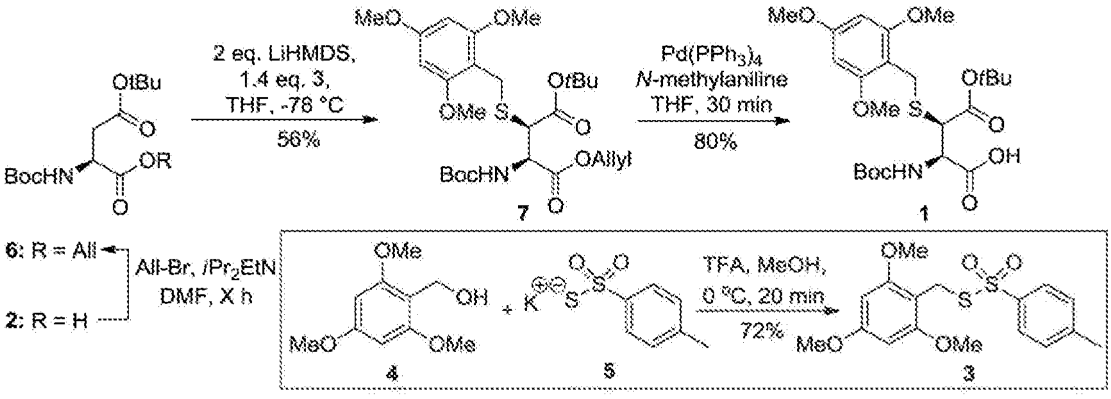


7 1

6: R = All

2: R = H

4 5 3

Fig. 2

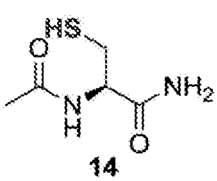

14

($C-S$ BDE = 308.5 $kJmol^{-1}$)

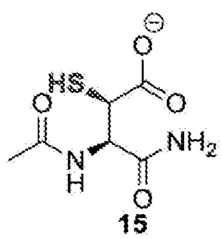

15

($C-S$ BDE = 308.0 $kJmol^{-1}$)

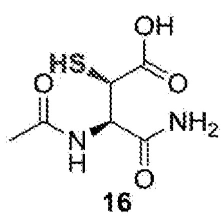

16

($C-S$ BDE = 298.3 $kJmol^{-1}$)

Fig. 3

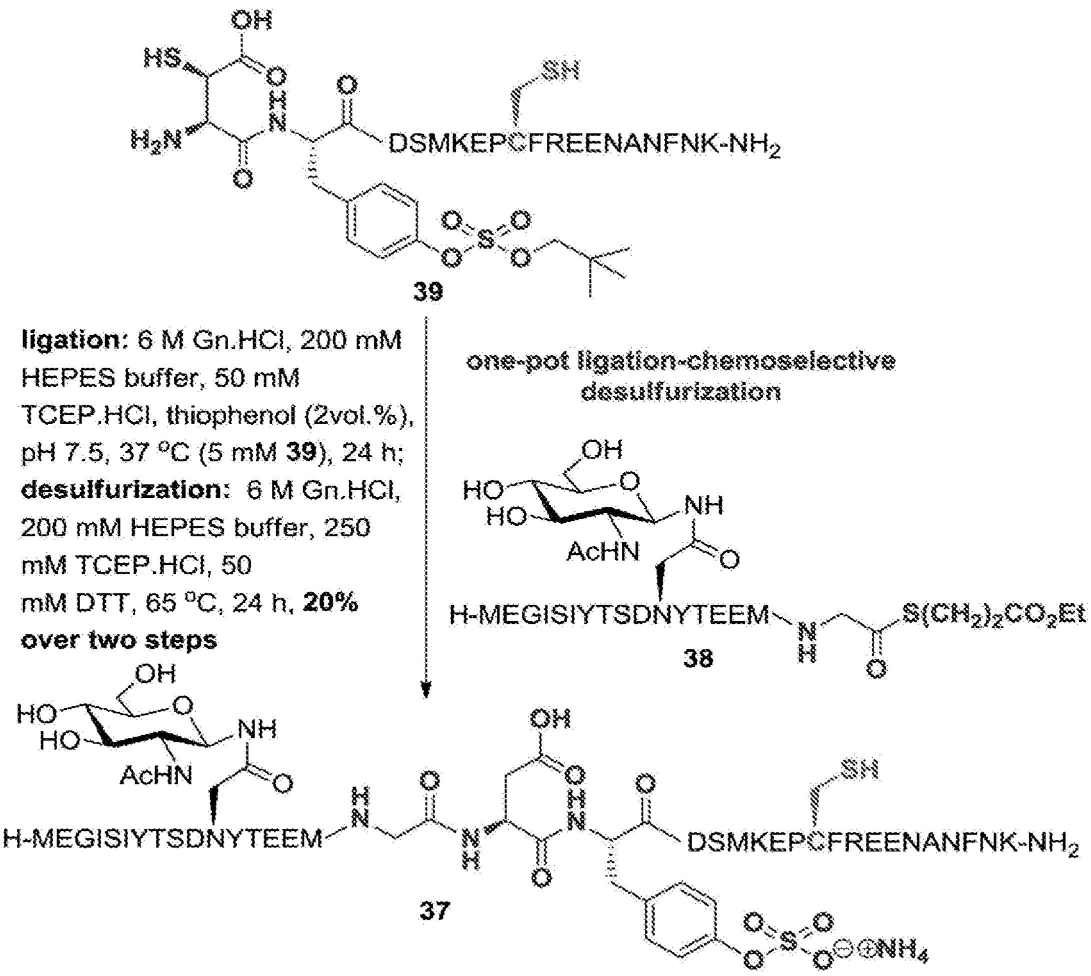
ligation: 6 M Gn.HCl, 200 mM HEPES buffer, 50 mM TCEP.HCl, thiophenol (2vol.%), pH 7.5, 37 °C (5 mM 39), 24 h;
desulfurization: 6 M Gn.HCl, 200 mM HEPES buffer, 250 mM TCEP.HCl, 50 mM DTT, 65 °C, 24 h, 20% over two steps
one-pot ligation-chemoselective desulfurization

39

38

37

Fig. 4

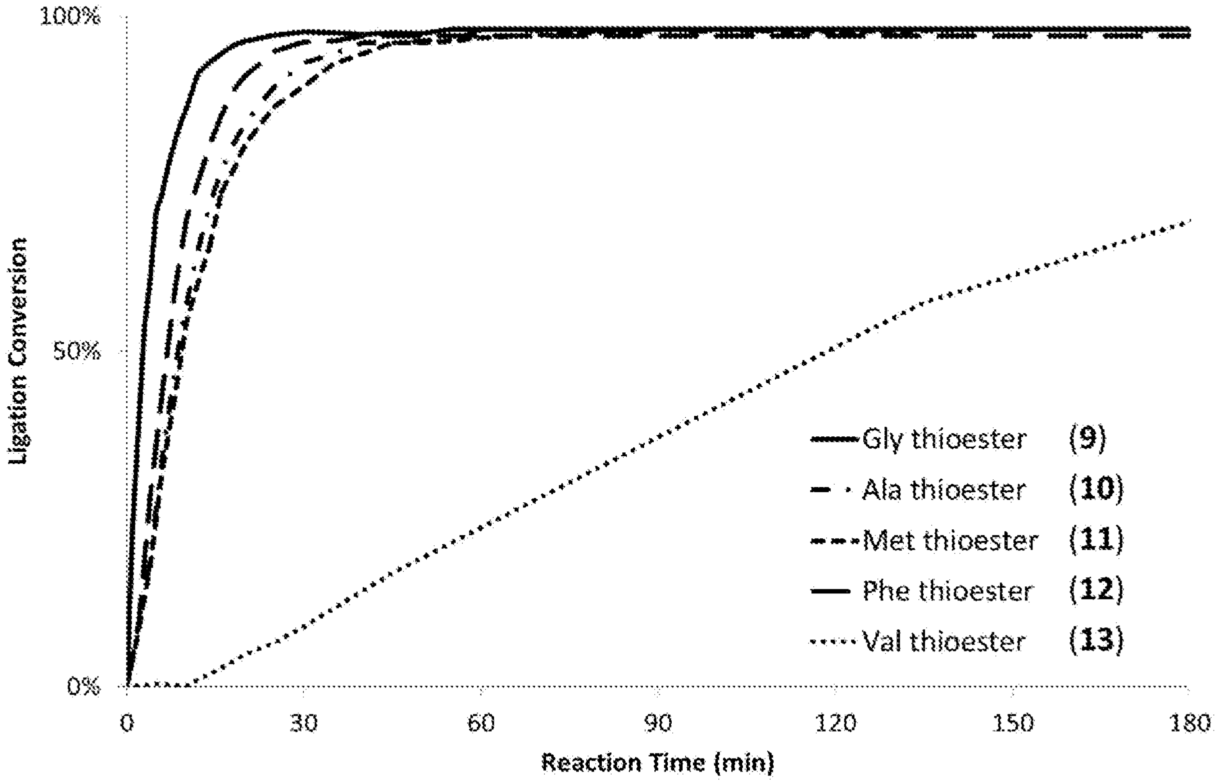
100%
50%
0%
Ligation Conversion
0
30
60
90
120
150
180
Reaction Time (min)
Gly thioester (9)
Ala thioester (10)
Met thioester (11)
Phe thioester (12)
Val thioester (13)

Fig. 5

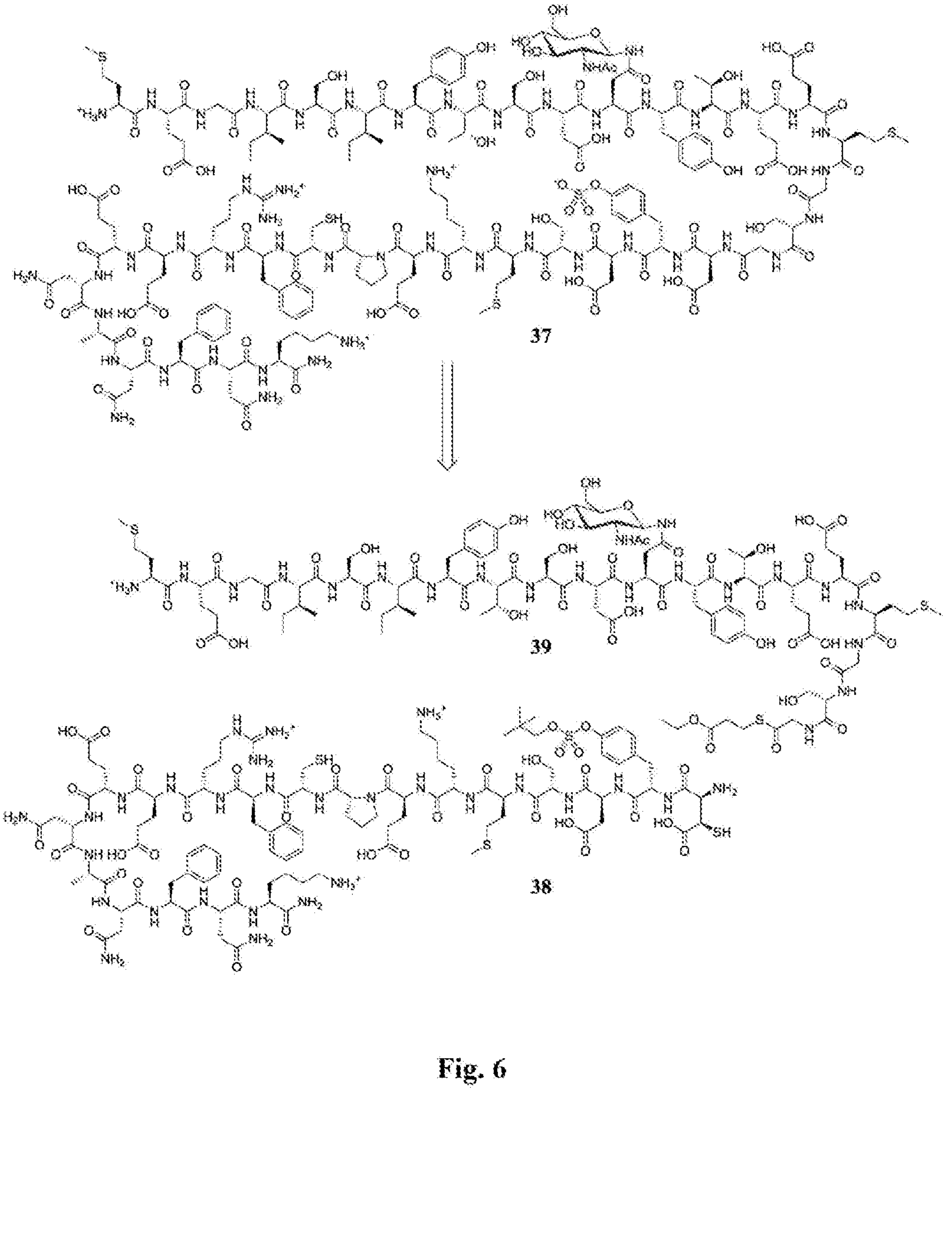

37

39

38

Fig. 6

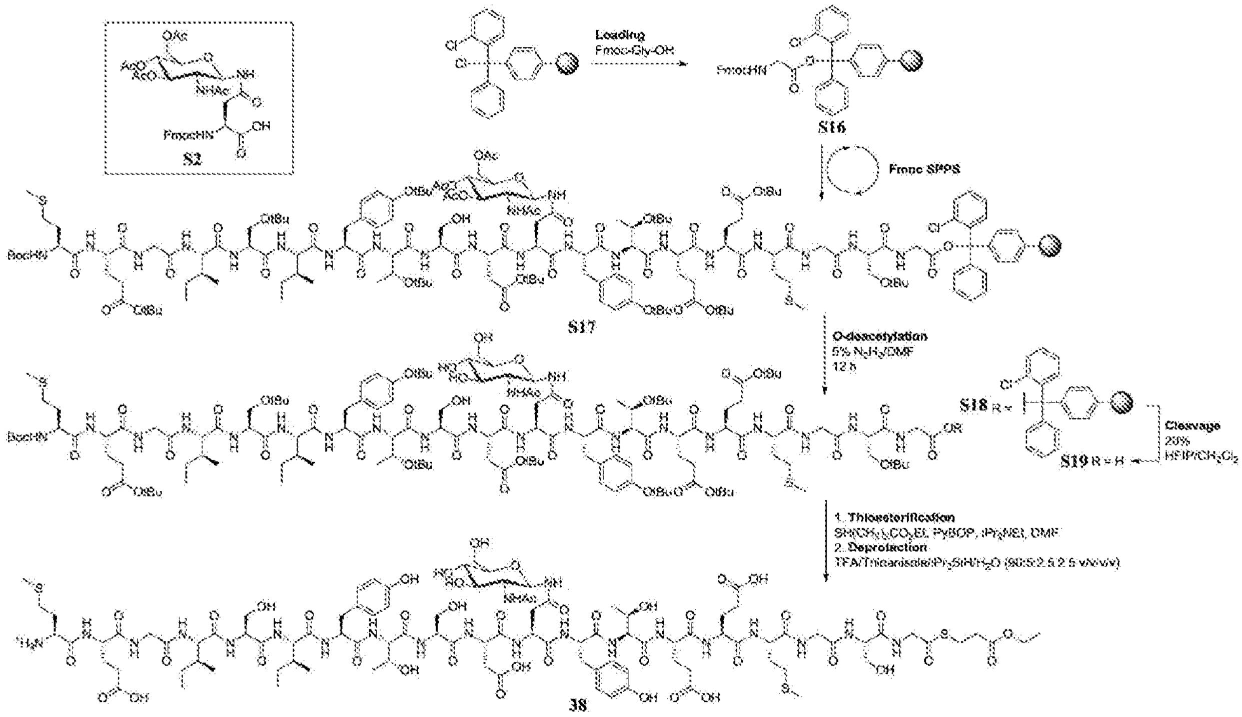
S2
Loading
Fmoc-Gly-OH
S16
Fmoc SPPS
S17
O-deacetylation
5% $N_2H_4$/DMF
12 h
S18 R =
Cleavage
20%
HFIP/$CH_2Cl_2$
S19 R = H
1. Thioesterification
$SH(CH_2)_2CO_2Et$, PyBOP, $iPr_2NEt$, DMF
2. Deprotection
TFA/Thioanisole/$iPr_3SiH$/$H_2O$ (90:5:2.5:2.5 v/v/v/v)
38

**Fig. 7**

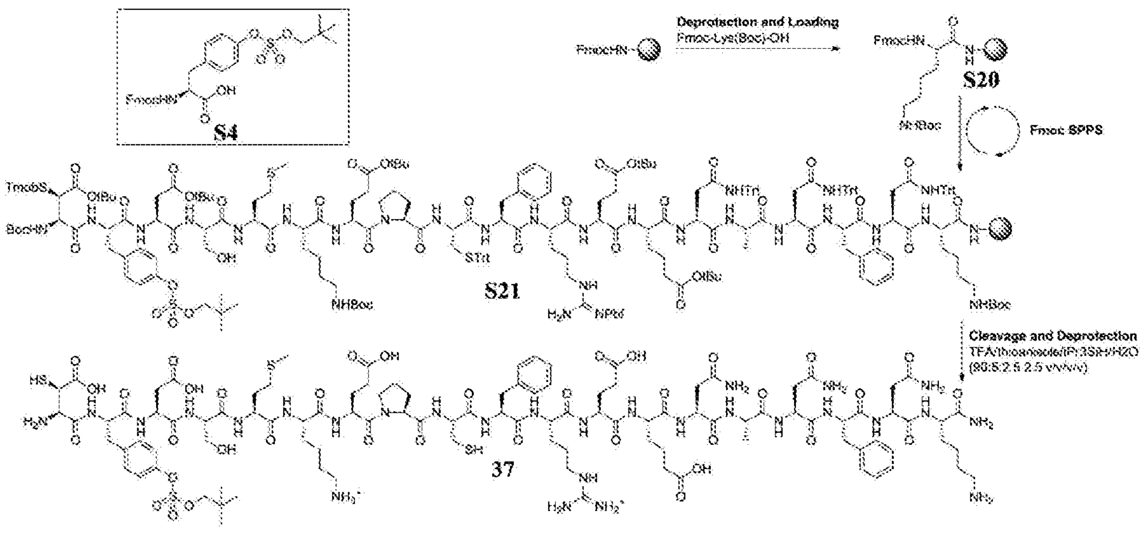
S4
Deprotection and Loading
Fmoc-Lys(Boc)-OH
S20
Fmoc SPPS
S21
Cleavage and Deprotection
TFA/thioanisole/$iPr_3SiH$/H2O
(90:5:2.5:2.5 v/v/v/v)
37

**Fig. 8**

**39 + 38**

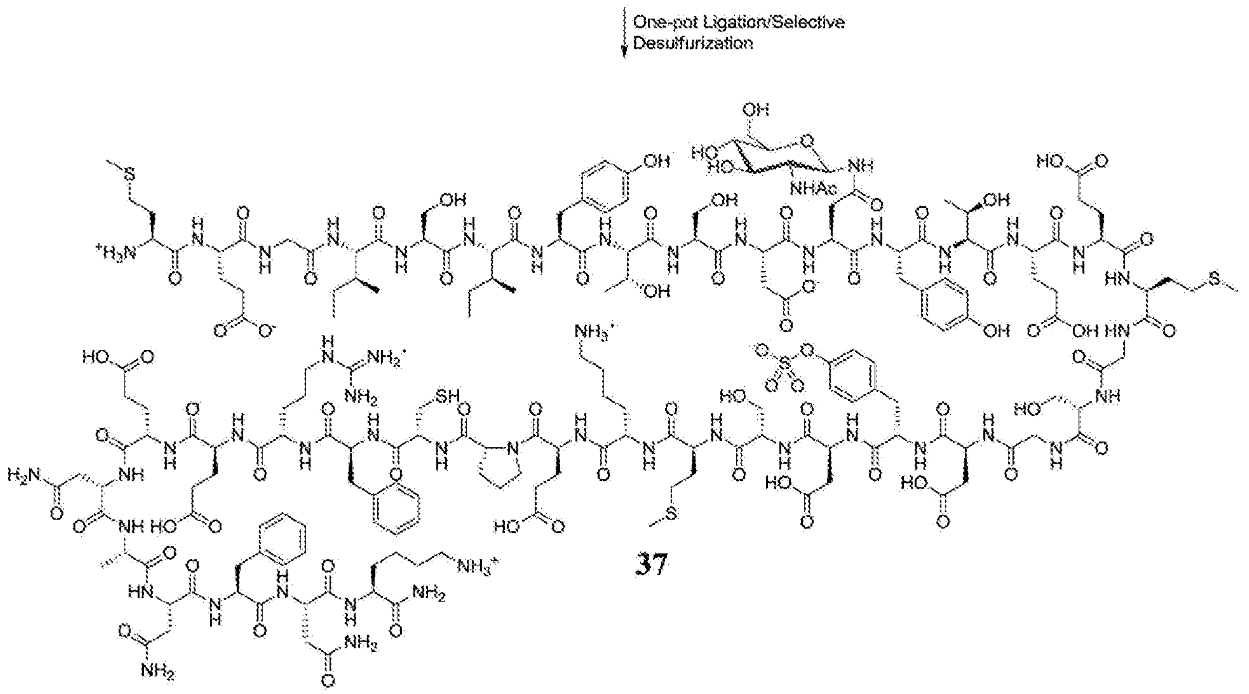


**37**

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Q. WAN ; S. J. DANISHEFSKY.** *Angew. Chem.,* 2007, vol. 119, 9408-9412
- **N. SHIBATA et al.** *Synlett,* 1996, vol. 6, 519-520
- **N. SHIBATA et al.** *Tetrahedron,* 1996, vol. 52 (39), 12839-12852
- **G. P. F. WOOD ; D. MORAN ; R. JACOB ; L. RADOM.** *J. Phys. Chem. A,* 2005, vol. 109, 6318-6325
- **D. J. HENRY ; M. B. SULLIVAN ; L. RADOM.** *J. Chem. Phys.,* 2003, vol. 118, 4849-4860